# EUROPEAN PATENT APPLICATION

(11) **EP 3 828 184 A1**
(43) Date of publication of application: **02.06.2021**
(21) Application number: 19853082.6
(22) Date of filing: 21.08.2019
(51) Int. Cl.: C07D 471/04, C07D 487/04, C07D 473/34, A61K 31/7076, A61P 37/02

(54) **PYRAZOLOPYRIMIDINE DERIVATIVE AND USE THEREOF AS PI3K INHIBITOR**

(30) Priority: 21.08.2018 CN 201810960230; 11.10.2018 CN 201811184709; 17.05.2019 CN 201910412148; 15.08.2019 CN 201910755701
(71) Applicant: Medshine Discovery Inc., Nanjing, Jiangsu 210032 (CN)
(72) Inventor: WU, Chengde, Shanghai 200131 (CN); HUANG, Jingjie, Shanghai 200131 (CN); YU, Tao, Shanghai 200131 (CN); LI, Jie, Shanghai 200131 (CN); GONG, Zhen, Shanghai 200131 (CN); LI, Jian, Shanghai 200131 (CN); CHEN, Shuhui, Shanghai 200131 (CN)
(74) Representative: Gille Hrabal Partnerschaftsgesellschaft mbB Patentanwälte
(86) International application number: PCT/CN2019/101741
(87) International publication number: WO 2020/038394

(57) **Abstract**

The present invention discloses a series of pyrazolopyrimidine derivatives and use thereof in preparing a medicament for treating a disease related to PI3K, and in particular, discloses a derivative compound of formula (I), a tautomer thereof or a pharmaceutically acceptable composition thereof.

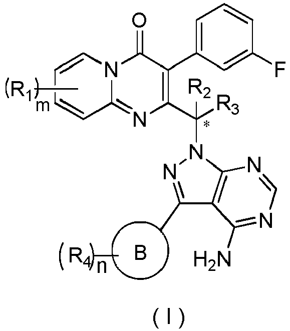

## Description

### CROSS-REFERENCE TO RELATED APPLICATIONS

The present application claims priority to Chinese Patent Application Nos.CN201810960230.0 filed on Aug. 21, 2018;CN201811184709.6 filed on Oct. 11, 2018;CN201910412148.9 filed on May 17, 2019; and CN201910755701.9 filed on Aug. 15, 2019.

### TECHNICAL FIELD

The present invention relates to a series of pyrazolopyrimidine derivatives and use thereof in preparing a medicament for the treatment ofa disease related to PI3K, in particular to a derivative compound of formula (I), a tautomer thereof or a pharmaceutically acceptable composition thereof.

### BACKGROUND

Phosphatidylinositol-3-kinase (PI3K) is a lipid kinase consisting of regulatory subunit p85 or p101, and catalytic subunit p110 (further categorized into four subtypes p110a, p110b, p110g and p110d), and plays a key role in cell proliferation, survival and metabolism by catalyzing phosphorylation of 3'-OH on the inositol ring of phosphatidylinositol 4,5-bisphosphate (PIP2) into phosphatidylinositol 3,4,5-trisphosphate (PIP3) to activate downstream Akt and the like. PI3K is overexpressed in tumor cells, resulting in rapid proliferation and growth of tumor cells.

The tumor suppressor gene PTEN (phosphatase, tension homolog deleted on chromosome ten) dephosphorylates PIP3 into PIP2, resulting in negative feedback regulation of PI3K signal pathway, inhibition of cell proliferation and promotion of apoptosis. Frequent occurrence of PI3K gene mutation and amplification in cancer, deletion of PTEN gene in cancer and the like all suggest that overexpression of PI3K is closely related to tumorigenesis.

TGR-1202 is a second generation PI3Kδ inhibitor developed by TG Therapeutics, Inc., and can significantly reduce hepatic and gastrointestinal toxic side effects in clinical trials compared to a first generation PI3Kδ inhibitor, and patients with large B-cell lymphoma also partially respond to TGR-1202. The structure of TGR-1202 is disclosed in International Patent Application No. WO2014006572. ACP-196 is a second generation BTK inhibitor which has been approved by FDA to be marketed, and as reported in the literature (PLoS ONE 12(2): e0171221.), the combination of the PI3Kδ inhibitor and the BTK inhibitor can jointly inhibit BCR signal pathways from two aspects, so that synergistic effect is achieved.

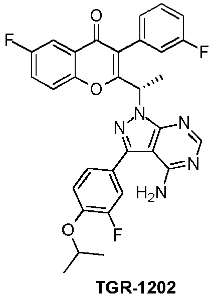

### SUMMARY

The present invention provides a compound of formula (I), an isomer thereof or a pharmaceutically acceptable salt thereof, 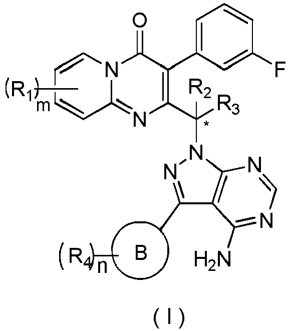 wherein,
R₁ is each independently selected from H, F, Cl, Br, I, OH, NH₂, CN, and C₁₋₃ alkyl optionally substituted with 1, 2 or 3 R_{c};
R₂ and R₃ are each independently selected from H and C₁₋₃ alkyl optionally substituted with 1, 2 or 3 Rₐ;
R₄ is selected from H, F, Cl, Br, I, OH, NH₂, CN, COOH, C₁₋₆ alkyl, C₁₋₆ alkoxy, C₁₋₆ alkylthio, C₁₋₆ alkylamino, C₃₋₆ cycloalkyl and C₃₋₆ cycloalkyl-O-, wherein the C₁₋₆ alkyl, C₁₋₆ alkoxy, C₁₋₆ alkylthio, C₁₋₆ alkylamino, C₃₋₆ cycloalkyl and C₃₋₆ cycloalkyl-O- are optionally substituted with 1, 2 or 3 R_{b};
or, two R₄ and atoms connected thereto together form a 5-8 membered heterocycloalkenyl; ring B is selected from phenyl and 5-6 membered heteroaryl;
m is selected from 1, 2 and 3;
n is selected from 1, 2 and 3;
Rₐ, R_{b} and R_{c} are each independently selected from F, Cl, Br, I, OH, NH₂, CN, COOH, CH₃, CH₂CH₃ and OCH₃; the carbon atom with "*" is a chiral carbon atom present in a form of a single (R)- or (S)- enantiomer or in a form enriched with one enantiomer; and
the 5-6 membered heteroaryl contains 1, 2, 3 or 4 heteroatoms or heteroatom groups independently selected from -NH-, -O-, -S- and N.

In some embodiments of the present invention, R₁ is each independently selected from H, F, Cl, Br, I, OH, NH₂, CN, and CH₃ optionally substituted with 1, 2 or 3 R_{c}, while the other variants are defined as herein.

In some embodiments of the present invention, R₁ is each independently selected from H, F, Cl, Br, I, OH, NH₂, CN and CH₃, while the other variants are defined as herein.

In some embodiments of the present invention, R₂ and R₃ are each independently selected from H and CH₃ optionally substituted with 1, 2 or 3 Rₐ, while the other variants are defined as herein.

In some embodiments of the present invention, R₂ and R₃ are each independently selected from H and CH₃, while the other variants are defined as herein.

In some embodiments of the present invention, R₄ iseach independently selected from H, F, Cl, Br, I, OH, NH₂, CN, COOH, C₁₋₃ alkyl, C₁₋₃ alkylamino, C₁₋₃ alkylthio, C₃₋₄ cycloalkyl-O- and C₁₋₃ alkoxy, wherein the C₁₋₃ alkyl, C₁₋₃ alkylamino, C₁₋₃ alkylthio, C₃₋₄ cycloalkyl-O- and C₁₋₃ alkoxy are optionally substituted with 1, 2 or 3 R_{b}, while the other variants are defined as herein.

In some embodiments of the present invention, R₄ is each independently selected from H, F, Cl, Br, I, OH, NH₂, CN, COOH, CH₃, CH(CH₃)₂, CH₂CH₃, NHCH₃, SCH₃, OCH₃, OCH₂CH₃, 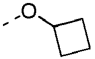 and 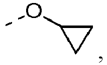 wherein the CH₃, CH(CH₃)₂, CH₂CH₃, NHCH₃, SCH₃, OCH₃, OCH₂CH₃, 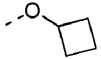 and 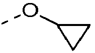 are optionally substituted with 1, 2 or 3 R_{b}, while the other variants are defined as herein.

In some embodiments of the present invention, R₄ is each independently selected from H, F, Cl, Br, I, OH, NH₂, CN, COOH, CH₃, CH₂F, CHF₂, CF₃, CH₂CH₃, CH(CH₃)₂, C(CH₃)₃, CH₂CF₃, NHCH₃, N(CH₃)₂, SCH₃, CH₂OCH₃, OCH₃, OCH(CH₃)₂, OCH₂CHF₂, OCH₂CF₃, OCH₂CH₂OCH₃, 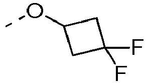 and 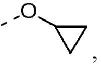 while the other variants are defined as herein.

In some embodiments of the present invention, two R₄ and atoms connected thereto together form 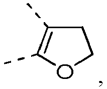 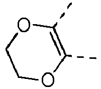 and 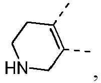 while the other variants are defined as herein.

In some embodiments of the present invention, ring B is selected from phenyl, thiazolyl, pyrazinyl, imidazolyl, pyrimidinyl, pyridazinyl, pyrazolyl and pyridinyl, while the other variants are defined as herein.

In some embodiments of the present invention, the structural unit 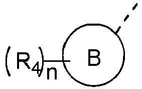 is selected from 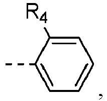 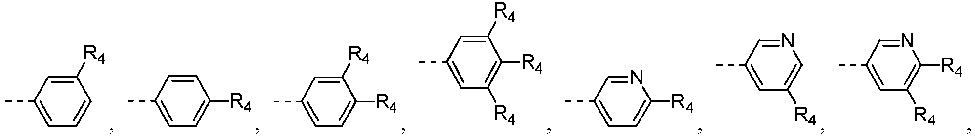 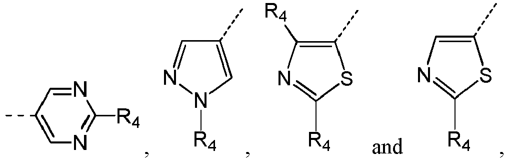 while the other variants are defined as herein.

In some embodiments of the present invention, the structural unit 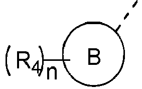 is selected from 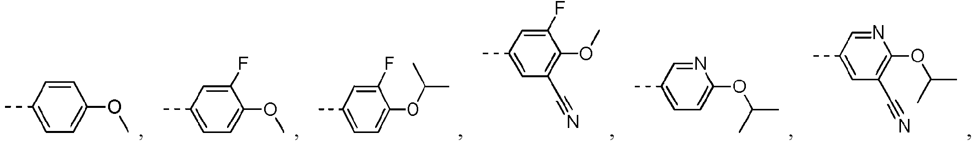 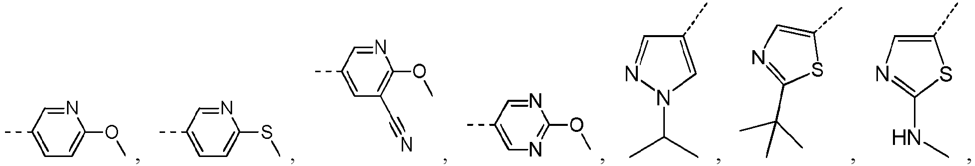 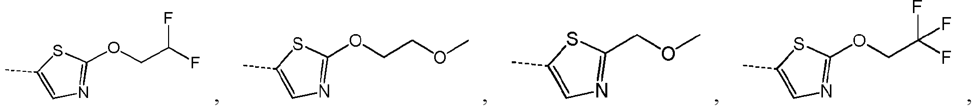 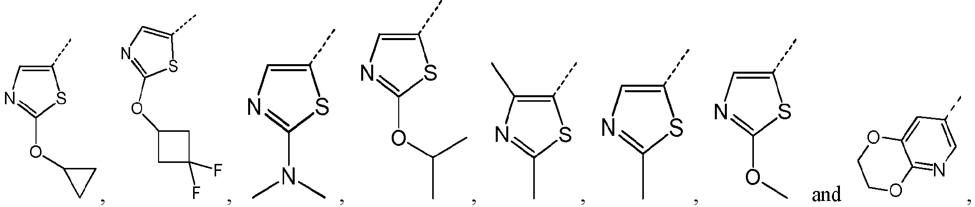 while the other variants are defined as herein.

Still some other embodiments of the present invention are derived from any combination of the variants described above.

In some embodiments of the present invention, provided is the compound selected from: 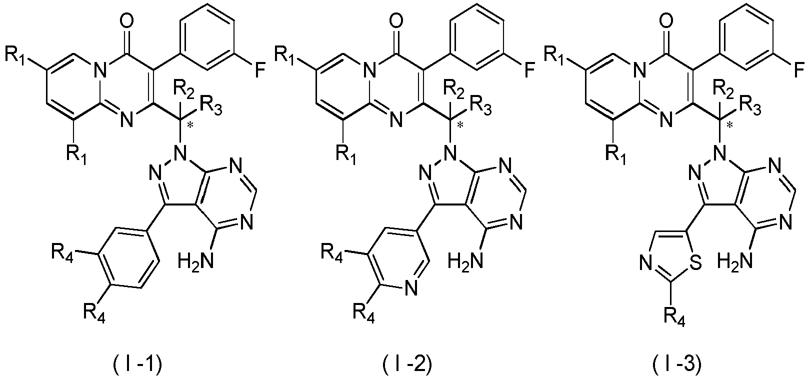 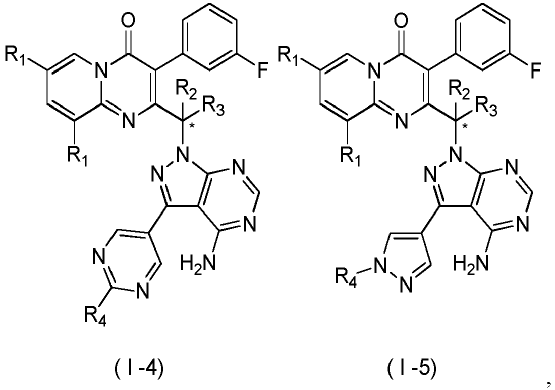 and the isomers thereof or the pharmaceutically acceptable salts thereof, wherein,
R₁ is defined as herein;
R₂ and R₃ are defined as herein;
the carbon atom with "*" is a chiral carbon atom present in a form of a single (R)- or (S)- enantiomer or in a form enriched with one enantiomer; and
R₄ is defined as herein.

In some embodiments of the present invention, provided is the compoundselected from: 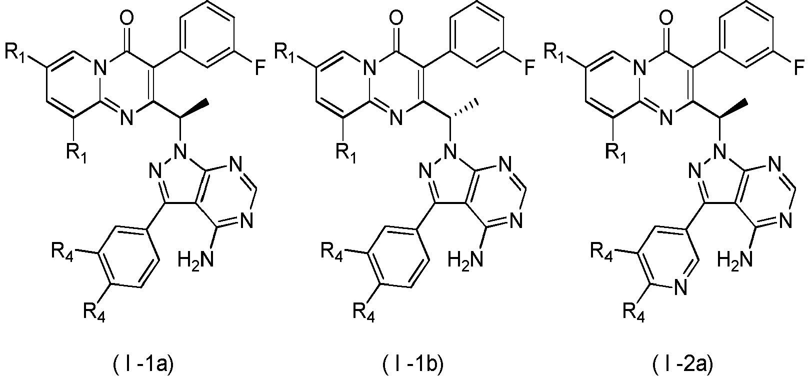 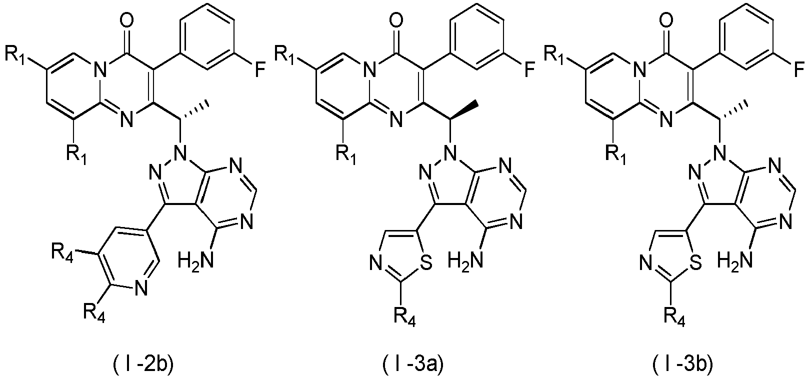 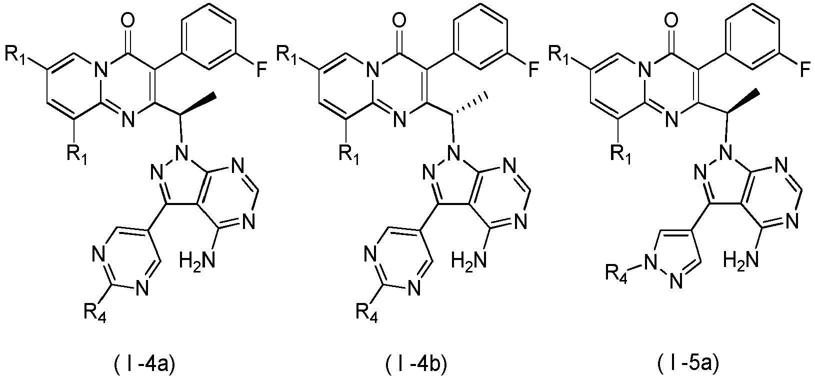 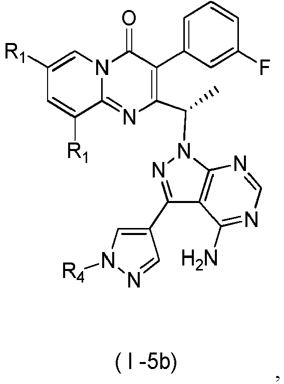 the isomer thereof or the pharmaceutically acceptable salt thereof,
wherein,
R₁ and R₄ are defined as herein.

The present invention also provides a compound, an isomer thereof or a pharmaceutically acceptable salt thereof, wherein the compound is selected from:

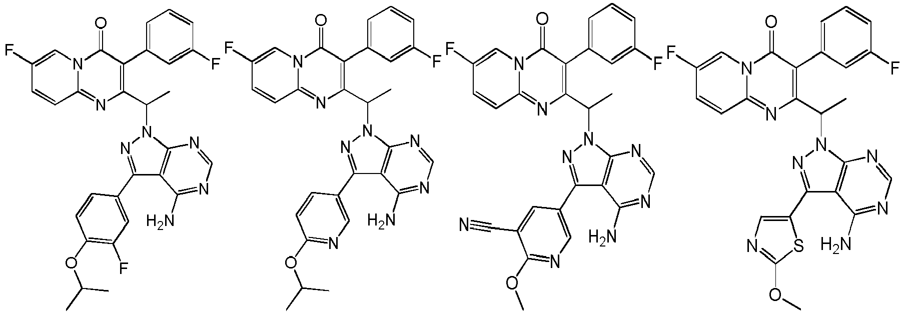

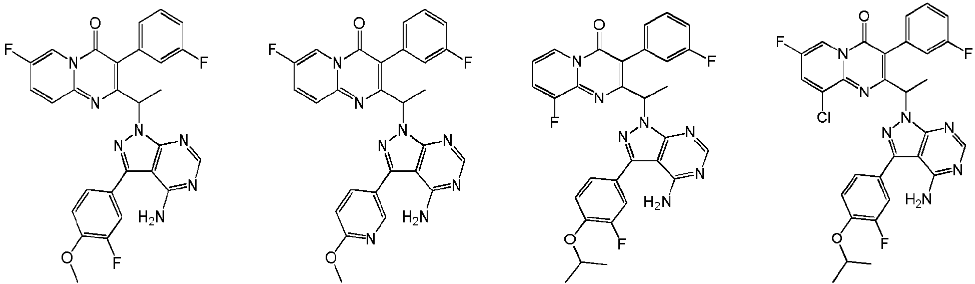

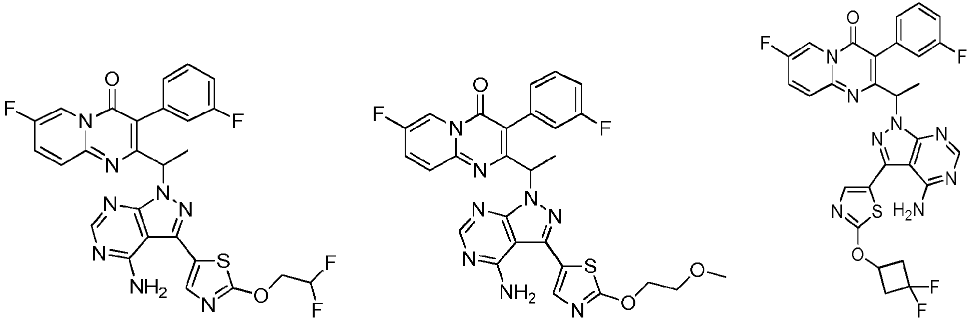

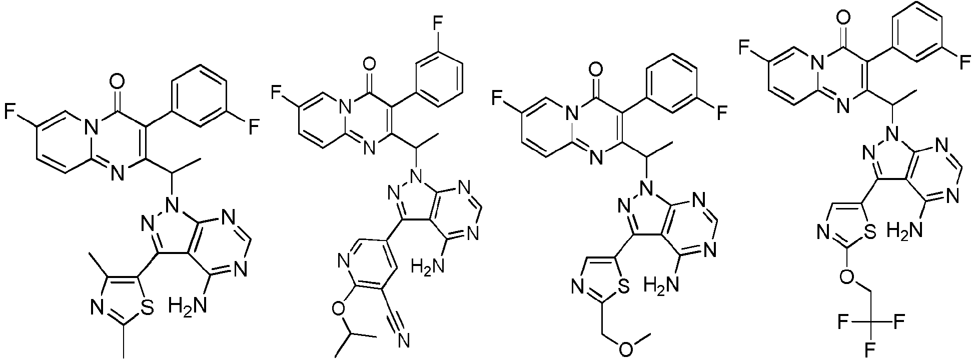

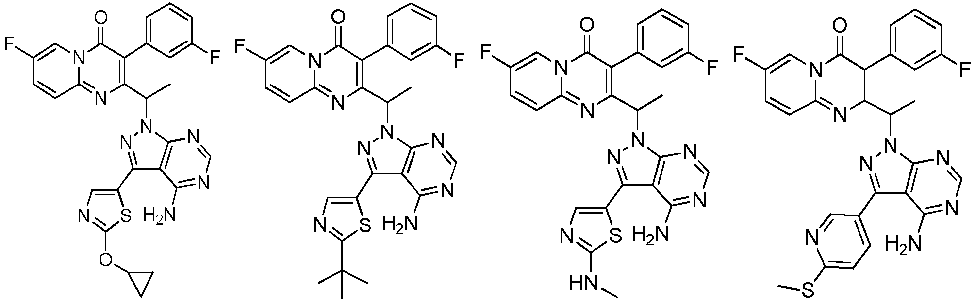

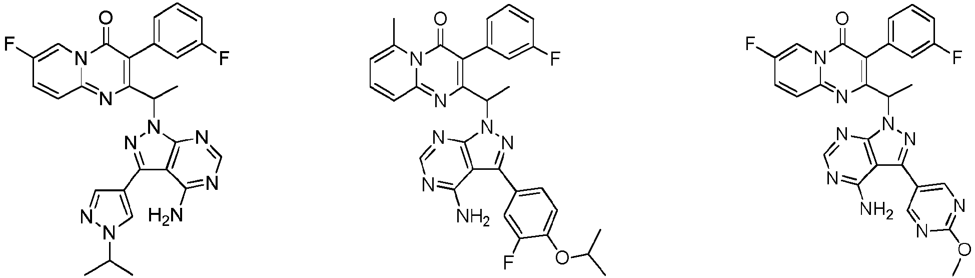

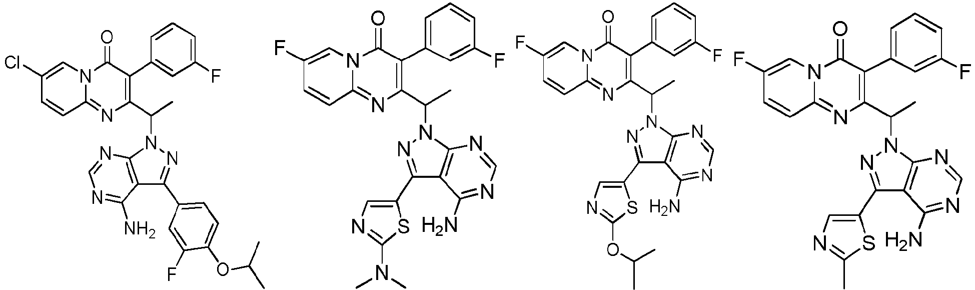

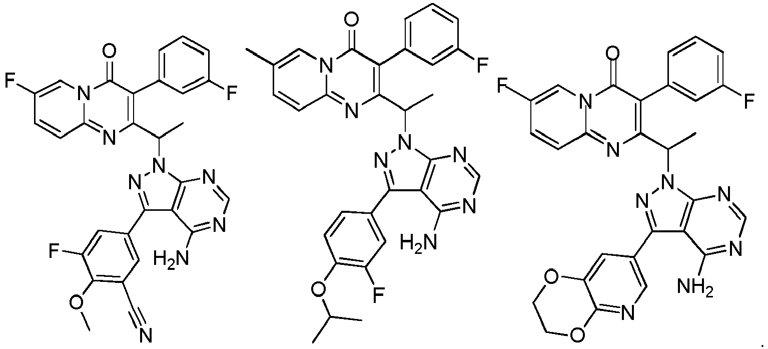

In some embodiments of the present invention, provided is the compound, selected from:

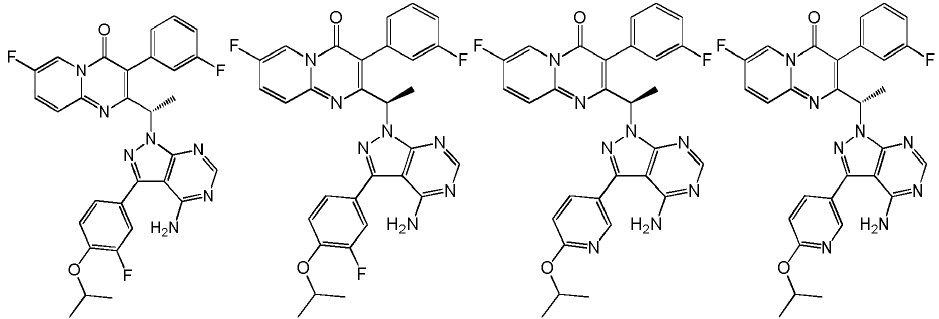

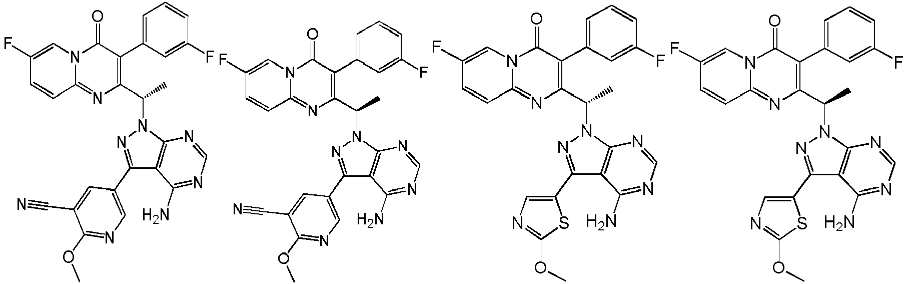

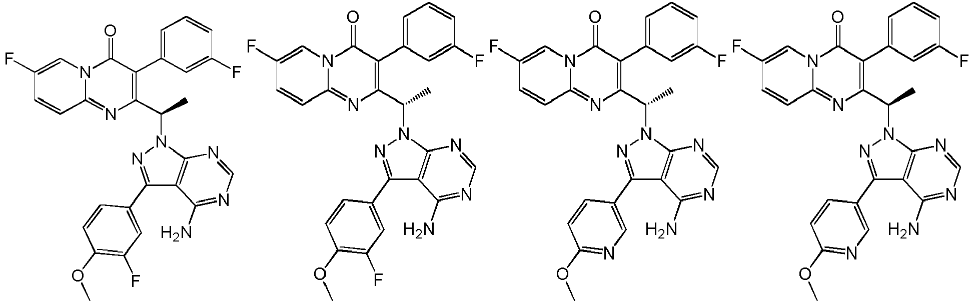

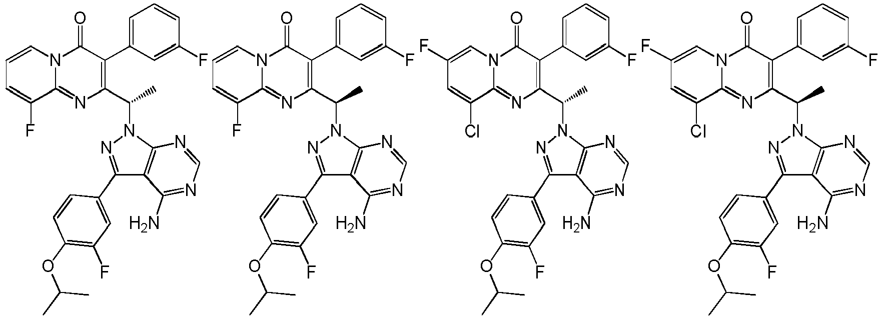

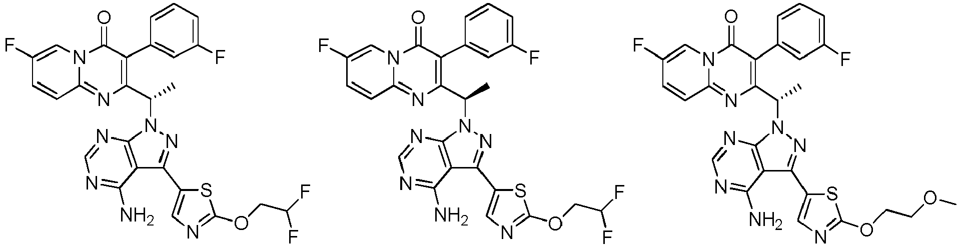

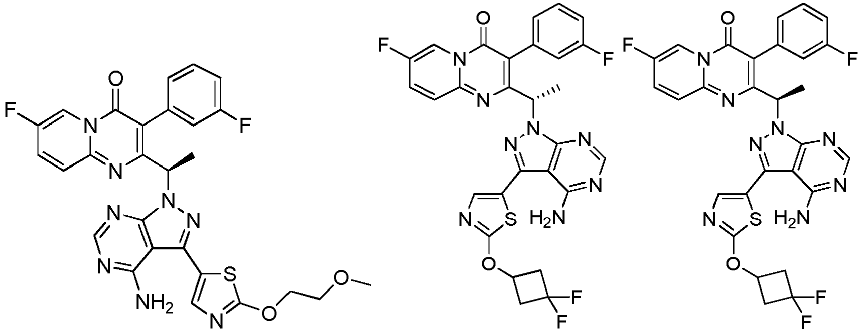

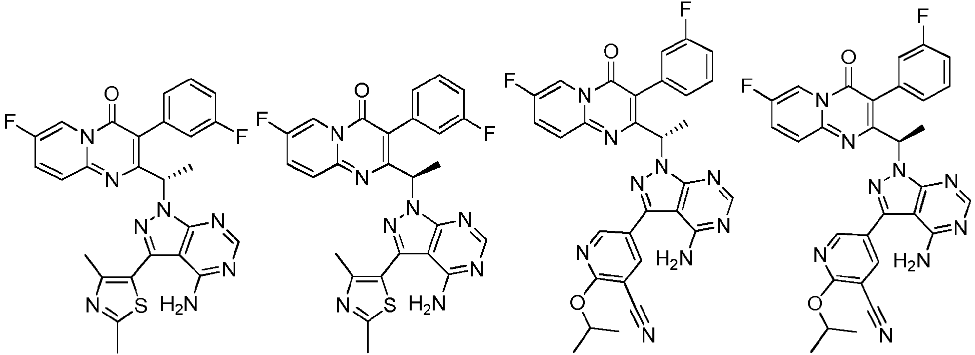

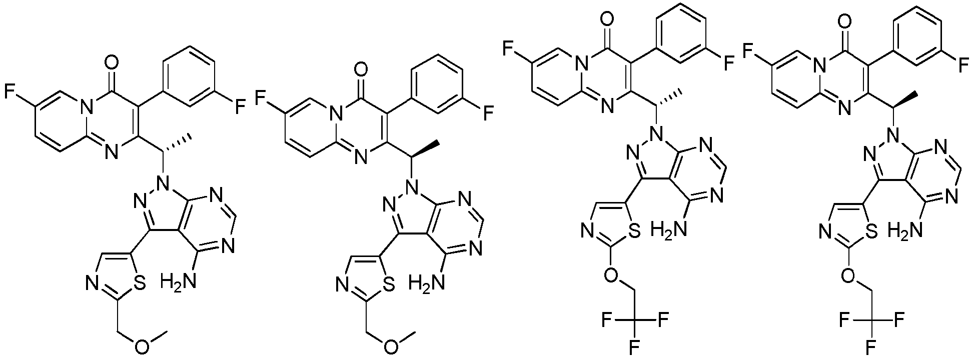

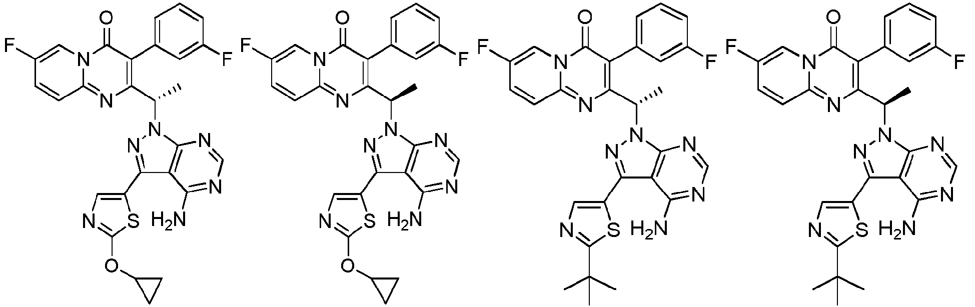

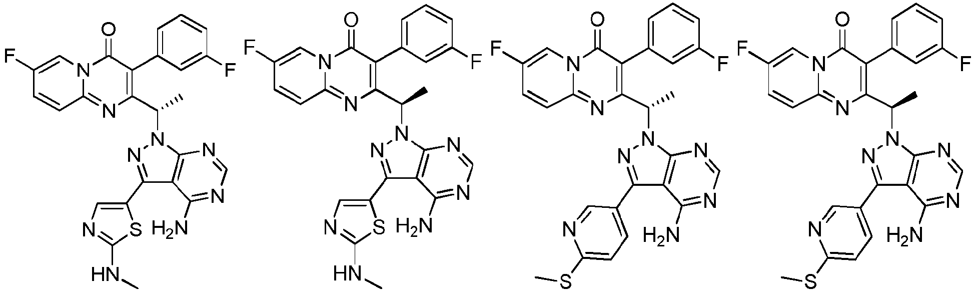

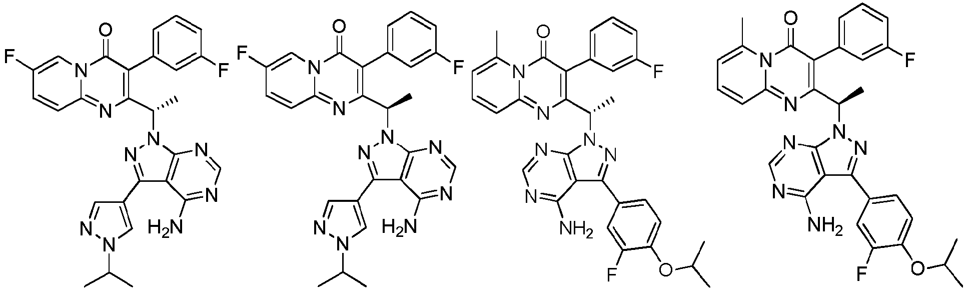

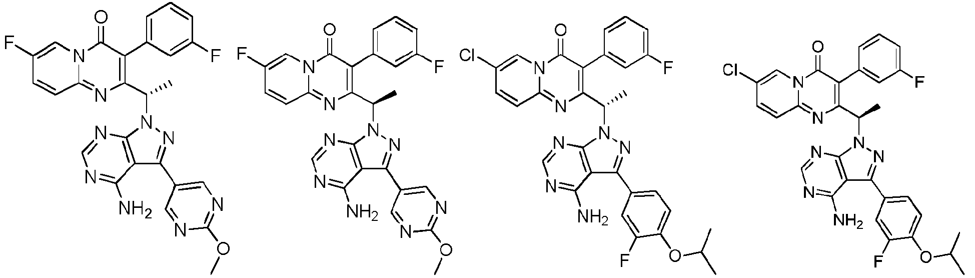

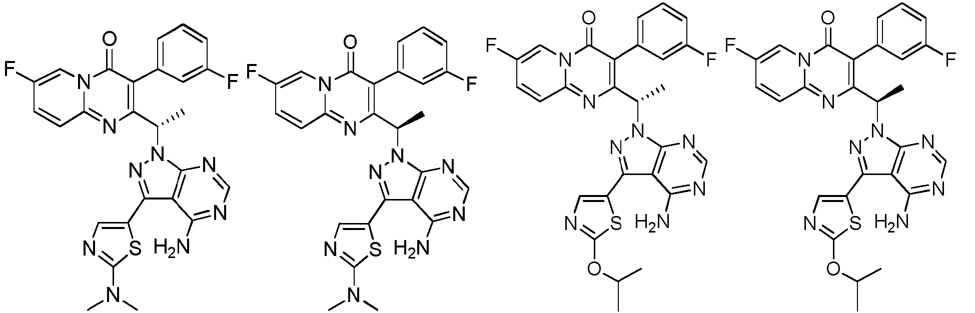

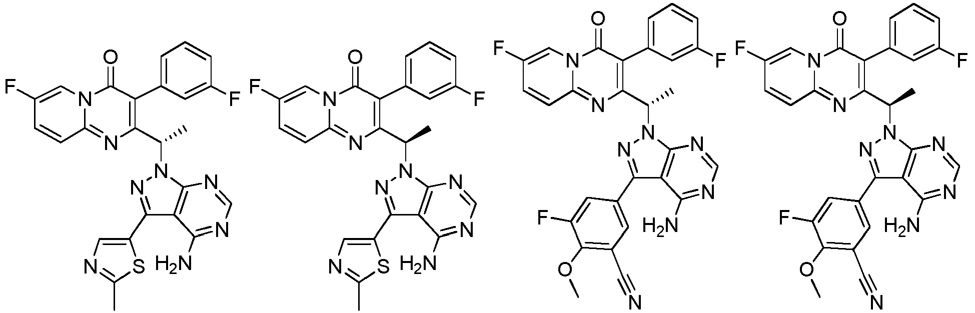

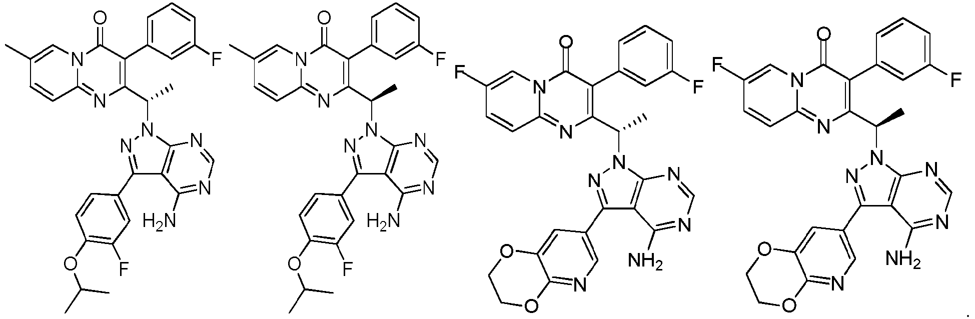

The present invention also provides use of the compound, the isomer thereof or the pharmaceutically acceptable salt thereof in preparing a medicament for the treatment ofa disease related to PI3K.

In some embodiments of the present invention, the medicament is a medicament for use in the treatment ofchronic lymphocytic leukemia, small lymphocytic lymphoma, marginal zone lymphoma, follicular lymphoma, mantle cell lymphoma, and diffuse large B-cell lymphoma.

### DEFINATION AND DESCRIPTION

Unless otherwise stated, the following terms and phrases used herein are intended to have the following meanings. A particular term or phrase, unless otherwise specifically defined, should not be considered as uncertain or unclear, but construed according to its ordinary meaning. When referring to a trade name, it is intended to refer to its corresponding commercial product or its active ingredient.

The term "pharmaceutically acceptable" is used herein for those compounds, materials, compositions, and/or dosage forms which are, within the scope of reliable medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of the compound of the invention, which is prepared from the compound having particular substituents disclosed herein and a relatively nontoxic acid or base. When the compounds of the invention contain a relatively acidic functional group, a base addition salt can be obtained by contacting the neutral form of such a compound with a sufficient amount of base in a pure solution or a suitable inert solvent. Pharmaceutically acceptable base addition salts include sodium, potassium, calcium, ammonium, organic amine, or magnesium salts, or similar salts. When the compounds of the invention contain a relatively basic functional group, an acid addition salt can be obtained by contacting the neutral form of such a compound with a sufficient amount of acid in a pure solution or a suitable inert solvent. Examples of pharmaceutically acceptable acid addition salts include salts derived from inorganic acids, such as hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and salts derived from organic acids, such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid, methanesulfonic acid and the like. Also included are salts of amino acids (*e.g*., arginine and the like) and salts of organic acids such as glucuronic acid. Certain specific compounds of the invention contain both basic and acidic functional groups that allow the compounds to be converted into any one ofbase or acid addition salts.

The pharmaceutically acceptable salt of the invention can be synthesized from the parent compound containing an acid radical or a basic group by conventional chemical methods. In general, such a salt is prepared by reacting the compounds in free acid or base form with a stoichiometric amount of the appropriate base or acid in water or an organic solvent or a mixture of the two.

The compounds disclosed herein can be in the form of a geometric isomer or stereoisomer. All such compounds are contemplated herein, including *cis-* and *trans-* isomers, (-)- and (+)-enantiomers, (R)- and (S)-enantiomers, diastereoisomers, (*D*)-isomers, (*L*)-isomers, and racemic mixtures and other mixtures thereof, such as an enantiomer or diastereoisomer enriched mixture, all of which are encompassed within the scope of the present invention. Substituents such as alkyl may have an additional asymmetric carbon atom. All these isomers and mixtures thereof are encompassed within the scope of the present invention.

Unless otherwise stated, the term "enantiomer" or "optical isomer" refers to stereoisomers that are mirror images of each other.

Unless otherwise stated, the term *"cis-*/*trans-* isomer" or "geometric isomer" results from the inability of a single bond of a ring carbon atom or a double bond to rotate freely.

Unless otherwise stated, the term "diastereoisomer" refers to stereoisomerswhosemolecules each have two or more chiral centers and are not mirror images of each other.

Unless otherwise stated, "(*D*)" or "(+)" stands for dextrorotation, "(*L*)" or "(-)" stands for levorotation, and "(*DL*)" or "(±)" stands for racemization.

Unless otherwise stated, the absolute configuration of a stereogenic center is represented by a wedged solid bond ( 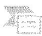 ) and a wedged dashed bond ( 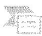 ), and the relative configuration of a stereogenic center is represented by a straight solid bond ( 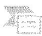 ) and a straight dashed bond ( 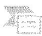 ). A wavy line ( 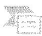 ) represents a wedged solid bond ( 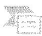 ) or a wedged dashed bond ( 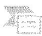 ), or a wavy line ( 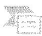 ) represents a straight solid bond ( 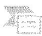 ) and a straight dashed bond ( 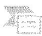 ).

The compounds of the invention may be present in particular form. Unless otherwise stated, the term "tautomer" or "tautomeric form" means that isomers with different functional groups are in dynamic equilibrium at room temperature and can be rapidly converted into each other. If a tautomer is possible (*e.g*., in solution), the chemical equilibrium of the tautomer can be reached. For example, a proton tautomer, also known as a prototropic tautomer, includes inter-conversion by proton transfer, such as keto-enol isomerization and imine-enamine isomerization. A valence isomer includes inter-conversion by recombination of some bonding electrons. A specific example of the keto-enol tautomerization is the inter-conversion between twotautomers pentane-2,4-dione and 4-hydroxypent-3-en-2-one.

Unless otherwise stated, the terms "enriched with one isomer", "isomer enriched", "enriched with one enantiomer" or "enantiomer enriched" mean that one of the isomers or enantiomers has a content less than 100%, for example, greater than or equal to 60%, or greater than or equal to 70%, or greater than or equal to 80% , or greater than or equal to 90% , or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise stated, the term "isomeric excess" or "enantiomeric excess" refers to the difference between the relative percentages of two isomers or enantiomers. For example, if the content of one isomer or enantiomer is 90%, and the content of the other isomer or enantiomer is 10%, the isomeric or enantiomeric excess (ee value) is 80%.

Optically active (*R*)- and (*S*)-isomers as well as *D*- and *L*-isomers can be prepared by chiral synthesis or chiral reagents or other conventional techniques. An enantiomer of certain compound of the invention can be prepared by asymmetric synthesis or derivatization using a chiral auxiliary, wherein the resulting diastereoisomeric mixture is separated and the auxiliary group is cleaved so as to give the desired pure enantiomer. Alternatively, when the molecule contains a basic functional group (such as amino) or an acidic functional group (such as carboxyl), the compound reacts with an appropriate optically active acid or base to form a salt of the diastereoisomer, which is then subjected to diastereoisomeric resolution through conventional methods in the art to obtain the pure enantiomer. Furthermore, the enantiomer and the diastereoisomer are generally isolated through chromatography using a chiral stationary phase, optionally in combination with chemical derivatization (*e.g*., carbamate formation from amines). The compounds disclosed herein may contain an unnatural proportion of atomic isotope at one or more of the atoms that constitute the compound. For example, the compound may be labeled with a radioisotope such as tritium (³H), iodine-125 (¹²⁵I) or C-14 (¹⁴C). For another example, hydrogen can be substituted with deuterium to form a deuterated medicament, and the bond formed by deuterium and carbon is firmer than that formed by common hydrogen and carbon. Compared with an un-deuterated medicament, the deuterated medicament has the advantages of reduced toxic side effect, increased stability, enhanced efficacy, prolonged biological half-life and the like. All isotopic variations of the compound described herein, whether radioactive or not, are encompassed within the scope of the present invention.

"Optional" or "optionally" means that the subsequently described event or circumstance may, but not necessarily, occur, and the description includes instances where the event or circumstance occurs and instances where the event or circumstance does not.

The term "substituted" means that any one or more hydrogen atoms on a specific atom are substituted with substituents which may include deuterium and hydrogen variants, as long as the valence of the specific atom is normal and the resulting compound is stable. When the substituent is oxygen (*i.e.*, =O), it means that two hydrogen atoms are substituted. Substitution by oxygen does not occur on aromatic groups. The term "optionally substituted" means that an atom may or may not be substituted with a substituent. Unless otherwise specified, the type and number of the substituent may be arbitrary as long as being chemically achievable.

When any variant (*e.g*., R) occurs more than once in the composition or structure of a compound, the definition of the variant in each case is independent. Thus, for example, if a group is substituted with0-2 R, the group can be optionally substituted with two R at most, and the definition of R in each case is independent. Furthermore, a combination of a substituent and/or a variant thereof is permissible only if the combination can result in a stable compound.

When the number of a connecting group is 0, such as -(CRR)₀-, it means that the connecting group is a single bond.

When a variant is selected from a single bond, it means that the two groups connected thereto are directly connected. For example, in A-L-Z, when L represents a single bond, it means that the structure is actually A-Z.

When a substituent is absent, it means that there is no such a substituent in a structure, *e.g*., when X is absent in A-X, it means that the structure is actually A. When it is not specified by which atom the listed substituent is connected to the group to be substituted, the substituent can be connected via any atom in the group. For example, pyridinyl as a substituent can be connected to a group to be substituted through any carbon atom on the pyridine ring. When the listed connecting group does not indicate the direction for connecting, the direction is arbitrary.

For example, when the connecting group L contained in 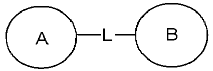 is -M-W-, -M-W- can either connect ring A and ring B to form 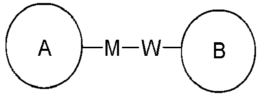 in a direction same as left-to-right reading order, or connect ring A and ring B to form 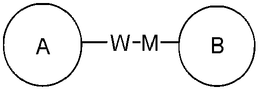 in a direction contrary to the left-to-right reading order. A combination of the connecting group, a substituent and/or a variant thereof is permissible only if the combination can result in a stable compound.

Unless otherwise specified, the number of atoms on a ring is generally defined as the number of ring members. For example, "5-7 membered ring" refers to a "ring" on which 5 to 7 atoms are arranged in a circle.

Unless otherwise specified, the term "C₁₋₆ alkyl" refers to a linear or branched saturated hydrocarbon group consisting of 1 to 6 carbon atoms. The C₁₋₆ alkyl includes C₁₋₅, C₁₋₄, C₁₋₃, C₁₋₂, C₂₋₆, C₂₋₄, C₆, C₅ alkyl and the like, and may be monovalent (*e.g.*, methyl), divalent (*e.g.*, methylene), or polyvalent (*e.g.*, methine group). Examples of C₁₋₆ alkyl include, but are not limited to, methyl (Me), ethyl (Et), propyl (including *n*-propyl and isopropyl), butyl (including *n*-butyl, isobutyl, *s*-butyl, and *t*-butyl), pentyl (including *n*-pentyl, isopentyl, and neopentyl), hexyl and the like.

Unless otherwise specified, the term "C₁₋₃ alkyl" refers to a linear or branched saturated hydrocarbon group consisting of 1 to 3 carbon atoms. The C₁₋₃ alkyl includes C₁₋₂ and C₂₋₃ alkyl, and the like, and may be monovalent (*e.g.*, methyl), divalent (*e.g*., methylene), or polyvalent (*e.g.*, methine group). Examples of C₁₋₃ alkyl include, but are not limited to, methyl (Me), ethyl (Et), propyl (including *n*-propyl and isopropyl) and the like.

Unless otherwise specified, the term "C₁₋₆ alkoxy" refers to those alkyl groups that each contains 1 to 6 carbon atoms and is connected to the rest part of the molecule viaan oxygen atom. The C₁₋₆alkoxy includes C₁₋₄, C₁₋₃, C₁₋₂, C₂₋₆, C₂₋₄, C₆, C₅, C₄ and C₃ alkoxy and the like. Examples of C₁₋₆ alkoxy include, but are not limited to, methoxy, ethoxy, propoxy (including *n*-propoxy and isopropoxy), butoxy (including *n*-butoxy, isobutoxy, *s*-butoxy and *t*-butoxy), pentyloxy (including *n*-pentyloxy, isopentyloxy and neopentyloxy), hexyloxy and the like.

Unless otherwise specified, the term "C₁₋₃ alkoxy" refers to those alkyl groups that each contains 1 to 3 carbon atoms and is connected to the rest part of the molecule viaan oxygen atom. The C₁₋₃ alkoxy includes C₁₋₂, C₂₋₃, C₃ and C₂ alkoxy and the like. Examples of C₁₋₃ alkoxy include, but are not limited to, methoxy, ethoxy, propoxy (including *n*-propoxy and isopropoxy) and the like.

Unless otherwise specified, the terms "5-6 membered heteroaromatic ring" and "5-6 membered heteroaryl" can be used interchangeably herein. The term "5-6 membered heteroaryl" refers to a monocyclic group which consists of 5 to 6 ring atoms and has a conjugated π-electron system, of which 1, 2, 3 or 4 ring atoms are heteroatoms independently selected from O, S, and N, the others being carbon atoms. The nitrogen atom is optionally quaternized, and the nitrogen and sulfur heteroatomscan beoptionally oxidized (*i.e.,* NO and S(O)ₚ, wherein p is 1 or 2). The 5-6 membered heteroaryl can be connected to the rest of the molecule through a heteroatom or a carbon atom. The 5-6 membered heteroaryl includes 5-membered heteroaryl and 6-membered heteroaryl. Examples of the 5-6 membered heteroaryl include, but are not limited to, pyrrolyl (including *N*-pyrrolyl, 2-pyrrolyl, 3-pyrrolyl and the like), pyrazolyl (including 2-pyrazolyl, 3-pyrazolyl, and the like), imidazolyl (including *N*-imidazolyl, 2-imidazolyl, 4-imidazolyl, 5-imidazolyl and the like), oxazolyl (including 2-oxazolyl, 4-oxazolyl, 5-oxazolyl and the like), triazolyl (including 1*H*-1,2,3-triazolyl, 2*H*-1,2,3-triazolyl, 1*H*-1,2,4-triazolyl, 4*H*-1,2,4-triazolyl and the like), tetrazolyl, isoxazolyl (3-isoxazolyl, 4-isoxazolyl, 5-isoxazolyl and the like), thiazolyl (including 2-thiazolyl, 4-thiazolyl, 5-thiazolyl and the like), furanyl (including 2-furanyl, 3-furanyl and the like), thienyl (including 2-thienyl, 3-thienyl and the like), pyridinyl (including 2-pyridinyl, 3-pyridinyl, 4-pyridinyl and the like), pyrazinyl, or pyrimidinyl (including 2-pyrimidinyl, 4-pyrimidinyl and the like).

Unless otherwise specified, the term "5-8 membered heterocycloalkenyl", by itself or in combination with other terms, refers to a partially unsaturated cyclic group containing 5 to 8 carbon atoms and at least one carbon-carbon double bond, of which 1, 2, 3 or 4 ring atoms are heteroatoms independently selected from O, S and N, with the remaining being carbon atoms. The nitrogen atom is optionally quaternized, and the nitrogen and sulfur heteroatoms can be optionally oxidized (*i.e.,* NO and S(O)ₚ, wherein p is 1 or 2). It includes monocyclic, bicyclic and tricyclic systems, wherein the bicyclic and tricyclic systems include spirocyclic, fused and bridged rings, any ring of which is non-aromatic. Furthermore, with respect to the "5-8 membered heterocycloalkenyl", a heteroatom may occupy the position where the heterocycloalkenyl is connected to the rest of the molecule. The 5-8 membered heterocycloalkenyl includes 5-7 membered, 5-6 membered, 4-5 membered, 4 membered, 5 membered, and 6 membered heterocycloalkenyl and the like. Examples of 5-8 membered heterocycloalkenyl include, but are not limited to, 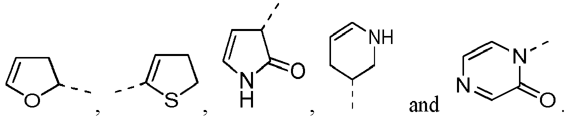 Unless otherwise specified, Cₙ₋ₙ₊ₘ or Cₙ-Cₙ₊ₘ includes any one of the specific cases of n to n+m carbons; for example, C₁₋₁₂ includes C₁, C₂, C₃, C₄, C₅, C₆, C₇, C₈, C₉, C₁₀, C₁₁ and C₁₂; Cₙ₋ₙ₊ₘ or Cₙ-Cₙ₊ₘ also includes any one of ranges in n to n+m; for example, C₁₋₁₂ includes C₁₋₃, C₁₋₆, C₁₋₉, C₃₋₆, C₃₋₉, C₃₋₁₂, C₆₋₉, C₆₋₁₂, C₉₋₁₂ and the like. Similarly, n-n+m membered represents that the number of atoms on the ring is n to n+m; for example, 3-12 membered ring includes 3 membered ring, 4 membered ring, 5 membered ring, 6 membered ring, 7 membered ring, 8 membered ring, 9 membered ring, 10 membered ring, 11 membered ring and 12 membered ring; n-n+m membered also represents any one of ranges in n to n+m; for example, 3-12 membered ring includes 3-6 membered ring, 3-9 membered ring, 5-6 membered ring, 5-7 membered ring, 6-7 membered ring, 6-8 membered ring, 6-10 membered ring and the like.

The term "leaving group" refers to a functional group or atom that can be substituted with another functional group or atom through a substitution reaction *(e.g.,* an affinity substitution reaction). For example, representative leaving groups include triflate; chlorine; bromine; iodine; sulfonate groups such as methanesulfonate, toluenesulfonate, *p*-bromobenzenesulfonate, and *p*-toluenesulfonate; and acyloxy groups such as acetoxy and trifluoroacetyloxy.

The term "protecting group" includes, but is not limited to, "amino protecting group", "hydroxyl protecting group" or "thiol protecting group". The term "amino protecting group" refers to a protecting group suitable for use in preventing side reaction at the amino nitrogen position. Representative amino protecting groups include, but are not limited to: formyl; acyl, for example alkanoyl (such as acetyl, trichloroacetyl or trifluoroacetyl); alkoxycarbonyl such as *t*-butoxycarbonyl (Boc); arylmethoxycarbonyl such as benzyloxycarbonyl (Cbz) and 9-fluorenylmethoxycarbonyl (Fmoc); arylmethyl such as benzyl (Bn), trityl (Tr), 1,1-bis-(4'-methoxyphenyl)methyl; silyl such as trimethylsilyl (TMS) and *t*-butyldimethylsilyl (TBS) and the like. The term "hydroxyl protecting group" refers to a protecting group suitable for use in preventing side reaction of a hydroxyl group. Representative hydroxyl protecting groups include, but are not limited to: alkyl groups such as methyl, ethyl and *t*-butyl; acyl groups such as alkanoyl (*e.g*., acetyl); arylmethyl groups such as benzyl (Bn), *p*-methoxybenzyl (PMB), 9-fluorenylmethyl (Fm) and diphenylmethyl (benzhydryl, DPM); silyl groups, such as trimethylsilyl (TMS) and *t*-butyldimethylsilyl (TBS) and the like.

The compounds disclosed herein can be prepared by a variety of synthetic methods well known to those skilled in the art, including the specific embodiments listed below, embodiments formed by combinations thereof with other chemical synthetic methods, and equivalents thereof known to those skilled in the art. Preferred embodiments include, but are not limited to, the examples disclosed herein.

The solvent used in the present invention can be commercially available. The following abbreviations are used in the present invention: aq for water; HATU for O-(7-azabenzotriazol-1-yl)-*N,N,N',N'*-tetramethyluronium hexafluorophosphate; EDC.HCl for *N*-(3-dimethylaminopropyl)-*N'*-ethylcarbodiimide hydrochloride; m-CPBA for 3-chloroperoxybenzoic acid; eq for equivalent; CDI for carbonyldiimidazole; DCM for dichloromethane; PE for petroleum ether; DIAD for diisopropyl azodicarboxylate; DMF for *N,N*-dimethylformamide; DMSO for dimethyl sulfoxide; EtOAc for ethyl acetate; EtOH for ethanol; MeOH for methanol; CBz for benzyloxycarbonyl, an amine protecting group; BOC for *t*-butoxycarbonyl, an amine protecting group; HOAc for acetic acid; NaCNBH₃ for sodium cyanoborohydride; r.t. for room temperature; O/N for overnight; THF for tetrahydrofuran; Boc₂O for di-*tert*-butyl dicarbonate; TFA for trifluoroacetic acid; DIPEA for diisopropylethylamine; SOCl₂ for thionyl chloride; CS₂ for carbon disulfide; TsOH for *p*-toluenesulfonic acid; NFSI for *N*-fluoro-*N*-(phenylsulfonyl)benzenesulfonamide; NBS for *N*-bromosuccinimide; -Bu₄NF for tetrabutylammonium fluoride; iPrOH for 2-propanol; mp for melting point; LDA for lithium diisopropylamide; PPA for polyphosphoric acid; PPh₃ for triphenylphosphine; Pd(PPh₃)₄ for tetrakis(triphenylphosphine)palladium(0); Pd(dppf)Cl₂ for [1,1'-bis(diphenylphosphino) ferrocene]dichloropalladium(II); Pd(dppf)Cl₂. CH₂Cl₂ for [1,1'-bis(diphenylphosphino)ferrocene]dichloropalladium(II) dichloromethane; TMEDA for *N*,*N*,*N*,*N*-tetramethylethylenediamine; and *n*-BuLi for *n*-butyllithium.

Compounds are named either manually or by ChemDraw® software, and supplier's catalog names are given for commercially available compounds.

### TECHNICAL EFFECTS

The compounds disclosed herein can well inhibit the activity of PI3K kinase, and has higher subtype selectivity for PI3Kα/β/γ. In addition, the phosphorylation level of Akt downstream of PI3K in cells can be well inhibited. The compounds disclosed herein exhibit high exposure, low clearance, and good oral bioavailability in mice.

### DETAILED DESCRIPTION

The present invention is described in detail below by way of examples. However, this is by no means disadvantageously limiting the scope of the present invention. Although the present invention has been described in detail herein and specific examples have also been disclosed, it will be apparent to those skilled in the art that various changes and modifications can be made to the specific examples without departing from the spirit and scope of the present invention.

### Reference Example 1: Fragment BB-1

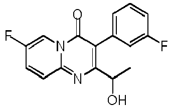

### Synthetic route:

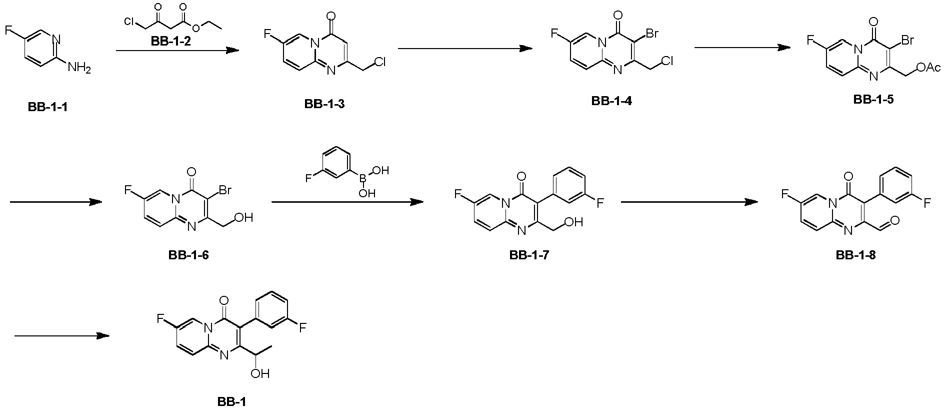

### Step 1: synthesis of compound BB-1-3

**BB-1-2** (43.90 g, 266.71 mmol, 1.3 *eq.)* was added to a solution of **BB-1-1** (23 g, 205.17 mmol, 1 *eq.)* in polyphosphoric acid (23 g, 17.84 mmol). The reaction system was stirred at 125 °C for 5 h under nitrogen atmosphere. After the reaction was completed, the reaction system was added with water (300 mL) to quench the reaction, and a solid was precipitated and directly filtered to give a filter cake. The filter cake was washed once with water (100 mL) and then purified by column chromatography (PE:EA = 1:1) to give the target compound **BB-1-3.** ¹H NMR (400MHz, CDCl₃) δ 8.94 (br s, 1H), 7.68 (br d, *J*=5.3 Hz, 2H), 6.65 (s, 1H), 4.51 (s, 2H).

### Step 2: synthesis of compound BB-1-4

NBS (19.36 g, 108.75 mmol, 1.1 *eq.)* was added to a solution of **BB-1-3** (21.02 g, 98.87 mmol, 1 *eq.*) in glacial acetic acid (210 mL). The reaction system was stirred at 25 °C for 1 h under nitrogen atmosphere. After the reaction was completed, the reaction system was added with water (200 mL) to quench the reaction, and a solid was produced and filtered to give a filter cake. After being washed with water (30 mL × 3), the filter cake was dissolved with dichloromethane (100 mL), dried over anhydrous sodium sulfate, concentrated, and slurried once with methyl *tert-butyl* ether (50 mL), and the filter cake was collected by filtration to give the target compound **BB-1-4,** batch 1. An aqueous phase was extracted with dichloromethane (100 mL × 3), combined with the mother solution obtaining by washing the methyl *tert*-butyl ether, and purified by column chromatography (petroleum ether:ethyl acetate = 1:1, target product Rf = 0.43) to give the target compound **BB-1-4**, batch 2. Two batches were dissolved and combined with dichloromethane, and the solvent was removed by rotary evaporation to give the target compound **BB-1-4.** ¹H NMR (400MHz, CDCl₃) δ 8.93 (dd, *J*=1.3, 3.1 Hz, 1H), 7.80 - 7.69 (m, 2H), 4.74 (s, 2H).

### Step 3: synthesis of compound BB-1-5

Potassium acetate (1.52 g, 15.44 mmol, 1.5 *eq*.) was added to a solution of **BB-1-4** (3 g, 10.29 mmol, 1 *eq.)* in *N,N*-dimethylformamide (30 mL). The reaction system was stirred at 40 °C for 3.5 h under nitrogen atmosphere. After the reaction was completed, the reaction system was added with water (60 mL) to quench the reaction, and a large amount of solid was precipitated and filtered to give a filter cake. The filter cake was dissolved with dichloromethane (100 mL), dried over anhydrous sodium sulfate and concentrated to give the target compound **BB-1-5**, batch 1. An aqueous phase was extracted with methyl *tert-butyl* ether (100 mL × 3) to give an organic phase, which was dried over anhydrous sodium sulfate and concentrated to give **BB-1-5** batch 2, and the two batches were combined, which was used directly in the next step. ¹H NMR (400MHz,CDCl₃) δ 9.07 - 8.88 (m, 1H), 7.71 (dd, *J*=1.7, 5.8 Hz, 2H), 5.31 - 5.26 (m, 2H), 2.22 (s, 3H).

### Step 4: synthesis of compound BB-1-6

Hydrochloric acid (12 M, 3.49 mL, 3.5 *eq*.) was added to a solution of **BB-1-5** (3.77 g, 11.96 mmol, 1 *eq.)* in dioxane (37 mL). The reaction system was stirred at 40 °C for 3.5 h under nitrogen atmosphere. After the reaction was completed, the reaction system was concentrated, added with water (2 mL) and adjusted to pH 9 with ammonia, and a cake was collected by filtration. The filter cake was dissolved with dichloromethane (100 mL), dried over anhydrous sodium sulfate and concentrated to give the target compound **BB-1-6,** which was used directly in the next step. ¹H NMR (400MHz, DMSO-*d*₆) δ 8.95 (dd, *J*=2.9, 4.6 Hz, 1H), 8.15 (dd, *J*=2.6, 7.0, 9.6 Hz, 1H), 7.86 (dd, *J*=5.3, 9.6 Hz, 1H), 5.35 (t, *J*=5.9 Hz, 1H), 4.58 (d, *J*=6.1 Hz, 2H).

### Step 5: synthesis of compound BB-1-7

Sodium carbonate (5.05 g, 47.61 mmol, 5 *eq.)* and Pd(PPh₃)₄ (550.15 mg, 476.09 µmol, 0.05 *eq.)* were added to a solution of **BB-1-6** (2.6 g, 9.52 mmol, 1 *eq.)* and 3-fluorobenzeneboronic acid (2.66 g, 19.04 mmol, 2 *eq.)* in acetonitrile/water (12.5 mL, volume ratio: 3/1). The reaction system was stirred at 85 °C for 4 h under nitrogen atmosphere. After the reaction was completed, the reaction system was added with dichloromethane (50 mL), then slowly added with water (5 mL) to quench the reaction, and the mixture was extracted with dichloromethane (50 mL × 3). The organic phases were combined, dried over anhydrous sodium sulfate, concentrated and purified by column chromatography (petroleum ether:ethyl acetate = 0:1) to give **BB-1-7.** ¹H NMR (400MHz, DMSO-*d*₆) δ 8.94 (dd, *J*=3.1, 4.8 Hz, 1H), 8.12 (ddd, *J*=2.6, 7.1, 10.0 Hz, 1H), 7.84 (dd, *J*=5.3, 10.1 Hz, 1H), 7.62 (s, 1H), 7.27 - 7.15 (m, 3H), 5.25 (t, *J*=5.9 Hz, 1H), 4.28 (d, *J*=5.7 Hz, 2H).

### Step 6: synthesis of compound BB-1-8

DMSO (2.28 g, 29.14 mmol, 2.28 mL, 6 *eq.)* was added to a solution of oxalyl chloride (1.85 g, 14.57 mmol, 1.28 mL, 3 *eq.)* in dichloromethane (20 mL) at -78 °C in a three-necked flask. The reaction system was stirred at -78 °C for 1 h under nitrogen atmosphere. A solution of **BB-1-7** (1.4 g, 4.86 mmol, 1 *eq.)* in dichloromethane (20 mL) was added, and the reaction system was stirred at -78 °C for 1 h. Triethylamine (4.91 g, 48.57 mmol, 6.76 mL, 10 *eq.)* was added, and the reaction system was stirred at -78 °C for 1 h and warmed to 25 °C and stirred for another 1 h. After the reaction was completed, the reaction system was added with dichloromethane (20 mL) at 0 °C, then slowly added with water (10 mL) to quench the reaction, and the mixture was extracted with dichloromethane (30 mL × 3). The organic phases were combined, dried over anhydrous sodium sulfate and concentrated to give compound **BB-1-8.** LCMS, m/z = 287.0 [M+1], Rt = 1.030 min.

### Step 7: synthesis of compound BB-1

Methylmagnesium bromide (3 M, 5.53 mL, 2.5 *eq.)* was added to a solution of **BB-1-8** (1.9 g, 6.64 mmol, 1 *eq.)* in tetrahydrofuran (50 mL) at 0 °C in a three-necked flask. The reaction system was stirred at 25 °C for 5 h under nitrogen atmosphere. After the reaction was completed, the reaction system was slowly added with water (10 mL) at 0 °C to quench the reaction, and the mixture was extracted with dichloromethane (10 mL × 3). The organic phases were combined, dried over anhydrous sodium sulfate, concentrated and purified by preparative high performance liquid chromatography (column: Phenomenex Luna C18 200 × 40 mm × 10 µm; mobile phase: [water (0.1% TFA)-acetonitrile]; B%: 15%-35%, 10 min) (B was acetonitrile) to give **BB-1.**

### Example 1: WX001 and WX002

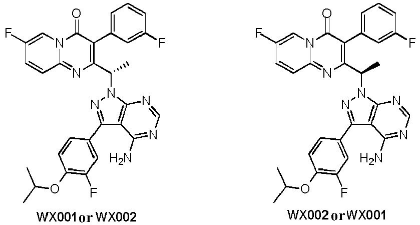

### Synthetic route:

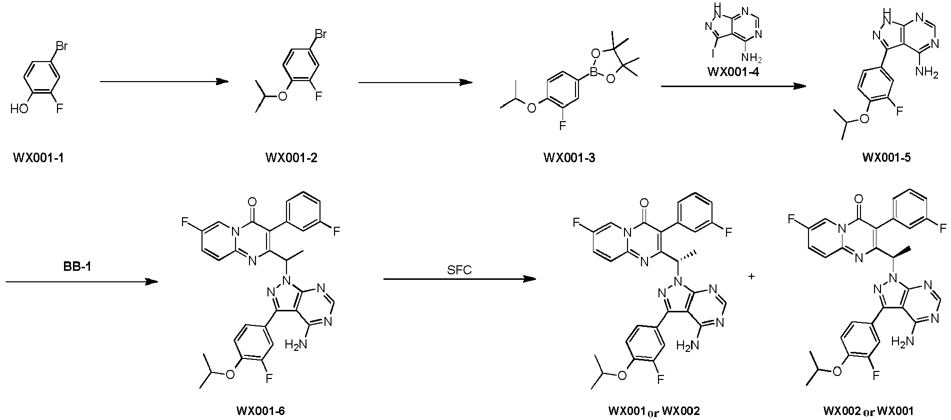

### Step 1: synthesis of compound WX001-2

2-iodopropane (3.56 g, 20.94 mmol, 2.09 mL, 2 *eq.)* was added to a solution of **WX001-1** (2 g, 10.47 mmol, 1 *eq.)* and potassium carbonate (4.34 g, 31.41 mmol, 3 *eq.)* in *N,N*-dimethylformamide (20 mL). The reaction system was stirred at 90 °C for 12 h under nitrogen atmosphere. After the reaction was completed, the reaction system was added with water (20 mL) to quench the reaction, and the mixture was extracted with methyl *tert-butyl* ether (10 mL × 3). The organic phases were combined, dried over anhydrous sodium sulfate, concentrated and purified by column chromatography (petroleum ether:ethyl acetate = 5:1) to give the target compound **WX001-2.** ¹H NMR (400MHz, CDCl₃) δ 7.23 (dd, J=2.4, 10.6 Hz, 1H), 7.16 (td, *J*=1.9, 8.8 Hz, 1H), 6.85 (t, *J*=8.7 Hz, 1H), 4.49 (spt, *J*=6.1 Hz, 1H), 1.35 (d, *J*=6.1 Hz, 6H).

### Step 2: synthesis of compound WX001-3

Bis(pinacolato)diboron (2.40 g, 9.44 mmol, 1.1 *eq*.), potassium acetate (1.68 g, 17.16 mmol, 2 *eq.)* and Pd(dppf)Cl₂ (627.87 mg, 858.09 µmol, 0.1 *eq*.) were added to a solution of **WX001-2** (2 g, 8.58 mmol, 1 *eq.)* in dioxane (20 mL). The reaction system was stirred at 90 °C for 3 h under nitrogen atmosphere. After the reaction was completed, the reaction system was added with water (20 mL) to quench the reaction, and the mixture was extracted with dichloromethane (30 mL × 3). The organic phases were combined, dried over anhydrous sodium sulfate and concentrated, and the crude product was purified by column chromatography (petroleum ether:ethyl acetate = 5:1) to give the target compound **WX001-3.** ¹H NMR (400MHz, CDCl₃) δ 7.54 - 7.44 (m, 2H), 6.95 (t, *J*=8.1 Hz, 1H), 4.60 (spt, *J*=6.1 Hz, 1H), 1.37 (s, 3H), 1.36 (s, 3H), 1.33 (s, 12H).

### Step 3: synthesis of compound WX001-5

Pd(PPh₃)₄ (546.55 mg, 472.97 µmol, 0.05 *eq*.) and sodium carbonate (3.01 g, 28.38 mmol, 3 *eq*.) were added to a solution of **WX001-3** (2.65 g, 9.46 mmol, 1 *eq*.) and **WX001-4** (2.47 g, 9.46 mmol, 1 *eq*.) in *N,N-*dimethylformamide/ethanol/water (265 mL, volume ratio: 2/1/1). The reaction system was stirred at 80 °C for 12 h under nitrogen atmosphere. After the reaction was completed, the reaction system was filtered while it was still hot (80 °C) to give mother solution. The mother solution was concentrated by rotary evaporation, added with dichloromethane (30 mL) and water (30 mL) to give a large amount of insoluble substances, and then filtered and purified by preparative high performance liquid chromatography to give the target compound **WX001-5.** LCMS, m/z= 288.1 [M+1], Rt =0.87 min.

### Step 4: synthesis of compound WX001-6

Diisopropyl azodicarboxylate (230.79 mg, 1.14 mmol, 221.91 µL, 1.5 *eq*.) was added to a solution of **BB-1** (230 mg, 760.90 µmol, 1 *eq.*), **WX001-5** (218.60 mg, 760.90 µmol, 1 *eq.*) and PPh₃ (299.36 mg, 1.14 mmol, 1.5 *eq.)* in tetrahydrofuran (50 mL) at 25 °C. The reaction system was stirred at 45 °C for 5 h under nitrogen atmosphere. After the reaction was completed, the reaction system was directly concentrated and purified by preparative thin layer chromatography (dichloromethane:methanol = 15:1) to give an isomer mixture **WX001-6.**

### Step 5: synthesis of compounds WX001 and WX002

**WX001-6** was purified by supercritical fluid chromatography (column: DAICEL CHIRALPAK AD-H (250 mm × 30 mm, 5 µm); mobile phase: [0.1% ammonia in ethanol]; B%: 22%-22%, 8 min (B was 0.1% ammonia in ethanol)) to give **WX001** (retention time: 2.29 min) and **WX002** (retention time: 2.62 min). **WX001:** ¹H NMR (400MHz, CD₃OD) δ 8.95 (br s, 1H), 8.03 (s, 1H), 8.00 - 7.92 (m, 1H), 7.91 - 7.82 (m, 1H), 7.42 - 7.29 (m, 2H), 7.27 - 7.09 (m, 1H), 7.22 (br t, *J*=8.6 Hz, 1H), 6.96 - 6.71 (m, 2H), 6.20 (q, *J*=6.6 Hz, 1H), 4.68 (td, *J*=6.1, 11.9 Hz, 1H), 1.91 (d, *J*=7.0 Hz, 3H), 1.36 (d, *J*=5.7 Hz, 6H), LCMS, m/z= 572.2 [M+1]. **WX002:** 1H NMR (400MHz, CD₃OD) δ 8.94 (br s, 1H), 8.03 (s, 1H), 7.99 - 7.91 (m, 1H), 7.90 - 7.83 (m, 1H), 7.39 - 7.28 (m, 2H), 7.21 (br t, *J*=8.3 Hz, 2H), 6.93 - 6.71 (m, 2H), 6.20 (q, *J*=6.9 Hz, 1H), 4.78 - 4.55 (m, 1H), 1.90 (d, *J*=6.6 Hz, 3H), 1.36 (d, *J*=6.1 Hz, 6H), LCMS, m/z= 572.2 [M+1].

### Example 2: WX003 and WX004

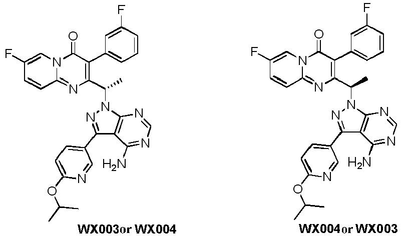

### Synthetic route:

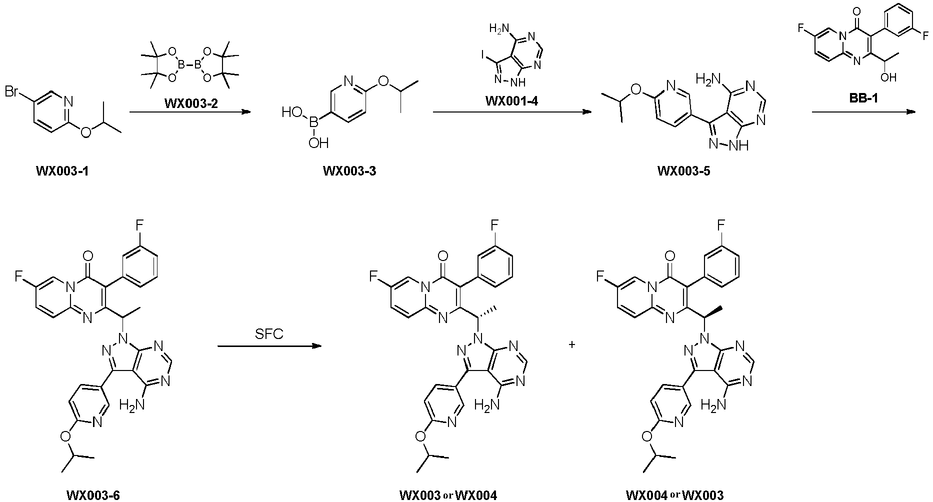

### Step 1: synthesis of compound WX003-3

**WX003-1** (0.2 g, 925.60 µmol, 1 *eq*.), **WX003-2** (0.24 g, 945.11 µmol, 1.02 *eq*.)*,* Pd(dppf)Cl₂ (0.034 g, 46.47 µmol, 5.02e-2 *eq*.) and potassium acetate (0.182 g, 1.85 mmol, 2 *eq*.) were dissolved in dioxane (6 mL), and the mixture was purged with nitrogen three times and then incubated at 80 °C for 16 h. After the reaction was completed, the reaction system was concentrated by rotary evaporation, diluted with water (50 mL) and extracted with dichloromethane (50 mL × 3). The organic phase was concentrated by rotary evaporation and purified by preparative thin layer chromatography (petroleum ether: ethyl acetate = 5:1) to give the target compound **WX003-3.** LCMS: m/z = 181.9 [M+1].

### Step 2: synthesis of compound WX003-5

**WX003-3** (0.13 g, 494.03 µmol, 1 *eq*.), **WX001-4** (0.13 g, 498.04 µmol, 1.01 *eq.)* and sodium carbonate (0.16 g, 1.51 mmol, 3.06 *eq*.) were dissolved in *N,N*-dimethylformamide (4 mL), ethanol (2 mL) and water (2 mL), and the reaction system was added with Pd(PPh₃)₄ (0.03 g, 25.96 µmol, 5.25e-2 *eq*.) under nitrogen atmosphere and stirred at 100 °C for 16 h. After the reaction was completed, the reaction system was diluted with water (50 mL) and extracted with dichloromethane (50 mL × 3). The organic phase was concentrated by rotary evaporation and purified by preparative thin layer chromatography (dichloromethane:methanol = 10:1) to give the target compound **WX003-5,** LCMS: m/z = 271.0 [M+1].

### Step 3: synthesis of compound WX003-6

**WX003-5** (0.05 g, 178.99 µmol, 1 *eq*.), **BB-1** (0.09 g, 281.78 µmol, 1.57 *eq*.) and PPh₃ (70.00 mg, 266.88 µmol, 1.49 *eq*.) were dissolved in tetrahydrofuran (4 mL), and the mixture was purged with nitrogen three times. Diisopropyl azodicarboxylate (52.00 mg, 257.16 µmol, 50.00 µL, 1.44 *eq*.) was added, and the mixture was stirred at 45 °C for 16 h. After the reaction was completed, the reaction system was concentrated by rotary evaporation and purified by preparative thin layer chromatography (dichloromethane:methanol = 10:1) to give the target compound **WX003-6.**

### Step 4: synthesis of compounds WX003 and WX004

**WX003-6** was separated by supercritical fluid chromatography (column: DAICEL CHIRALCEL OD-H (250 mm × 30 mm, 5 µm); mobile phase: [0.1% ammonia in ethanol]; B%: 45%-45%), (B was 0.1% ammonia in ethanol) to give the target compound **WX003** (retention time: 1.729 min) and **WX004** (retention time: 2.213 min). **WX003:** ¹H NMR (400 MHz, CDCl₃) δ 1.31 (d, *J*=6.27 Hz, 6 H) 1.86 (d, *J*=7.03 Hz, 3 H) 5.28 (br t, *J*=5.90 Hz, 2 H) 6.12 (q, *J*=6.78 Hz, 1 H) 6.74 (d, *J*=8.53 Hz, 1 H) 6.83 (br t, *J*=7.53 Hz, 2 H) 6.97 (br d, *J*=7.28 Hz, 1 H) 7.53 - 7.68 (m, 2 H) 7.77 (dd, *J*=8.53, 2.26 Hz, 1 H) 8.14 (s, 1 H) 8.35 (d, *J*=1.76 Hz, 1H) 8.87 (br d, *J*=2.76 Hz, 1H), LCMS: m/z= 555.1 [M+1]. **WX004:** 1H NMR (400 MHz, CDCl₃) δ 1.31 (d, *J*=6.27 Hz, 6 H) 1.87 (d, *J*=6.78 Hz, 3 H) 5.22 - 5.34 (m, 1 H) 5.50 (br s, 1 H) 6.13 (q, *J*=7.03 Hz, 1 H) 6.74 (d, *J*=8.53 Hz, 1 H) 6.84 (br t, *J*=7.53 Hz, 2 H) 6.92 - 7.02 (m, 1 H) 7.53 - 7.67 (m, 2 H) 7.76 (dd, *J*=8.53, 2.26 Hz, 1 H) 8.13 (s, 1H) 8.35 (d, *J*=2.26 Hz, 1 H) 8.83 - 8.93 (m, 1H), LCMS:m/z= 555.1 [M+1].

### Example 3: WX005 and WX006

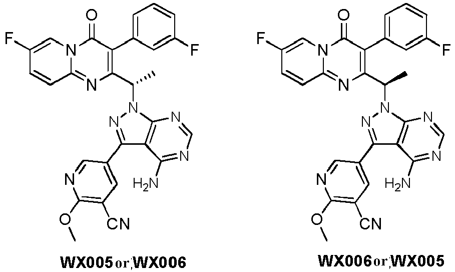

### Synthetic route:

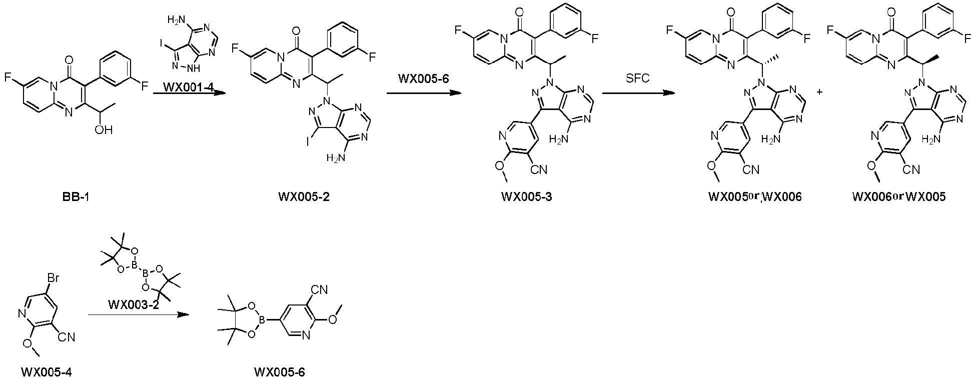

### Step 1: synthesis of compound WX005-2

**BB-1** (0.6 g, 1.98 mmol, 1 *eq.),* **WX001-4** (0.67 g, 2.57 mmol, 1.29 *eq.)* and PPh₃ (0.78 g, 2.97 mmol, 1.5 *eq.)* were added to tetrahydrofuran (40 mL), and the reaction system was added dropwise with diisopropyl azodicarboxylate (520.00 mg, 2.57 mmol, 0.5 mL, 1.30 *eq.)* and then incubated at 45 °C for 16 h. After the reaction was completed, the reaction system was filtered, and the filtrate was concentrated by rotary evaporation and purified by column chromatography (methanol:dichloromethane = 0%-5%) to give the target compound **WX005-2,** LCMS: m/z= 545.9 [M+1]; ¹H NMR (400 MHz, CDCl₃) δ 8.96 (dd, *J*=3.9, 2.6 Hz, 1 H), 8.18 (s, 1 H), 7.65 - 7.68 (m, 1 H), 7.53 - 7.61 (m, 1H), 7.29 - 7.35 (m, 1 H), 7.06 (d, *J*=7.8 Hz, 1 H), 6.93 - 7.02 (m, 2 H), 6.11 (q, *J*=7.0 Hz, 1 H), 5.85 (br s, 2 H), 1.90 (d, *J*=7.0 Hz, 3 H).

### Step 2: synthesis of compound WX005-6

**WX005-4** (5 g, 23.47 mmol, 1 *eq*.), **WX003-2** (8.94 g, 35.21 mmol, 1.5 *eq*.), Pd(dppf)Cl₂ (1.72 g, 2.35 mmol, 0.1 *eq.)* and potassium acetate (6.91 g, 70.41 mmol, 3 *eq.*) were added to dioxane (100 mL), and the reaction system was incubated at 100 °C for 40 min. After the reaction was completed, the reaction system was concentrated by rotary evaporation, diluted with water (100 mL) and extracted with dichloromethane (100 mL). The organic phase was concentrated by rotary evaporation to give the target compound **WX005-6**, which was directly used in the next step without being further purified. LCMS: m/z = 178.9 [M-81].

### Step 3: synthesis of compound WX005-3

**WX005-2** (0.13 g, 188.41 µmol, 1 *eq*.), **WX005-6** (0.1 g, 384.47 µmol, 2.04 *eq*.), Pd(PPh₃)₄ (0.043 g, 37.21 µmol, 1.97e-1 *eq.)* and sodium carbonate (0.059 g, 556.66 µmol, 2.95 *eq.)* were added to *N,N*-dimethylformamide (4 mL), ethanol (2 mL) and water (2 mL), and the reaction system was purged with ammonia 3 times and then incubated at 100 °C for 3 h. After the reaction was completed, the reaction system was concentrated by rotary evaporation, diluted with water (50 mL) and extracted with dichloromethane (50 mL × 2). The organic phase was concentrated by rotary evaporation, purified by preparative thin layer chromatography (dichloromethane:methanol = 15:1), and further purified by preparative high performance liquid (column: Boston Prime C18 150 × 30 mm 5 µm; mobile phase: [water (0.05% ammonium hydroxide v/v)-acetonitrile]; B%: 47%-77%, 8 min) (B was acetonitrile) to give the target compound **WX005-3.** LCMS: m/z= 552.0 [M+1].

### Step 4: synthesis of compounds WX005 and WX006

The compound **WX005-3** was separated by supercritical fluid chromatography (column: DAICEL CHIRALPAK AS-H (250 mm × 30 mm, 5 µm); mobile phase: [0.1% ammonia in methanol]; B%: 40%-40%, (B was 0.1% ammonia in methanol)) to give **WX005** (retention time: 1.713 min) and **WX006** (retention time: 1.945 min). **WX005:** ¹H NMR (400 MHz, DMSO-*d*₆) δ 8.90 - 8.97 (m, 1 H), 8.56 (d, *J*=2.3 Hz, 1 H), 8.36 (d, *J*=2.0 Hz, 1 H), 8.09 - 8.16 (m, 1 H), 8.06 (s, 1 H), 7.85 (dd, *J*=9.8, 5.3 Hz, 1H), 7.27 (br d, *J*=6.3 Hz, 1 H), 6.93 - 7.02 (m, 3 H), 6.11 (q, *J*=6.5 Hz, 1 H), 4.07 (s, 3 H), 1.81 (br d, *J*=6.8 Hz, 3 H); LCMS: m/z= 552.0 [M+1]. **WX006:** ¹H NMR (400 MHz, DMSO-*d*₆) δ 8.90 - 8.97 (m, 1 H), 8.56 (d, *J*=2.3 Hz, 1 H), 8.36 (d, *J*=2.0 Hz, 1 H), 8.09 - 8.16 (m, 1 H), 8.06 (s, 1 H), 7.85 (dd, *J*=9.8, 5.3 Hz, 1 H), 7.27 (br d, *J*=6.3 Hz, 1 H), 6.93 - 7.02 (m, 3 H), 6.11 (q, *J*=6.5 Hz, 1 H), 4.07 (s, 3 H), 1.81 (br d, *J*=6.8 Hz, 3 H); LCMS: m/z= 552.0 [M+1].

### Example 4: WX007 and WX008

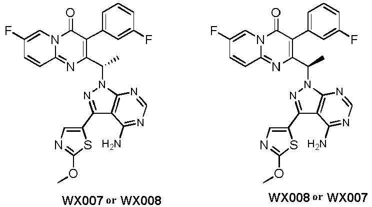

### Synthetic route:

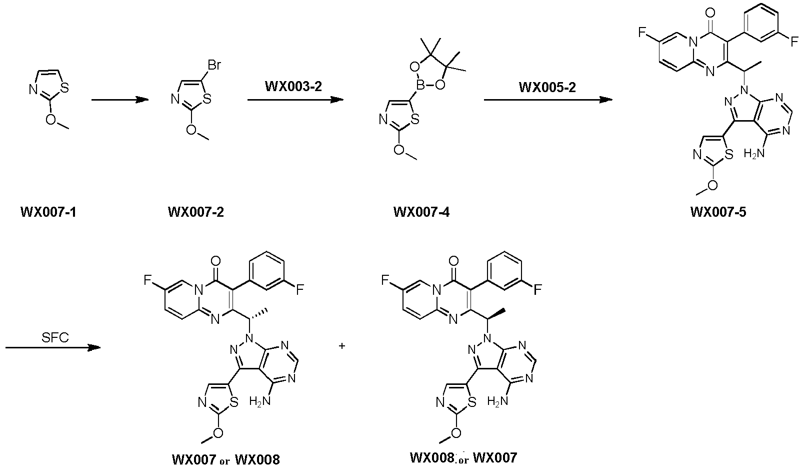

### Step 1: synthesis of compound WX007-2

**WX007-1** (10 g, 86.84 mmol, 1 *eq.)* and NBS (20.09 g, 112.89 mmol, 1.3 *eq*.) were added to dichloromethane (80 mL), and the reaction system was incubated at 15 °C for 16 h. After the reaction was completed, the reaction system was concentrated by rotary evaporation and then purified by silica gel column chromatography (EA:PE = 0%-5%) to give the target compound **WX007-2.** ¹H NMR (400 MHz, CDCl₃) δ 6.90 - 7.03 (m, 1 H), 3.92 - 4.01 (m, 3 H). LCMS: m/z= 195.8 [M+1].

### Step 2: synthesis of compound WX007-4

**WX007-2** (0.2 g, 1.03 mmol, 1 *eq.*), **WX003-2** (392.00 mg, 1.54 mmol, 1.5 *eq.*), Pd(dppf)Cl₂ (76.00 mg, 103.87 µmol, 1.01e-1 *eq.*) and potassium acetate (304.00 mg, 3.10 mmol, 3.01 *eq.*) were added to dioxane (5 mL), and the reaction system was purged with nitrogen 3 times and then incubated at 100 °C for 30 min. After the reaction was completed, the reaction system was concentrated by rotary evaporation to give the target compound **WX007-4,** which was directly used in the next step.

### Step 3: synthesis of compound WX007-5

**WX005-2** (0.2 g, 209.25. µmol, 1 *eq.*), **WX007-4** (300.00 mg, 1.24 mmol, 5.95 *eq.*), Pd(PPh₃)₄ (48.00 mg, 41.54 µmol, 1.99e-1 *eq*.) and sodium carbonate (66.00 mg, 622.71 µmol, 2.98 *eq*.) were added to *N,N*-dimethylformamide (6 mL), ethanol (3 mL) and water (3 mL), and the reaction system was purged with nitrogen 3 times and then incubated at 100 °C for 2.5 h. After the reaction was completed, the reaction system was concentrated by rotary evaporation, diluted with water (50 mL) and extracted with dichloromethane (50 mL). The organic phase was concentrated by rotary evaporation, purified by silica gel column chromatography (methanol:dichloromethane = 0%-10%), and further purified by preparative high performance liquid chromatography (column: Boston Prime C18 150 × 30 mm 5 µm; mobile phase: [water (0.05% ammonium hydroxide v/v)-acetonitrile]; B%: 50%-58%, 9 min) (B was acetonitrile) to give the target compound **WX007-5.** LCMS: m/z= 532.9 [M+1].

### Step 4: synthesis of compounds WX007 and WX008

The compound **WX007-5** was separated by supercritical fluid chromatography (column: DAICEL CHIRALPAK AS (250 mm × 30 mm, 10 µm); mobile phase: [0.1% ammonia in ethanol]; B%: 30%-30%, (B was 0.1% ammonia in ethanol)) to give **WX007** (retention time: 2.194 min) and **WX008** (retention time: 2.413 min). **WX007:** ¹H NMR (400 MHz, DMSO-*d*₆) δ 8.93 (dd, *J*=4.5, 2.8 Hz, 1 H), 8.12 (ddd, *J*=9.9, 7.1, 2.9 Hz, 1 H), 8.06 (s, 1 H), 7.81 (dd, *J*=9.8, 5.3 Hz, 1 H), 7.44 (s, 1 H), 7.28 (br d, *J*=6.5 Hz, 1 H), 6.99 (br t, *J*=7.4 Hz, 3 H), 6.05 (q, *J*=6.6 Hz, 1 H), 4.08 (s, 3 H), 1.78 (d, *J*=7.0 Hz, 3 H); LCMS: m/z= 533.1 [M+1]. **WX008:** ¹H NMR (400 MHz, DMSO-*d*₆) δ 8.93 (dd, *J*=4.5, 2.8 Hz, 1 H), 8.12 (ddd, *J*=9.9, 7.2, 2.8 Hz, 1 H), 8.06 (s, 1 H), 7.81 (dd, *J*=9.8, 5.3 Hz, 1 H), 7.44 (s, 1 H), 7.28 (br d, *J*=7.0 Hz, 1 H), 6.99 (br t, *J*=7.8 Hz, 3 H), 6.05 (q, *J*=6.8 Hz, 1 H), 4.08 (s, 3 H), 1.78 (d, *J*=6.8 Hz, 3 H); LCMS: m/z= 533.1 [M+1].

### Example 5: WX009 and WX010

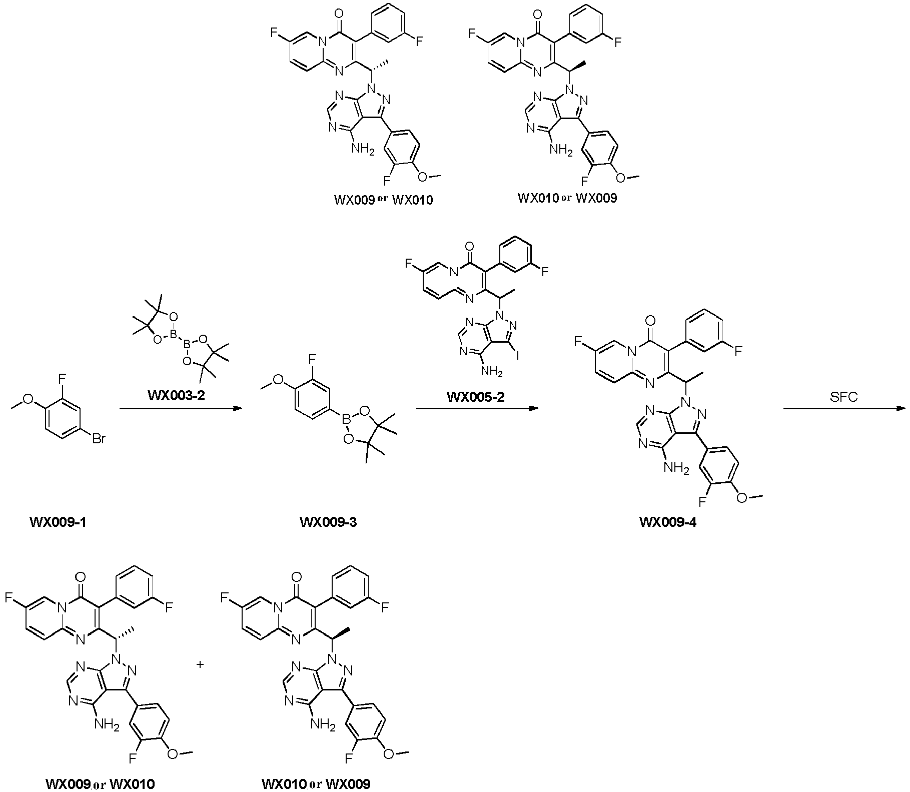

### Step 1: synthesis of compound WX009-3

**WX009-1** (2 g, 7.93 mmol, 1 *eq.)* was dissolved in dioxane (30 mL), then **WX003-2** (2.12 g, 8.33 mmol, 1.05 *eq.)* and potassium acetate (3.11 g, 31.73 mmol, 4 *eq.)* were added, and [1,1'-bis(diphenylphosphino)ferrocene] dichloropalladium(II) dichloromethane (323.95 mg, 396.68 µmol, 0.05 *eq.)* was added under nitrogen atmosphere. The mixture was heated to 80 °C and stirred for 3 h. After the reaction was completed, the reaction system was filtered, the filtrate was collected, the reaction was quenched with water (100 mL), and the organic solvent was removed by rotary evaporation and extracted with ethyl acetate (100 mL × 3). The organic phases were combined, dried over anhydrous sodium sulfate, filtered, and separated by silica gel column chromatography (petroleum ether:ethyl acetate = 20:1) to give the target compound **WX009-3.** ¹H NMR (400MHz, CD₃OD) δ 7.52 (s, 1H), 7.38 (dd, *J***=**1.3, 11.8 Hz, 1H), 7.19 - 7.02 (m, 1H), 3.97 - 3.89 (m, 3H), 1.37 - 1.30 (m, 12H).

### Step 2: synthesis of compound WX009-4

The compound **WX005-2** (50 mg, 91.70 µmol, 1 *eq.*) and **WX009-3** (23.12 mg, 91.70 µmol, 1 *eq.*) were dissolved in a mixed solvent of *N,N*-dimethylformamide (5 mL), ethanol (2.5 mL) and water (2.5 mL) before potassium carbonate (38.02 mg, 275.09 µmol, 3.0 *eq.*) was added. Under nitrogen atmosphere, Pd(PPh₃)₄ (21.19 mg, 18.34 µmol, 0.2 *eq*.) was added, and the reaction system was stirred at 100 °C for 2 h. After the reaction was completed, the reaction system was concentrated by rotary evaporation, added with water (10 mL) and extracted with dichloromethane (10 mL × 3). The organic phases were combined, dried over anhydrous sodium sulfate, filtered, separated by silica gel column chromatography (dichloromethane:methanol = 10:1), and further separated by preparative high performance liquid chromatography to give the target compound **WX009-4.**

### Step 3: synthesis of compounds WX009 and WX010

The compound **WX009-4** was separated by supercritical fluid chromatography (column: Chiralcel OD-3 100 mm × 4.6 mm I.D., 3 µm; mobile phase: A: CO₂ B: ethanol (0.05% DEA), elution gradient: ratio of mobile phase B from 5% to 40% over 4.5 min and maintained at 40% for 2.5 min, followed by 5% mobile phase B: ethanol; flow rate: 2.8 mL/min; column temperature: 40 °C) to give **WX009** (retention time: 4.939 min) and **WX010** (retention time: 4.631 min). **WX009:** ¹H NMR (400MHz, CDCl₃) δ 8.87 (dd, *J*=2.9, 3.9 Hz, 1H), 8.12 (s, 1H), 7.84 - 7.51 (m, 2H), 7.47 - 7.26 (m, 2H), 7.19 - 7.13 (m, 1H), 7.10 - 6.91 (m, 2H), 6.82 (m,2H), 6.12 (q, *J*=7.0 Hz, 1H), 5.72 - 5.11 (m, 2H), 3.88 (s, 3H), 1.86 (d, *J*=7.0 Hz, 3H); LCMS: m/z= 544.1[M+1]. **WX010:** ¹H NMR (400MHz, CDCl₃) δ 8.87 (dd, *J*=2.8, 4.0 Hz, 1H), 8.12 (s, 1H), 7.82 - 7.49 (m, 2H), 7.47 - 7.27 (m, 2H), 7.22 - 7.20 (m, 1H), 7.11 - 6.90 (m, 2H), 6.82 (m, 2H), 6.12 (q, J=7.0 Hz, 1H), 5.41 (m, 2H), 3.88 (s, 3H), 1.86 (d, J=6.8 Hz, 3H); LCMS: m/z= 544.1 [M+1].

### Example 6: WX011 and WX012

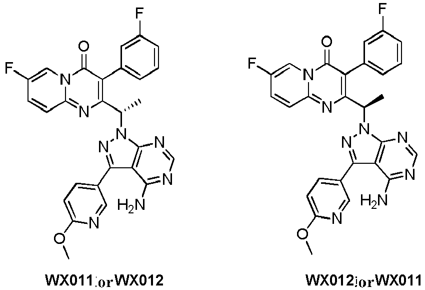

### Synthetic route:

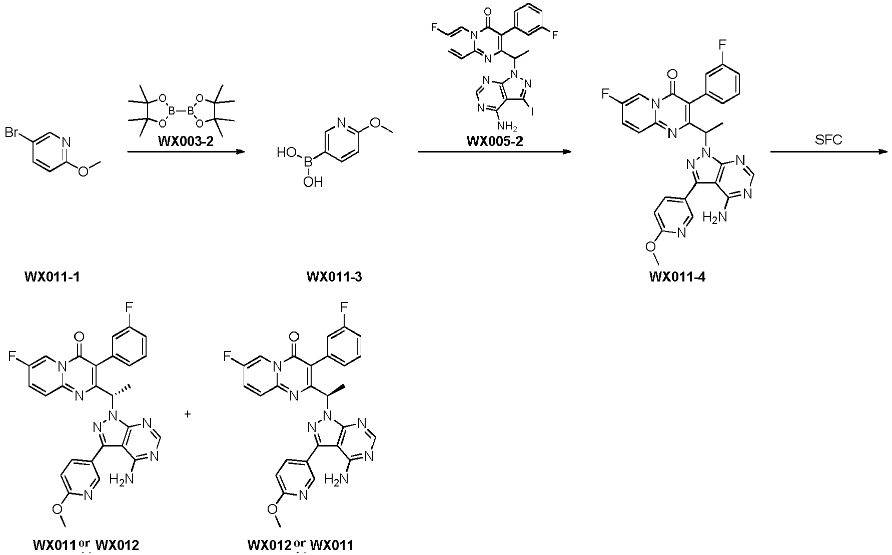

### Step 1: synthesis of compound WX011-3

**WX011-1** (1 g, 5.32 mmol, 689.66 µL, 1 *eq.*), **WX003-2** (1.35 g, 5.32 mmol, 1 *eq.*), Pd(dppf)Cl₂ (0.195 g, 266.50 µmol, 5.01e-2 *eq*.) and potassium acetate (1.04 g, 10.64 mmol, 2 *eq.*) were dissolved in dioxane (10 mL), and the mixture was purged with nitrogen three times and then incubated at 80 °C for 16 h. After the reaction was completed, the reaction system was concentrated by rotary evaporation and then purified by silica gel column chromatography (ethyl acetate:petroleum ether = 2%-8%) to give the target compound **WX011-3**. LCMS: m/z= 153.8 [M+1].

### Step 2: synthesis of compound WX011-4

**WX005-2** (0.2 g, 209.25 µmol, 1 *eq*.), **WX011-3** (40.03 mg, 261.76 µmol, 1.25 *eq*.) and sodium carbonate (0.08 g, 754.80 µmol, 3.61 *eq*.) were dissolved in *N,N*-dimethylformamide (4 mL), ethanol (2 mL) and water (2 mL), and the reaction system was added with Pd(PPh₃)₄ (40.00 mg, 34.62 µmol, 1.65e-1 *eq.*) under nitrogen atmosphere and stirred at 100 °C for 16 h. After the reaction was completed, the reaction system was concentrated by rotary evaporation and purified by preparative thin layer chromatography (dichloromethane:methanol = 10:1) to give the target compound **WX011-4**.

### Step 3: synthesis of compounds WX011 and WX012

The compound **WX011-4** was separated by supercritical fluid chromatography (column: DAICEL CHIRALCEL OD-H (250 mm × 30 mm, 5 µm); mobile phase: [0.1% ammonia in ethanol]; B%: 55%-55%, (B was 0.1% ammonia in ethanol)) to give **WX011** (retention time: 0.728 min) and **WX012** (retention time: 1.660 min). **WX011:** ¹H NMR (400 MHz, CDCl₃) δ 1.82 - 1.91 (m, 3 H) 3.92 (s, 3 H) 5.41 (br s, 2 H) 6.12 (q, *J*=6.86 Hz, 1 H) 6.73 - 6.88 (m, 3 H) 6.94 - 7.01 (m, 1 H) 7.52 - 7.66 (m, 2 H) 7.80 (dd, *J*=8.53, 2.26 Hz, 1 H) 8.13 (s, 1 H) 8.37 (d, *J*=2.01 Hz, 1 H) 8.81 - 8.93 (m, 1 H); LCMS: m/z= 527.1 [M+1]. **WX012:** ¹H NMR (400 MHz, CDCl₃) δ 1.86 (d, *J*=7.03 Hz, 3 H) 3.88 - 3.97 (m, 3 H) 5.42 (br s, 2 H) 6.13 (q, *J*=6.94 Hz, 1 H) 6.77 - 6.88 (m, 3 H) 6.90 - 7.04 (m, 1 H) 7.51 - 7.69 (m, 2 H) 7.80 (dd, *J*=8.53, 2.26 Hz, 1 H) 8.13 (s, 1 H) 8.37 (d, *J*=2.26 Hz, 1 H) 8.87 (dd, *J*=4.02, 2.76 Hz, 1 H); LCMS: m/z = 527.1 [M+1].

### Example 7: WX013

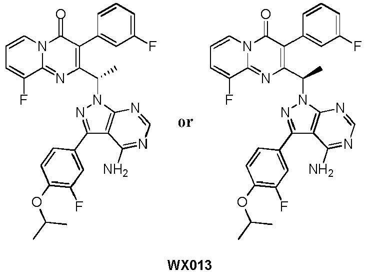

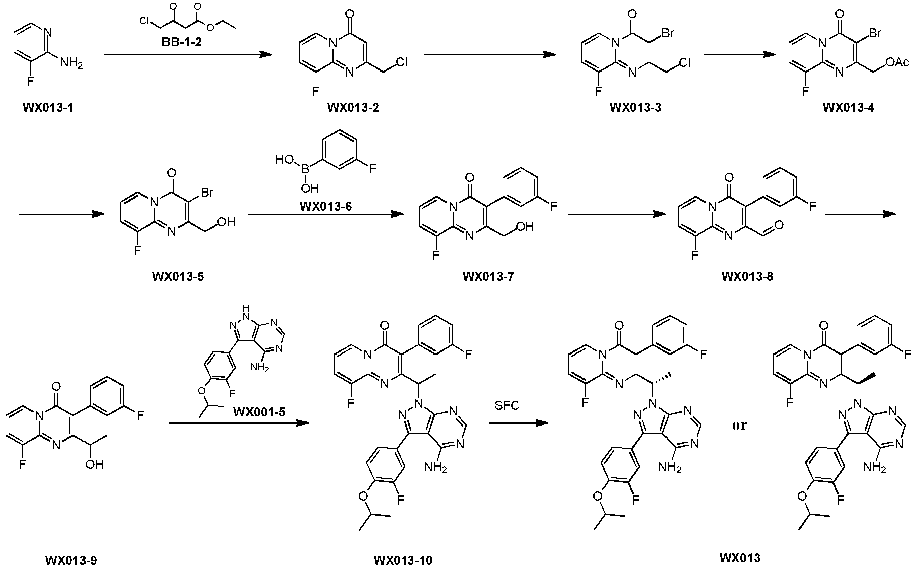

### Step 1: synthesis of compound WX013-2

**WX013-1** (9 g, 80.28 mmol, 1 *eq.*) and **BB-1-2** (17.18 g, 104.37 mmol, 1.3 *eq.)* were added to a pre-dried flask, followed by polyphosphoric acid (110 mL). The mixture was stirred at 125 °C for 12 h. After the reaction was completed, the reaction mixture was poured into water (1.5 L) while it was still hot, adjusted to pH 10 with 2.5 M sodium hydroxide and saturated sodium bicarbonate and extracted with ethyl acetate (500 mL × 3). The organic phases were combined, washed with saturated brine (1000 mL), dried over anhydrous sodium sulfate and concentrated under reduced pressure to give the target compound **WX013-2.** ¹H NMR (400MHz, CDCl₃) δ 8.78 (d, *J*=7.3 Hz, 1H), 7.50 - 7.40 (m, 1H), 7.04 (dt, *J*=4.6, 7.4 Hz, 1H), 6.68 (s, 1H), 4.51 (s, 2H).

### Step 2: synthesis of compound WX013-3

**WX013-2** (12 g, 56.44 mmol, 1 *eq.)* was added to a pre-dried flask, followed by glacial acetic acid (120 mL), and further followed by NBS (11.05 g, 62.09 mmol, 1.1 *eq.*). The mixture was stirred at 20 °C for 1 h. After the reaction was completed, water (100 mL) was added to the reaction system, and the filter cake was collected by filtration to give the target compound **WX013-3.** ¹H NMR (400MHz, CDCl₃) δ 8.79 (td, *J*=1.2, 7.3 Hz, 1H), 7.48 (dt, *J*=1.3, 8.1 Hz, 1H), 7.12 (dt, *J*=4.5, 7.5 Hz, 1H), 4.78 - 4.66 (m, 2H).

### Step 3: synthesis of compound WX013-4

**WX013-3** (10.5 g, 36.02 mmol, 1 *eq.)* was added to a flask, dissolved with *N,N*-dimethylformamide (120 mL), followed by the addition of potassium acetate (5.30 g, 54.03 mmol, 1.5 *eq.*). The reaction system was stirred at 20 °C for 12 h under nitrogen atmosphere. After the reaction was completed, the reaction system was diluted with water (100 mL)/ethyl acetate (100 mL), the organic phase was collected after liquid separation, and the aqueous phase was extracted with ethyl acetate (50 mL × 3). The organic phases were combined, washed with saturated brine (200 mL), dried over anhydrous sodium sulfate, and concentrated under reduced pressure to give the target compound **WX013-4,** which was directly used in the next step.

### Step 4: synthesis of compound WX013-5

**WX013-4** (4.1 g, 13.01 mmol, 1 *eq.*) was added to a pre-dried flask, followed by tetrahydrofuran (40 mL). The reaction system was cooled to 0 °C and added with a solution of lithium hydroxide monohydrate (1.64 g, 39.04 mmol, 3 *eq.)* in water (30 mL). The mixture was stirred at 20 °C for 1 h. After the reaction was completed, the reaction system was diluted with water (10 mL)/ethyl acetate (10 mL) and adjusted to pH 3 with 1 N hydrochloric acid, the organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and slurried with ethyl acetate:dichloromethane = 20:1 (20 mL), and the filter cake was collected by filtration to give **WX013-5.** ¹H NMR (400MHz, CDCl₃) δ 8.85 (td, J=1.2, 7.3 Hz, 1H), 7.60 - 7.48 (m, 1H), 7.19 - 7.10 (m, 1H), 4.71 (s, 2H), 4.07 - 3.79 (m, 1H).

### Step 5: synthesis of compound WX013-7

**WX013-5** (0.7 g, 2.28 mmol, 1 *eq.*), **WX013-6** (700.73 mg, 5.01 mmol, 2.2 *eq.)* and potassium acetate (670.22 mg, 6.83 mmol, 3 *eq*.) were added to a pre-dried flask, followed by dioxane (7 mL) and water (0.7 mL). The reaction system was purged with nitrogen 3 times and added with Pd(dppf)Cl₂ (166.57 mg, 227.64 µmol, 0.1 *eq.*). The mixture was stirred at 70 °C for 12 h. After the reaction was completed, the reaction system was diluted with water (10 mL)/ethyl acetate (10 mL), the organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and purified by silica gel thin layer chromatography (dichloromethane:methanol = 20:1) to give **WX013-7.** LCMS: m/z=289.0 [M+1].

### Step 6: synthesis of compound WX013-8

DMSO (1.20 g, 15.38 mmol, 1.20 mL, 6 *eq.)* was added to a solution of oxalyl chloride (976.18 mg, 7.69 mmol, 673.22 mL, 3 *eq.*) in dichloromethane (15 mL) at -78 °C in a flask. The reaction system was stirred at -78 °C for 1 h under nitrogen atmosphere. A solution of **WX013-7** (738.94 mg, 2.56 mmol, 1 *eq.*) in dichloromethane (15 mL) was added, and the reaction system was stirred at -78 °C for 1 h. Triethylamine (2.59 g, 25.64 mmol, 3.57 mL, 10 *eq.)* was added, and the reaction system was stirred at -78 °C for 1 h, warmed to 20 °C and stirred for another 0.5 h. After the reaction was completed, the reaction system was diluted with water (10 mL)/ethyl acetate (10 mL), the organic phase was collected after liquid separation, and the aqueous phase was extracted with ethyl acetate (5 mL × 3). The organic phase phases were washed with saturated brine (20 mL), dried over anhydrous sodium sulfate, and concentrated under reduced pressure to give the target compound **WX013-8**, which was directly used in the next step.

### Step 7: synthesis of compound WX013-9

**WX013-8** (624.81 mg, 2.18 mmol, 1 *eq.*) was added to a pre-dried flask, followed by tetrahydrofuran (7 mL), and further followed by methylmagnesium bromide (3 M, 2.18 mL, 3 *eq.*) at 0 °C. The mixture was stirred at 0 °C for 1 h. After the reaction was completed, the reaction system was diluted with saturated ammonium chloride (10 mL)/ethyl acetate (10 mL), the organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and purified by silica gel thin layer chromatography (PE:EA = 1:1) to give the target compound **WX013-9.** MS: m/z= 303.0 [M+1].

### Step 8: synthesis of compound WX013-10

Triphenylphosphine (329.73 mg, 1.26 mmol, 2 *eq*.) was added to a pre-dried flask, and then the mixture was dissolved with tetrahydrofuran (10 mL). The reaction system was cooled to 0 °C and added with diisopropyl azodicarboxylate (254.20 mg, 1.26 mmol, 244.43 µL, 2 *eq.*)*.* The reaction system was stirred at 0 °C for 0.5 h, the solution became turbid, and then a mixed solution of **WX013-9** (190 mg, 628.57 µmol, 1 *eq.*) and **WX001-5** (270.87 mg, 942.85 µmol, 1.5 *eq.*) in tetrahydrofuran (10 mL) was added at 0 °C. The reaction system was stirred at 20 °C for 3 h. After the reaction was completed, the reaction system was diluted with water (10 mL)/ethyl acetate (10 mL), the organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and purified by silica gel thin layer chromatography (dichloromethane:methanol = 20:1) to give **WX013-10.** MS: m/z= 572.1 [M+1].

### Step 9: synthesis of compound WX013

The compound **WX013-10** (130 mg, 227.45 µmol, 1 *eq.*) was separated by supercritical fluid chromatography (column: DAICEL CHIRALPAK AD-H (250 mm × 30 mm, 5 µm); mobile phase: [0.1% ammonia in isopropanol]; B%: 40%-40%, 7 min: 60 min) (B was 0.1% ammonia in isopropanol) to give the target compound **WX013** (retention time: 2.31 min) and enantiomer thereof (retention time: 2.87 min). ¹H NMR (400MHz, CD₃OD) δ 8.81 (br d, *J*=7.1 Hz, 1H), 8.03 (s, 1H), 7.74 (br t, *J*=8.4 Hz, 1H), 7.35 (s, 1H), 7.38 - 7.27 (m, 3H), 7.27 - 7.17 (m, 2H), 6.92 - 6.75 (m, 2H), 6.23 (q, *J*=6.9 Hz, 1H), 4.72 - 4.65 (m, 1H), 1.93 (d, *J*=7.1 Hz, 3H), 1.36 (dd, *J*=1.0, 6.1 Hz, 6H); **MS,** m/z= 572.1 [M+1].

### Example 8: WX014 and WX015

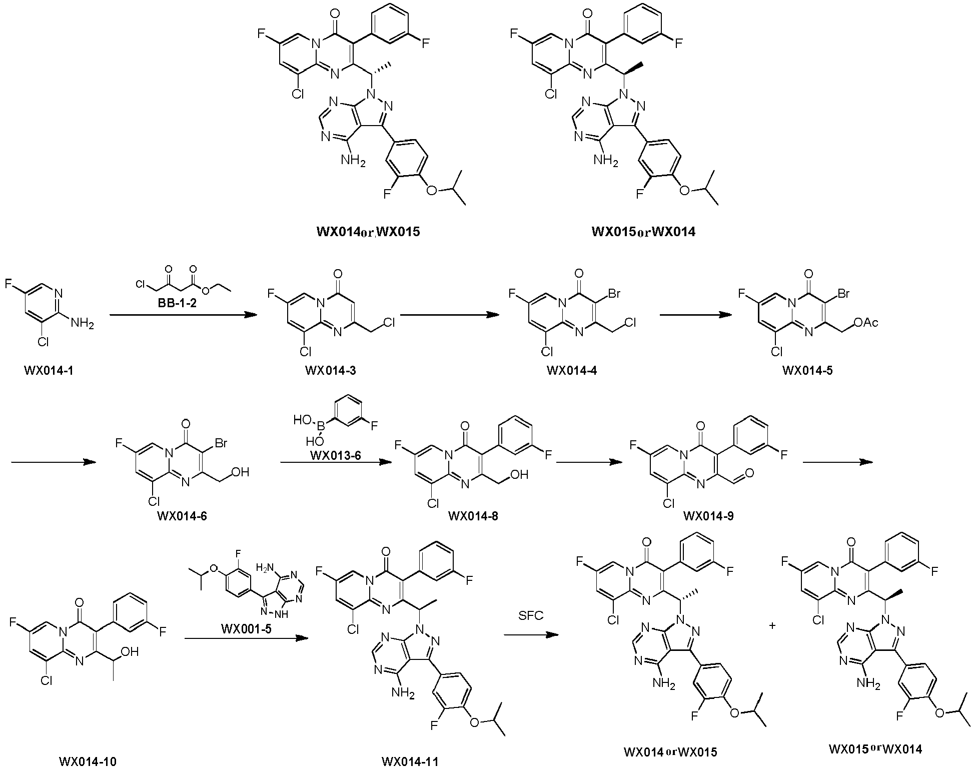

### Step 1: synthesis of compound WX014-3

**WX014-1** (10 g, 68.24 mmol, 1 *eq*.) and **BB-1-2** (14.60 g, 88.71 mmol, 1.3 *eq*.) were added to a pre-dried flask, followed by polyphosphoric acid (120 mL). The mixture was stirred at 125 °C for 12 h. After the reaction was completed, the reaction system was poured intowater (1500 mL) while it was still hot, adjusted to pH 10 with 2.5 N sodium hydroxide and saturated sodium bicarbonate, and extracted with dichloromethane (300 mL × 3). The organic phases were combined, washed with saturated brine (1000 mL), dried over anhydrous sodium sulfate, and concentrated under reduced pressure to give the target compound **WX014-3,** which was directly used in the next step. ¹H NMR (400MHz, CDCl₃) δ 8.85 (dd, *J*=2.8, 4.1 Hz, 1H), 7.81 (dd, *J*=2.8, 6.7 Hz, 1H), 6.69 (s, 1H), 4.53 (s, 2H).

### Step 2: synthesis of compound WX014-4

**WX014-3** (13.5 g, 54.64 mmol, 1 *eq.)* was added to a pre-dried flask, followed by glacial acetic acid (130 mL), and further followed by NBS (10.70 g, 60.11 mmol, 1.1 *eq.).* The mixture was stirred at 20 °C for 1 h. After the reaction was completed, water (100 mL) was added, and the filter cake was collected by filtration to give the target compound **WX014-4,** which was used directly in the next step. ¹H NMR (400MHz, CDCl₃) δ 8.91 (dd, *J*=2.6, 4.4 Hz, 1H), 7.90 (dd, *J*=2.6, 7.0 Hz, 1H), 4.79 (s, 2H).

### Step 3: synthesis of compound WX014-5

**WX014-4** (12 g, 36.82 mmol, 1 *eq*.) was added to a pre-dried flask, followed by *N,N*-dimethylformamide (5 mL), and further followed by potassium acetate (5.42 g, 55.22 mmol, 1.5 *eq*.)*.* The mixture was stirred at 20 °C for 12 h. After the reaction was completed, the reaction system was diluted with water (10 mL)/ethyl acetate (10 mL), the organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and slurried with ethyl acetate:methyl*tert*-butyl ether = 1:10 (150 mL), and the filter cake was collected by filtration to give the target compound **WX014-5.** ¹H NMR (400MHz, CDCl₃) δ 8.85 (dd, *J*=2.7, 4.3 Hz, 1H), 7.82 (dd, *J*=2.7, 6.7 Hz, 1H), 5.28 - 5.16 (m, 2H), 2.26 - 2.15 (m, 3H).

### Step 4: synthesis of compound WX014-6

**WX014-5** (9 g, 25.75 mmol, 1 *eq.*) was added to a pre-dried flask, and the reaction system was dissolved with dioxane (90 mL). Then hydrochloric acid (12 M, 15.02 mL, 7 *eq.*) was then added at 0 °C. The reaction system was stirred at 50 °C for 12 h. After the reaction was completed, the reaction system was diluted with water (10 mL)/ethyl acetate (10 mL), the organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and slurried with ethyl acetate:dichloromethane = 10:1 (15 mL), and the filter cake was collected by filtration to give the target compound **WX014-6.** ¹H NMR (400MHz, CDCl₃) δ 8.98 (dd, *J*=2.6, 4.2 Hz, 1H), 7.95 (dd, *J*=2.8, 6.7 Hz, 1H), 7.26 (s, 1H), 4.78 (s, 2H).

### Step 5: synthesis of compound WX014-8

**WX014-6** (3 g, 9.76 mmol, 1 *eq.),* **WX013-6** (1.64 g, 11.71 mmol, 1.2 *eq.)* and potassium acetate (2.87 g, 29.27 mmol, 3 *eq.)* were added to a pre-dried flask, followed by dioxane (30 mL) and water (3 mL). The reaction system was purged with nitrogen 3 times and added with Pd(dppf)Cl₂ (713.86 mg, 975.60 µmol, 0.1 *eq.*)*.* The mixture was stirred at 50 °C for 3 h. **WX013-6** (1.64 g, 11.71 mmol, 1.2 *eq.*) was supplemented and the reaction system was stirred at 50 °C for 12 h. After the reaction was completed, the reaction system was diluted with water (10 mL)/ethyl acetate (10 mL), the organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and separated and purified by silica gel column chromatography (dichloromethane:methanol = 500:1 to 50:1) to give the target compound **WX014-8.**

### Step 6: synthesis of compound WX014-9

DMSO (2.06 g, 26.34 mmol, 2.06 mL, 6 *eq.*) was added to a solution of oxalyl chloride (1.67 g, 13.17 mmol, 1.15 mL, 3 *eq*.) in dichloromethane (20 mL) at -78 °C in a flask. The reaction system was stirred at -78 °C for 1 h under nitrogen atmosphere. A solution of **WX014-8** (1.42 g, 4.39 mmol, 1 *eq.*) in dichloromethane (20 mL) was added, and the reaction system was stirred at -78 °C for 1 h. Triethylamine (4.44 g, 43.90 mmol, 6.11 mL, 10 *eq.*) was added, and the reaction system was stirred at -78 °C for 1 h, warmed to 20 °C, and stirred for another 0.5 h. After the reaction was completed, the reaction system was diluted with water (10 mL)/ethyl acetate (10 mL), the organic phase was collected after liquid separation, and the aqueous phase was extracted with ethyl acetate (5 mL × 3). The organic phases were combined, washed with saturated brine (20 mL), dried over anhydrous sodium sulfate, and concentrated under reduced pressure to give the target compound **WX014-9**, which was directly used in the next step.

### Step 7: synthesis of compound WX014-10

**WX014-9** (1.35 g, 4.21 mmol, 1 *eq*.) was added to a pre-dried flask, followed by tetrahydrofuran (20 mL). The reaction system was cooled to 0 °C, then methylmagnesium bromide solution (3 M, 4.21 mL, 3 *eq*.) was added slowly. The mixture was stirred at 0 °C for 1 h. After the reaction was completed, the reaction system was diluted with saturated ammonium chloride (50 mL)/ethyl acetate (50 mL), the organic phase was collected after liquid separation, and the aqueous phase was extracted with ethyl acetate (15 mL × 3). The organic phases were combined, washed with saturated brine (20 mL), dried over anhydrous sodium sulfate, concentrated under reduced pressure, and purified by silica gel thin layer chromatography (petroleum ether:ethyl acetate = 1:1) to give the target compound **WX014-10.**

### Step 8: synthesis of compound WX014-11

PPh₃ (525.02 mg, 2.00 mmol, 2 *eq*.) was added to a pre-dried flask, and then the mixture was dissolved with tetrahydrofuran (4 mL). The reaction system was purged with nitrogen three times, cooled to 0 °C, and added with diisopropyl azodicarboxylate (404.76 mg, 2.00 mmol, 389.19 µL, 2 *eq.*)*.* After the reaction system was stirred at 0 °C for 0.5 h, the solution became turbid. Then a mixed solution of **WX014-10** (337 mg, 1.00 mmol, 1 *eq*.) and **WX001-5** (431.30 mg, 1.50 mmol, 1.5 *eq*.) in tetrahydrofuran (4 mL) was added at 0 °C. The reaction system was stirred at 20 °C for 3 h. After the reaction was completed, the reaction system was diluted with water (10 mL)/ethyl acetate (10 mL), the organic phase was collected after liquid separation, and the aqueous phase was extracted with ethyl acetate (5 mL × 3). The organic phases were combined, washed with saturated brine (20 mL), dried over anhydrous sodium sulfate, concentrated under reduced pressure, and purified by silica gel thin layer chromatography (dichloromethane:methanol = 20:1), and separated by preparative high performance liquid chromatography (column: Xbridge Prep OBD C18 150 × 40 mm, 10 µm; mobile phase: [water (10 mM NH₄HCO₃)-acetonitrile]; B%: 40%-70%, 11 min) (B was acetonitrile) to give the target compound **WX014-11.**

### Step 9: synthesis of compounds WX014 and WX015

The compound **WX014-11** was separated by supercritical fluid chromatography (column: DAICEL CHIRALPAK AD-H (250 mm × 30 mm, 5 µm); mobile phase: [0.1% ammonia in isopropanol]; B%: 40%-40%, 10 min) (B was 0.1% ammonia in isopropanol) to give **WX014** (retention time: 1.61 min) and **WX015** (retention time: 2.33 min).WX014: ¹H NMR (400MHz,CDCl₃) δ 8.94 - 8.83 (m, 1H), 8.21 (s, 1H), 7.82 (dd, *J*=2.2, 6.6 Hz, 1H), 7.40 (br d, *J*=11.4 Hz, 1H), 7.34 (br d, *J*=8.3 Hz, 1H), 7.26 - 7.24 (m, 1H), 7.10 (t, *J*=8.6 Hz, 1H), 7.04 (d, *J=*7.9 Hz, 1H), 6.95 - 6.83 (m, 2H), 6.27 (q, *J*=6.9 Hz, 1H), 5.39 (br s, 2H), 4.62 (td, *J*=6.2, 12.1 Hz, 1H), 1.97 (d, *J*=7.0 Hz, 3H), 1.40 (d, *J*=6.1 Hz, 6H); LCMS: m/z= 606.0[M+1].**WX015**: ¹H NMR (400MHz, CDCl₃) δ 8.90 - 8.82 (m, 1H), 8.21 (s, 1H), 7.82 (dd, *J*=2.6, 7.0 Hz, 1H), 7.40 (d, *J*=11.4 Hz, 1H), 7.34 (br d, *J*=9.2 Hz, 1H), 7.23 (br s, 1H), 7.10 (t, *J*=8.3 Hz, 1H), 7.04 (d, *J*=7.5 Hz, 1H), 6.94 - 6.82 (m, 2H), 6.27 (q, *J*=6.7 Hz, 1H), 5.41 (br s, 2H), 4.62 (quin, *J*=6.0 Hz, 1H), 1.97 (d, *J*=7.0 Hz, 3H), 1.40 (d, *J*=6.1 Hz, 6H); LCMS: m/z= 606.0[M+1].

### Example 9: WX016 and WX017

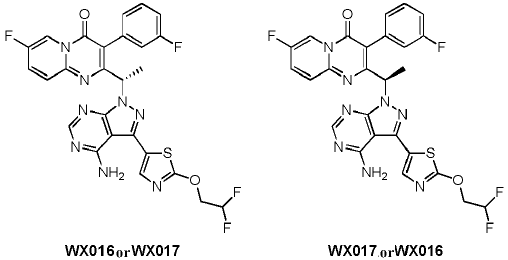

### Synthetic route:

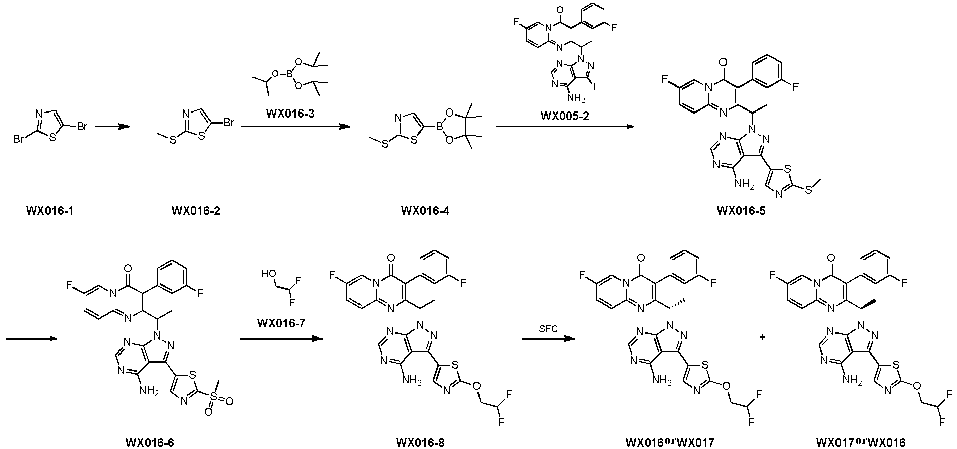

### Step 1: synthesis of compound WX016-2

**WX016-1** (5 g, 20.58 mmol, 1 *eq*.) was dissolved in ethanol (50 mL), then sodium thiomethoxide (40.68 g, 116.09 mmol, 36.98 mL, 5.64 *eq*.) was slowly added dropwise under nitrogen atmosphere at 0 °C, and the reaction system was stirred at 20 °C for 2 h. After the reaction was completed, the reaction system was added with water (150 mL), and extracted with ethyl acetate (100 mL × 3). The organic phases were collected and combined, washed with saturated brine (200 mL × 2), dried over anhydrous sodium sulfate, and filtered. The filtrate was concentrated by rotary evaporation to give crude **WX016-2.**

### Step 2: synthesis of compound WX016-4

**WX016-2** (3.26 g, 13.91 mmol, 1 *eq.;* 89.646% purity) and **WX016-3** (10.35 g, 55.64 mmol, 11.35 mL, 4 *eq.)* were dissolved in tetrahydrofuran (120 mL), then *n*-butyllithium (2.5 M, 16.69 mL, 3 *eq.)* was added slowly under nitrogen atmosphere at-78 °C, and the reaction system was stirred at-78 °C for 2 h. After the reaction was completed, methanol (10 mL) was slowly added to quench at -78 °C to quench the reaction and the liquid was removed by rotary evaporation to give crude **WX016-4.**

### Step 3: synthesis of compound WX016-5

**WX005-2** (1.5 g, 2.75 mmol, 1 *eq.*) and **WX016-4** (17 g, 66.10 mmol, 24.03 *eq.;* crude product) were dissolved in dioxane (60 mL) and water (30 mL), then potassium acetate (1.08 g, 11.00 mmol, 4 *eq*.*)* was added, followed by [1,1'-bis(diphenylphosphino)ferrocene]dichloropalladium(II) (450.00 mg, 615.00 µmol, 2.24e-1 *eq.*) under nitrogen atmosphere. The mixture was heated to 70 °C and stirred for 2 h. After the reaction was completed, the solvent was removed under reduced pressure by rotary evaporation (water pump), and water (200 mL) and dichloromethane (100 mL × 3) were added for extraction. The organic phase was collected, combined, washed with saturated brine (200 mL), and removed by rotary evaporation. The crude product was purified by silica gel column chromatography (ethyl acetate/petroleum ether = 0%-80%-90%) to give the target compound **WX016-5.**

### Step 4: synthesis of compound WX016-6

**WX016-5** (600 mg, 1.03 mmol, 1 *eq.*; 89.675% purity) was dissolved in tetrahydrofuran (15 mL) and methanol (15 mL), then potassium monopersulfate (2.7 M, 3.07 mL, 8.03 *eq.)* was added, and the reaction system was stirred at 20 °C for 16 h. After the reaction was completed, the solvent was removed under reduced pressure by rotary evaporation (water pump), and the reaction system was added with water (100 mL), and extracted with dichloromethane (50 mL × 3). The organic phases were collected and combined, washed with saturated brine (100 mL), dried over anhydrous sodium sulfate, and filtered. The filtrate was concentrated by rotary evaporation to give crude **WX016-6.**

### Step 5: synthesis of compound WX016-8

**WX016-6** (200 mg, 344.48 µmol, 1 *eq*.; crude) and **WX016-7** (140 mg, 1.71 mmol, 66.59 µL, 4.95 *eq.)* were dissolved in tetrahydrofuran (2 mL) and water (1 mL), then potassium hydroxide (100.00 mg, 1.78 mmol, 5.17 *eq.)* was added, and the reaction system was stirred at 20 °C for 16 h. After the reaction was completed, the reaction system was added with water (20 mL), and extracted with dichloromethane (10 mL × 3). The organic phases were collected and combined, washed with saturated brine (30 mL), dried over anhydrous sodium sulfate and filtered. The filtrate was concentrated by rotary evaporation to give a crude product which was purified by preparative high performance liquid chromatography (column: Phenomenex Gemini-NX 150 × 30 mm × 5µm; mobile phase: [water (0.04% aqueous ammonia + 10mM NH₄HCO₃)-acetonitrile]; B%: 38%-68%, 8 min) (B was acetonitrile) to give **WX016-8.**

### Step 6: synthesis of compounds WX016 and WX017

**WX016-8** was separated by supercritical fluid chromatography (column: DAICEL CHIRALPAK AD (250 mm × 50 mm, 10 µm); mobile phase: [0.1% ammonia in isopropanol]; B%: 45%-45% (B was 0.1% ammonia in isopropanol)) to give **WX016** (retention time: 1.895 min) and **WX017** (retention time: 2.277 min).**WX016**:¹H NMR (400 MHz, DMSO-*d*₆) δ 1.78 (3 H, d, *J*=7.03 Hz) 4.77 (2 H, td, *J*=15.06, 3.01 Hz) 6.06 (1 H, q, *J*=6.94 Hz) 6.33 - 6.60 (1 H, t, *J*=25.4 Hz) 6.99 (3 H, br t, *J*=7.53 Hz) 7.09 (2 H, br s) 7.28 (1 H, q, *J*=7.11 Hz) 7.47 (1 H, s) 7.77 - 7.84 (1 H, m) 8.07 (1 H, s) 8.08 - 8.15 (1 H, m) 8.92 (1 H, dd, *J*=4.64, 2.89 Hz),MS, m/z= 583.0[M+1].**WX017**:¹H NMR (400 MHz, DMSO-*d*₆) δ 1.84 (3 H, d, *J*=7.03 Hz) 4.82 (2 H, td, *J*=15.00, 3.14 Hz) 6.11 -6.52 (1 H, t, *J*=25.4 Hz) 6.65 (1 H, t, *J*=3.01 Hz) 7.04 (3 H, br t, *J*=7.53 Hz) 7.14 (2 H, br s) 7.28 - 7.39 (1 H, m) 7.52 (1 H, s) 7.86 (1 H, dd, *J*=9.79, 5.27 Hz) 8.12 (1 H, s) 8.17 (1 H, ddd, *J*=9.79, 7.03, 2.76 Hz) 8.97 (1 H, dd, *J*=4.52, 2.76 Hz) ,MS, m/z= 583.0[M+1].

### Example 10: WX018 and WX019

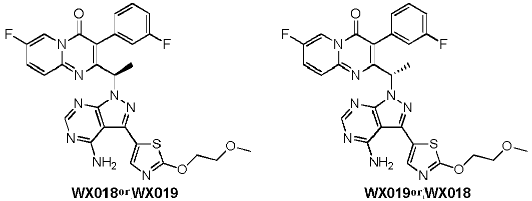

### Synthetic route:

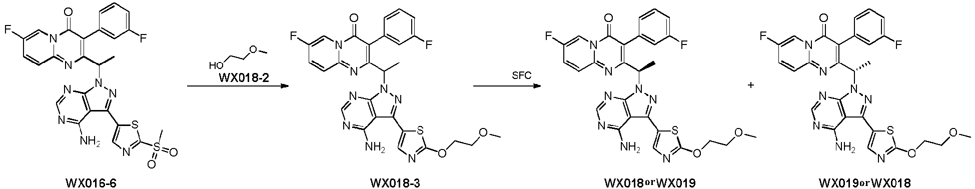

### Step 1: synthesis of compound WX018-3

Compound **WX016-6** (100 mg, 168.90 µmol, 1 *eq.;* 98.059% purity) and **WX018-2** (64.26 mg, 844.48 µmol, 66.59 µL, 5 *eq.*) were dissolved in tetrahydrofuran (2 mL) and water (1 mL), then potassium hydroxide (50 mg, 891.18 µmol, 5.28 *eq*.) was added, and the mixture was stirred at 20 °C for 16 h. The reaction system was added with water (20 mL), and extracted with dichloromethane (10 mL × 3). The organic phases were collected and combined, washed with saturated brine (20 mL), dried over anhydrous sodium sulfate, and filtered. The filtrate was concentrated by rotary evaporation to give the target compound **WX018-3.**

### Step 2: synthesis of compounds WX018 and WX019

Compound **WX018-3** (100 mg, 160.12 µmol, 1 *eq.*; 92.32% purity) was separated by supercritical fluid chromatography (column: DAICEL CHIRALPAK AD (250 mm × 50 mm, 10 µm); mobile phase: [0.1% ammonia in isopropanol]; B%: 45%-45% (B was 0.1% ammonia in isopropanol), min) to give **WX018** (retention time: 2.136 min) and **WX019** (retention time: 2.479 min).**WX018:** ¹H NMR (400 MHz, DMSO-*d*₆) δ 1.78 (3 H, d, *J*=7.03 Hz) 3.31 (3 H, s) 3.67 - 3.73 (2 H, m) 4.50 - 4.58 (2 H, m) 6.05 (1 H, q, *J*=6.94 Hz) 6.99 (3 H, br t, *J*=7.53 Hz) 7.02 - 7.20 (2 H, m) 7.28 (1 H, br d, *J*=7.03 Hz) 7.43 (1 H, s) 7.81 (1 H, dd, *J*=9.79, 5.52 Hz) 8.06 (1 H, s) 8.12 (1 H, ddd, *J*=9.85, 7.09, 2.89 Hz) 8.93 (1 H, dd, *J*=4.52, 2.76 Hz); LCMS: m/z= 577.0 [M+1].**WX019**: ¹H NMR (400 MHz, DMSO-*d*₆) δ 1.78 (3 H, d, *J*=7.03 Hz) 3.31 (3 H, s) 3.67 - 3.74 (2 H, m) 4.52 - 4.59 (2 H, m) 6.05 (1 H, q, *J*=7.03 Hz) 6.92 - 7.02 (3 H, m) 7.03 - 7.19 (2 H, m) 7.24 - 7.33 (1 H, m) 7.43 (1 H, s) 7.81 (1 H, dd, *J*=9.91, 5.40 Hz) 8.06 (1 H, s) 8.12 (1 H, ddd, *J*=9.85, 7.09, 2.89 Hz) 8.93 (1 H, dd, *J*=4.64, 2.89 Hz); LCMS: m/z= 577.0 [M+1].

### Example 11: WX020 and WX021

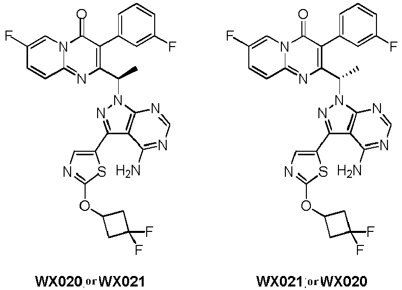

### Synthetic route:

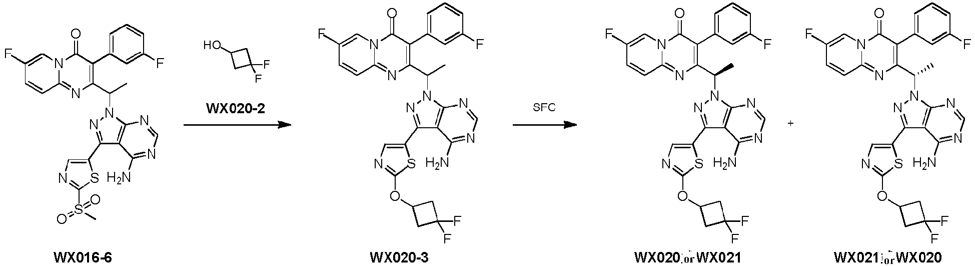

### Step 1: synthesis of compound WX020-3

**WX016-6** (200 mg, 344.48 µmol, 1 *eq.;* crude) and **WX020-2** (186.17 mg, 1.72 mmol, 5 *eq.)* were dissolved in tetrahydrofuran (4 mL) and water (2 mL), then potassium hydroxide (100.69 mg, 1.79 mmol, 5.21 *eq.)* was added, and the reaction system was stirred at 20 °C for 16 h. The reaction system was added with water (20 mL), and extracted with dichloromethane (20 mL × 3). The organic phases were collected and combined, washed with saturated brine (50 mL), dried over anhydrous sodium sulfate, and filtered. The filtrate was concentrated by rotary evaporation to give a crude product. The crude product was separated by preparative high performance liquid chromatography (column: Welch Xtimate C18 150 mm × 25 mm × 5 µm; mobile phase: [water (0.04% ammonia + 10 mM NH₄HCO₃)-acetonitrile]; B%: 28%-58%, 7.5 min (B was acetonitrile) to give the target compound **WX020-3.**1H NMR (400 MHz, DMSO-*d*₆) δ 1.78 (3 H, d, *J*=7.03 Hz) 2.84 - 2.97 (2 H, m) 3.16 - 3.28 (2 H, m) 5.22 (1 H, br dd, *J*=8.28, 4.52 Hz) 6.05 (1 H, q, *J*=6.69 Hz) 6.91 - 7.02 (3 H, m) 7.02 - 7.19 (2 H, m) 7.28 (1 H, br d, *J*=6.53 Hz) 7.45 (1 H, s) 7.81 (1 H, dd, *J*=9.79, 5.27 Hz) 8.06 (1 H, s) 8.12 (1 H, ddd, *J*=9.85, 7.09, 2.89 Hz) 8.93 (1 H, dd, *J*=4.52, 3.01 Hz).

### Step 2: synthesis of compounds WX020 and WX021

The compound **WX020-3** was separated by supercritical fluid chromatography (column: DAICEL CHIRALCEL OD-H (250 mm × 30 mm, 5 µm); mobile phase: [0.1% ammonia in ethanol]; B%: 45%-45% (B was 0.1% ammonia in ethanol), min) to give **WX020** (retention time: 1.882 min) and **WX021** (retention time: 2.481 min).**WX020:** 1H NMR (400 MHz, DMSO-*d*₆) δ 1.78 (3 H, d, *J*=7.03 Hz) 2.84 - 2.97 (2 H, m) 3.16 - 3.28 (2 H, m) 5.22 (1 H, br dd, *J*=8.28, 4.52 Hz) 6.05 (1 H, q, *J*=6.69 Hz) 6.91 - 7.02 (3 H, m) 7.02 - 7.19 (2 H, m) 7.28 (1 H, br d, *J*=6.53 Hz) 7.45 (1 H, s) 7.81 (1 H, dd, *J*=9.79, 5.27 Hz) 8.06 (1 H, s) 8.12 (1 H, ddd, *J*=9.85, 7.09, 2.89 Hz) 8.93 (1 H, dd, *J*=4.52, 3.01 Hz); LCMS: m/z= 609.0[M+1].**WX021**:1H NMR (400 MHz, DMSO-*d*₆) δ 1.78 (3 H, d, *J*=7.03 Hz) 2.84 - 2.97 (2 H, m) 3.19 - 3.27 (2 H, m) 5.17 - 5.27 (1 H, m) 6.05 (1 H, q, *J*=7.03 Hz) 6.93 - 7.02 (3 H, m) 7.08 (2 H, br s) 7.28 (1 H, br d, *J*=5.77 Hz) 7.45 (1 H, s) 7.81 (1 H, dd, *J*=9.79, 5.27 Hz) 8.06 (1 H, s) 8.12 (1 H, ddd, *J*=9.85, 7.09, 2.89 Hz) 8.93 (1 H, dd, *J*=4.64, 2.89 Hz); LCMS: m/z= 609.0[M+1].

### Example 12: WX022 and WX023

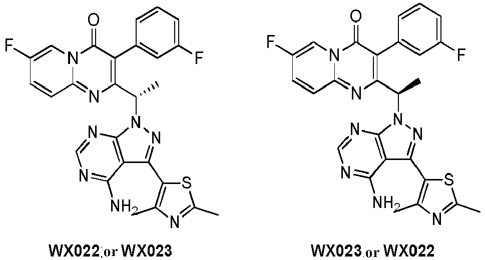

### Synthetic route:

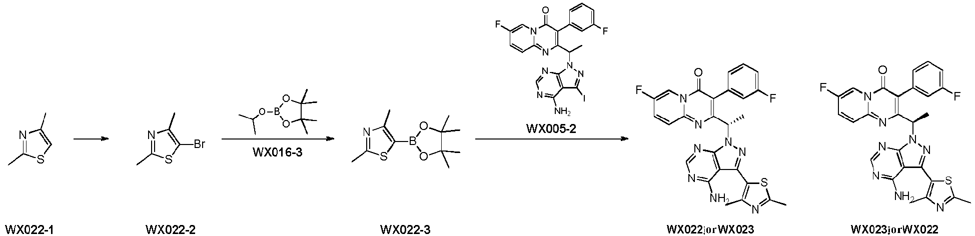

### Step 1: synthesis of compound WX022-2

**WX022-1** (3 g, 26.51 mmol, 1 *eq.)* was dissolved in dichloromethane (30 mL), then a solution of potassium carbonate (2.65 g, 19.20 mmol, 7.24e-1 *eq.)* and bromine (4.24 g, 26.51 mmol, 1.37 mL, 1 *eq.)* in dichloromethane (10 mL) was added, and the mixture was stirred at room temperature (about 28 °C) for 3 h. After the reaction was completed, the reaction system was added with water (about 200 mL) to quench the reaction, and extracted with dichloromethane (100 mL × 3). The organic phases were combined, dried over anhydrous sodium sulfate, filtered, and concentrated to give **WX022-2.**¹H NMR (400MHz, CDCl₃) δ 2.55 (s, 3H), 2.28 (s, 3H).

### Step 2: synthesis of compound WX022-3

**WX022-2** (5 g, 26.03 mmol, 1 *eq.;* crude) and **WX016-3** (14.53 g, 78.09 mmol, 15.93 mL, 3 *eq.)* were dissolved in tetrahydrofuran (150 mL), and *n*-butyllithium (2.5 M, 31.24 mL, 3 *eq.)* was added at -78 °C. The mixture was stirred at -78 °C for 1 h and then at 30 °C for 1.5 h. The reaction system was added with methanol (about 100 mL) to quench the reaction, and concentrated to give crude **WX022-3,** which was used directly in the next step.

### Step 3: synthesis of compounds WX022 and WX023

**WX022-3** (0.4 g, 733.56 µmol, 1 *eq.;* crude) and **WX005-2** (175.43 mg, 733.56 µmol, 1 *eq.)* was dissolved in a mixed solvent of *N*,*N-*dimethylformamide (15 mL), ethanol (8 mL) and water (8 mL) before potassium carbonate (304.16 mg, 2.20 mmol, 3.0 *eq.)* was added. Under nitrogen atmosphere, tetrakis(triphenylphosphine)palladium(0) (169.54 mg, 146.71 µmol, 0.2 *eq.)* was added, and the reaction system was stirred at 100 °C for 2 h. After the reaction was completed, the reaction system was concentrated by rotary evaporation, added with water (about 20 mL) to quench the reaction, and extracted with dichloromethane (20 mL × 3). The organic phases were combined, dried over anhydrous sodium sulfate, filtered, concentrated, separated by a silica gel column, and further purified by preparative high performance liquid chromatography (column: Xtimate C18 150 × 25 mm × 5 µm; mobile phase: [water (0.225% FA)-acetonitrile]; B%: 32%-52% (B was acetonitrile), 7 min). The intermediate product was separated by supercritical fluid chromatography (column: DAICEL CHIRALPAK AS (250 mm × 50 mm, 10 µm); mobile phase: [0.1% ammonia in ethanol]; B%: 30%-30% (B was 0.1% ammonia in ethanol), min) to give **WX022** (retention time: 3.411 min) and **WX023** (retention time: 3.978 min).**WX022:** ¹H NMR (400MHz, CDCl₃) δ 8.97 - 8.69 (m, 1H), 8.19 - 8.04 (m, 1H), 7.57 (dd, *J*=1.5, 5.8 Hz, 2H), 7.23 (m, 1H), 7.06 - 6.81 (m, 3H), 6.13 (q, *J*=6.9 Hz, 1H), 5.37 (br s, 2H), 2.66 (s, 3H), 2.40 - 2.27 (m, 3H), 1.84 (d, *J*=7.0 Hz, 3H); LCMS: m/z= 531.3[M+1].**WX023**:¹H NMR (400MHz, CDCl₃) δ 9.00 - 8.67 (m, 1H), 8.13 (s, 1H), 7.57 (dd, *J*=1.6, 5.9 Hz, 2H), 7.23 (m, 1H), 7.14 - 6.76 (m, 3H), 6.13 (q, *J*=7.0 Hz, 1H), 5.40 (br s, 2H), 2.66 (s, 3H), 2.34 (s, 3H), 1.84 (d, *J*=7.0 Hz, 3H); LCMS: m/z= 531.3[M+1].

### Example 13: WX024 and WX025

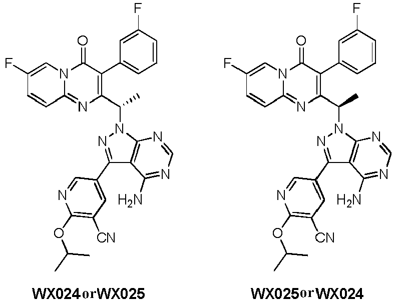

### Synthetic route:

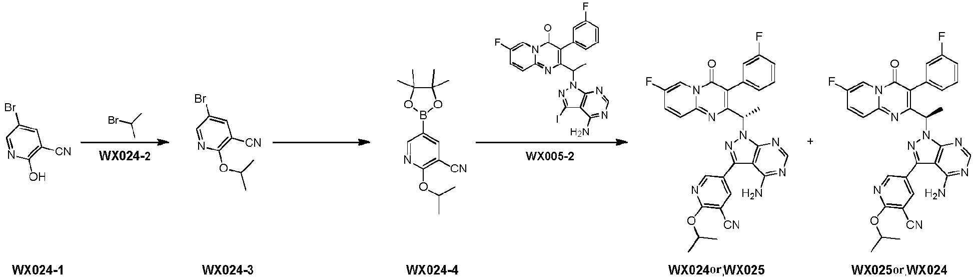

### Step 1: synthesis of compound WX024-3

**WX024-1** (1.64 g, 8.24 mmol, 1 *eq.)* and **WX024-2** (1.03 g, 8.38 mmol, 787 µL, 1.02 *eq.)* were dissolved in *N*,*N-*dimethylformamide (30 mL), then cesium carbonate (5.38 g, 16.51 mmol, 2 *eq.)* was added, and the reaction system was stirred at 80 °C for 1 h. After the reaction system was completed, the reaction system was filtered. The filtrate was concentrated by rotary evaporation, added with water (20 mL), and extracted with dichloromethane (20 mL × 3). The organic phases were collected and combined, washed with saturated brine (50 mL), dried over anhydrous sodium sulfate, and filtered. The filtrate was concentrated by rotary evaporation to give a crude product. The crude product was purified by silica gel chromatographic column chromatography (dichloromethane) to give **WX024-3.**

### Step 2: synthesis of compound WX024-4

**WX024-3** (950 mg, 3.94 mmol, 1 *eq.)* and bis(pinacolato)diboron (1.10 g, 4.34 mmol, 1.1 *eq.)* were dissolved in dioxane (5 mL), and potassium acetate (1.16 g, 11.81 mmol, 3 *eq.)* was added. Under nitrogen atmosphere, [1,1'-bis(diphenylphosphino)ferrocene]dichloropalladium(II) dichloromethane (323.00 mg, 395.53 µmol, 0.1 *eq.)* was added and the reaction system was stirred at 80 °C for 16 h. After the reaction was completed, the solvent was removed under reduced pressure by rotary evaporation, and the reaction system was added with dichloromethane (20 mL), and filtered. The filtrate was extracted with water (20 mL), and the aqueous phase was extracted with dichloromethane (20 mL × 2). The organic phases were collected and combined, washed with saturated brine (60 mL), dried over anhydrous sodium sulfate and filtered. The filtrate was concentrated by rotary evaporation to give a crude product. The crude product was purified by silica gel column chromatography (petroleum ether:ethyl acetate = 1:0-20:1) to give **WX024-4.**

### Step 3: synthesis of compounds WX024 and WX025

**WX005-2** (200 mg, 366.78. µmol, 1 *eq.;* crude), **WX024-4** (310 mg, 1.08 mmol, 2.93 *eq.)* were dissolved in dioxane (20 mL) and water (2.5 mL), and potassium acetate (110.00 mg, 1.12 mmol, 3.06 *eq.)* was added. The system was purged with nitrogen, added with [1,1'-bis(diphenylphosphino)ferrocene]dichloropalladium(II) (60.00 mg, 82.00 µmol, 2.24e-1 *eq.)* at 70 °C, followed by another nitrogen purging. The reaction system was incubated at 70 °C for 16 h. After the reaction was completed, the solvent was removed under reduced pressure by rotary evaporation.The crude product was purified by silica gel column chromatography (petroleum ether: ethyl acetate = 1:0-1:4), and separated by supercritical fluid chromatography (column: DAICEL CHIRALCEL OD (250 mm × 30 mm, 10 µm); mobile phase: [0.1% ammonia in ethanol]; B%: 55%-55% (B was 0.1% ammonia in ethanol), min) to give **WX024** (retention time: 1.978 min) and **WX025** (retention time: 1.585 min).**WX024:** ¹H NMR (400 MHz, DMSO-*d*₆) δ 1.39 (6 H, d, *J*=6.27 Hz) 1.81 (3 H, d, *J*=6.78 Hz) 5.44 (1 H, dt, *J*=12.36, 6.24 Hz) 6.11 (1 H, q, *J*=6.86 Hz) 6.91 - 7.07 (4 H, m) 7.26 (1 H, br d, *J*=7.28 Hz) 7.85 (1 H, dd, *J*=9.66, 5.40 Hz) 8.05 (1 H, s) 8.12 (1 H, ddd, *J*=9.91, 7.28, 2.89 Hz) 8.31 (1 H, d, *J*=2.26 Hz) 8.54 (1 H, d, *J*=2.26 Hz) 8.92 (1 H, dd, *J*=4.52, 3.01 Hz); LCMS: m/z= 580.4[M+1].**WX025**: ¹H NMR (400 MHz, DMSO-*d*₆) δ ppm 1.39 (6 H, d, *J*=6.27 Hz) 1.81 (3 H, d, *J*=6.78 Hz) 5.44 (1 H, quin, *J*=6.21 Hz) 6.11 (1 H, q, *J*=6.78 Hz) 6.92 - 7.07 (4 H, m) 7.21 - 7.31 (1 H, m) 7.84 (1 H, dd, *J*=9.91, 5.40 Hz) 8.05 (1 H, s) 8.12 (1 H, ddd, *J*=9.79, 7.03, 2.76 Hz) 8.31 (1 H, d, *J*=2.51 Hz) 8.53 (1 H, d, *J*=2.26 Hz) 8.92 (1 H, dd, *J*=4.64, 2.89 Hz); LCMS: m/z= 580.4[M+1].

### Example 14: WX026 and WX027

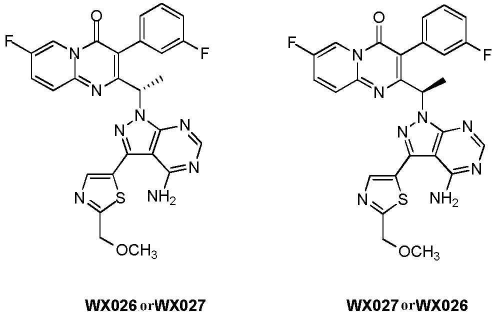

### Synthetic route:

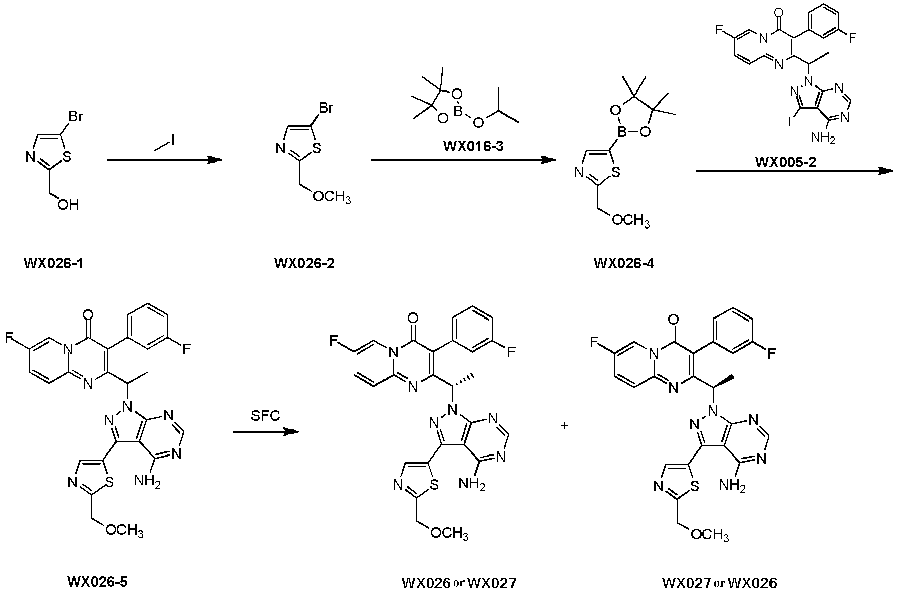

### Step 1: synthesis of compound WX026-2

Compound **WX026-1** (0.3 g, 1.55 mmol, 1 *eq.)* was dissolved in *N,N*-dimethylformamide (3 mL) and purged with nitrogen. At -25 °C, the reaction system was added with sodium hydride (534.16 mg, 13.36 mmol, 60% purity, 8.64 *eq.)* and stirred at -25 °C for 20 min. Methyl iodide (1.32 g, 9.28 mmol, 577.46 µL, 6 *eq.)* was then added dropwise to a flask, and the mixture was stirred at -25 °C for 15 min. The reaction system was added with water (15 mL) to quench the reaction, and extracted with ethyl acetate (15 mL × 3). The organic phase was collected, washed with water (15 mL) and saturated brine (15 mL), dried over anhydrous sodium sulfate (20 g), filtered, and concentrated under reduced pressure by rotary evaporation (45 °C, water pump) to give the target compound **WX026-2.** 1H NMR (400 MHz, DMSO-*d*₆) δ ppm 7.85 (s, 1 H), 4.68 (s, 2 H), 3.39 (s, 3 H).

### Step 2: synthesis of compound WX026-4

The compound **WX026-2** was dissolved in anhydrous tetrahydrofuran (7 mL), and **WX016-3** (1.32 g, 7.11 mmol, 1.45 mL, 4 *eq.)* and TMEDA (268.64 mg, 2.31 mmol, 348.88 µ L, 1.3 *eq.)* were added. After purging with nitrogen three times, the reaction system was cooled to -65 °C, and slowly added dropwise with *n*-BuLi (2.5 M, 2.13 mL, 3 *eq.).* The system was stirred at -65 °C for 2 h, and then at 15 °C for 0.5 h. The reaction system was added with methanol (10 mL) to quench the reaction, and concentrated under reduced pressure by rotary evaporator (water pump, 45 °C) to give the target compound **WX026-4.**

### Step 3: synthesis of compound WX026-5

Compound **WX005-2** (0.42 g, 770.24 µmol, 1 *eq.)* and **WX026-4** (133.25 mg, 770.24 µmol, 1 *eq.)* were dissolved in 1,4-dioxane (28 mL) and water (14 mL), and potassium carbonate (226.78 mg, 2.31 mmol, 3 *eq.)* was added. The system was purged with nitrogen, added with Pd(dppf)Cl₂ (169.08 mg, 231.07 µmol, 0.3 *eq.),* followed by another nitrogen purging. The reaction system was stirred for 5 h in a 70 °C oil bath. The reaction system was diluted with water (42 mL), and extracted with dichloromethane (50 mL × 3). The organic phase was collected, washed with saturated brine (100 mL), and concentrated under reduced pressure by rotary evaporator (water pump, 55 °C). The crude product was separated by flash column chromatography (silica gel: 100-200 mesh; dichloromethane:methanol = 100:1-10:1). TLC separation: the crude product was purified by thin layer chromatography (dichloromethane:methanol = 20:1). The purified crude product was separated by high performance liquid chromatography (column: Waters Xbridge 150 mm × 25 mm 5 µm; mobile phase: [water (10 mM NH₄HCO₃)-acetonitrile]; B%: 20%-50% (B was acetonitrile),10 min) to give the target compound WX026-5.

### Step 4: synthesis of compounds WX026 and WX027

Compound **WX026-5** (0.11 g, 201.26 µmol, 1 *eq.)* was separated by supercritical fluid chromatography (column: DAICEL CHIRALPAK AD-H (250 mm × 30 mm, 5 µm); mobile phase: [0.1% ammonia in isopropanol]; B%: 50%-50% (B was 0.1% ammonia in isopropanol), 10 min) to give **WX026** (retention time: 2.04 min) and **WX027** (retention time: 2.45 min).**WX026:** 1H NMR (400 MHz, DMSO-*d*₆) δ 8.92 (dd, *J*=4.82, 2.63 Hz, 1 H), 8.12 (ddd, *J*=9.98, 7.13, 2.63 Hz, 1 H), 8.07 (s, 1 H), 7.96 (s, 1 H), 7.81 (dd, *J*=9.87, 5.48 Hz, 1 H), 7.27 (br d, *J*=6.58 Hz, 1 H), 6.98 (br d, *J*=8.77 Hz, 2 H), 6.08 (q, *J*=6.87 Hz, 1 H), 4.74 (s, 2 H), 3.44 (s, 3 H), 1.79 (d, *J*=7.02 Hz, 3 H); LCMS: m/z= 547.1 [M+1].**WX027:** 1H NMR (400 MHz, DMSO-*d*₆) δ8.92 (dd, *J*=4.60, 2.85 Hz, 1 H), 8.11 (ddd, *J*=9.76, 7.13, 2.85 Hz, 1 H), 8.06 (s, 1 H), 7.96 (s, 1 H), 7.81 (dd, *J*=10.09, 5.26 Hz, 1 H), 7.26 (br d, *J*=7.89 Hz, 1 H), 6.98 (br d, *J*=8.33 Hz, 2 H), 6.07 (q, *J*=6.87 Hz, 1 H), 4.74 (s, 2 H), 3.43 (s, 3 H), 1.79 (d, *J*=7.02 Hz, 3 H); LCMS: m/z= 547.1 [M+1].

### Example 15: WX028 and WX029

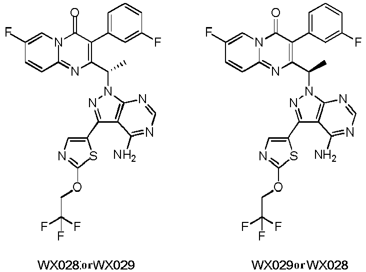

Synthetic route:

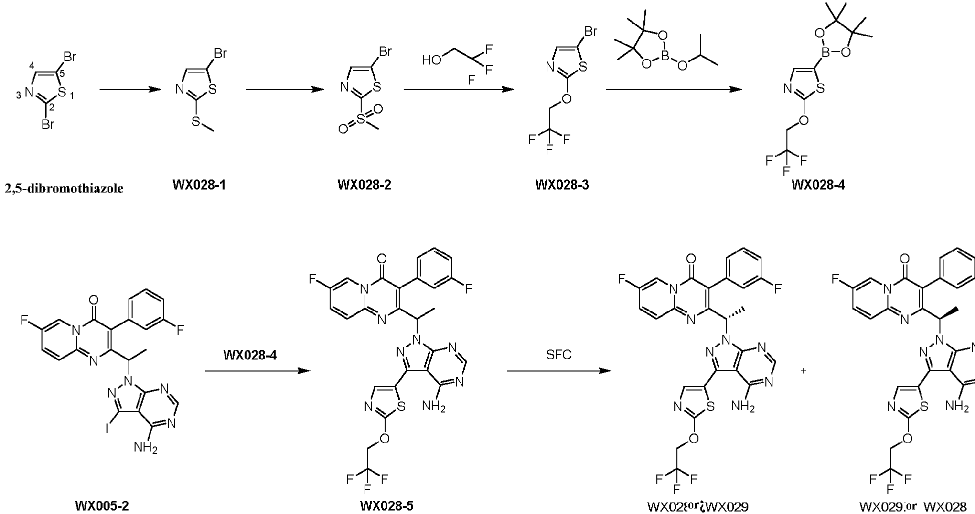

### Step 1: synthesis of compound WX028-1

2,5-dibromothiazole (10 g, 41.17 mmol, 1 *eq.)* and ethanol (100 mL) were added to a pre-dried flask. The system was purged with nitrogen three times, slowly added with sodium thiomethoxide (43.28 g, 123.50 mmol, 39.34 mL, 20% purity, 3 *eq.)* at 0 °C, and stirred for 2 h at 25 °C. The reaction system was poured into water (100 mL), which was then diluted with ethyl acetate (100 mL). The aqueous phase was extracted with ethyl acetate (100 mL × 3). The organic phases were combined, washed successively with saturated brine (100 mL), dried over anhydrous sodium sulfate, and concentrated under reduced pressure to give **WX028-1.**¹H NMR (400 MHz, CDCl₃) δ 2.68 (s, 3 H) 7.52 (s, 1 H); LCMS: m/z= 209.9 [M+1].

### Step 2: synthesis of compound WX028-2

To a pre-dried reaction flask were added WX028-1 (8.3 g, 39.50 mmol, 1 *eq.),* tetrahydrofuran (84 mL) and methanol (84 mL). At 0 °C, potassium bisulfate (48.57 g, 79.00 mmol, 2 *eq.),* sodium acetate (16.20 g, 197.51 mmol, 5 *eq.)* and water (84 mL) were added, and the reaction system was stirred at 0 °C for 1 h and then at 29 °C for 15 h. The reaction system was added with water (100 mL) to quench the reaction, and the aqueous phase was extracted with ethyl acetate (150 mL × 3). The organic phases were combined, washed with saturated brine (150 mL × 2), dried over anhydrous sodium sulfate, filtered, and concentrated under reduced pressure to give a crude product. The crude product was purified by silica gel column chromatography (petroleum ether: ethyl acetate =1:0 to 20:1 to 10:1 to 5:1) to give **WX028-2**.¹H NMR (400 MHz, CDCl₃) δ ppm 3.31 (s, 3 H) 7.94 (s, 1 H); LCMS: m/z= 241.9 [M+1].

### Step 3: synthesis of compound WX028-3

2,2,2-trifluoroethanol (619.79 mg, 6.20 mmol, 445.89 µL, 1.5 *eq.)* and tetrahydrofuran (10 mL) were added to a pre-dried reaction flask. At 0 °C, sodium hydrogen (330.39 mg, 8.26 mmol, 60% purity, 2 *eq.)* was added slowly. The system was stirred at 25 °C for 1 h, then added with **WX028-2** (1 g, 4.13 mmol, 1 *eq.)* and stirred for 3 h at 25 °C. The reaction was quenched with water (50 mL), and the aqueous phase was extracted with dichloromethane (50 mL × 3). The organic phases were combined, washed with saturated brine (50 mL × 2), dried over anhydrous sodium sulfate, filtered, and concentrated under reduced pressure to give a crude product. The crude product was purified by silica gel thin layer chromatography (petroleum ether:ethyl acetate = 10:1) to give the target compound **WX028-3**.¹H NMR (400 MHz, CDCl₃) δ 4.79 (q, *J*=8.19 Hz, 2 H) 7.08 (s, 1 H); LCMS: m/z= 261.9 [M+1].

### Step 4: synthesis of compound WX028-4

**WX028-3** (610 mg, 2.33 mmol, 1 *eq.),* **WX016-3** (1.73 g, 9.31 mmol, 1.90 mL, 4 *eq.)* and tetrahydrofuran (15 mL), *N*,*N*,*N*',*N*'-tetramethylethylenediamine (351.66 mg, 3.03 mmol, 456.70 µL, 1.3 *eq.)* and tetrahydrofuran (15 mL) were added to a pre-dried flask. The system was purged with nitrogen three times, and added dropwise with *n*-BuLi (2.5 M, 2.79 mL, 3 *eq.)* at -78 °C. The system was stirred at -78 °C for 2 h and at 25 °C for 1 h. Methanol (5 mL) was added directly to the reaction system to quench the reaction. The reaction system was concentrated by rotary evaporation to give **WX028-4.** LCMS: m/z= 310.1[M+1].

### Step 5: synthesis of compound WX028-5

To a pre-dried reaction flask were added **WX028-4** (308.39 mg, 679.36 µmol, 1.5 *eq.),* **WX005-2** (246.96 mg, 452.91 µmol, 1 *eq.),* dioxane (10 mL), water (5 mL), and potassium carbonate (187.79 mg, 1.36 mmol, 3 *eq.).* The system was purged with nitrogen three times, and added with Pd(dppf)Cl₂ (66.28 mg, 90.58 µmol, 0.2 *eq.),* followed by another three nitrogen purges. The reaction system was stirred at 70 °C for 16 h. The reaction system was filtered through celite, and the filtrate was added with water (10 mL). The aqueous phase was extracted with dichloromethane (10 mL × 3). The organic phases were combined, washed with saturated brine (10 mL × 2), dried over anhydrous sodium sulfate, filtered, and concentrated under reduced pressure to give a crude product. The crude product was purified by silica gel thin layer chromatography (petroleum ether:ethyl acetate = 0:1) to give a pure product. The pure product was purified by preparative HPLC (column: Waters Xbridge Prep OBD C18 150 × 40 mm, 10 µm; mobile phase: [water (10 mM NH₄HCO₃)-acetonitrile]; B%: 35%-55% (B was acetonitrile), 11 min) to give **WX028-5**.LCMS: m/z= 601.0 [M+1].

### Step 6: synthesis of compounds WX028 and WX029

**WX028-5** (100 mg, 166.52 µmol, 1 *eq.)* was separated by supercritical chromatography (column: DAICEL CHIRALPAK AS (250 mm × 30 mm, 10 µm); mobile phase: 0.1% ammonia in isopropanol; B%: 40%-40%), and (B: 0.1% ammoniain isopropanol) to give the target compounds **WX028** and **WX029.** The retention time of **WX028** in SFC was 1.59 min. The retention time of **WX029** in SFC was 1.74 min.**WX028:** ¹H NMR (400 MHz, DMSO-*d*₆) δ 1.78 (d, *J*=6.97 Hz, 3 H) 5.20 (q, *J*=8.80 Hz, 2 H) 6.05 (q, *J*=6.81 Hz, 1 H) 6.95 - 7.17 (m, 5 H) 7.28 (br d, *J*=7.46 Hz, 1 H) 7.49 (s, 1 H) 7.80 (dd, *J*=9.84, 5.32 Hz, 1 H) 8.05 - 8.15 (m, 2 H) 8.92 (dd, *J*=4.65, 2.69 Hz, 1 H), LCMS: m/z= 601.1 [M+1]; **WX029:** ¹H NMR (400 MHz, DMSO-*d*₆) δ 1.78 (d, *J*=6.97 Hz, 3 H) 5.20 (q, *J*=8.72 Hz, 2 H) 6.05 (q, *J*=7.34 Hz, 1 H) 6.94 - 7.15 (m, 5 H) 7.28 (br d, *J*=7.58 Hz, 1 H) 7.49 (s, 1 H) 7.80 (dd, *J*=10.03, 5.26 Hz, 1 H) 8.05 - 8.15 (m, 2 H) 8.89 - 8.96 (m, 1 H); LCMS: m/z= 601.1 [M+1].

### Example 16: WX030 and WX031

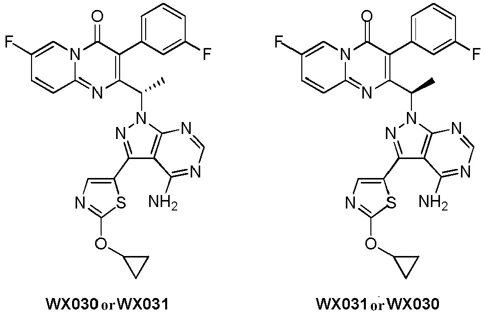

### Synthetic route:

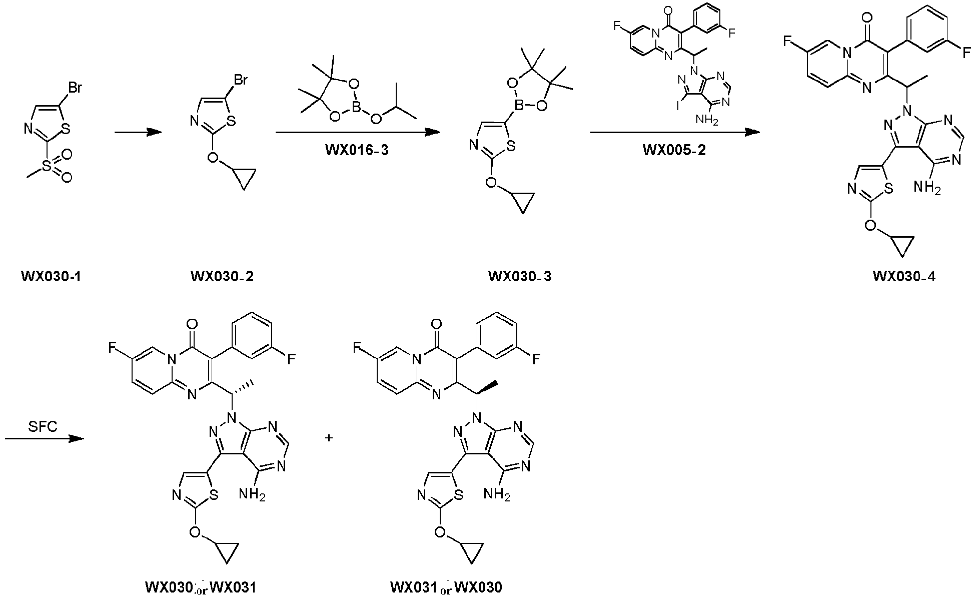

### Step 1: synthesis of compound WX030-2

**WX030-1** (1 g, 4.13 mmol, 1 *eq.),* cyclopropanol (359.82 mg, 6.20 mmol, 1.5 *eq.)* and tetrahydrofuran (10 mL) were sequentially added to a pre-dried flask (40 mL). After anitrogen purge, the reaction system was added with sodium hydride (330.39 mg, 8.26 mmol, 60% purity, 2 *eq.)* at 0 °C. The reaction system was stirred at 25 °C for 3 h. After the reaction was completed, the reaction system was added with methanol (3 mL), and concentrated under reduced pressure to give a crude product. The crude product was purified by silica gel column chromatography (petroleum ether:ethyl acetate = 20:1-1:1) to give the target compound **WX030-2.** ¹H NMR (400MHz, CDCl₃) δ 7.14 (s, 1H), 4.21 (tt, J=3.0, 6.0 Hz, 1H), 0.98 - 0.88 (m, 2H), 0.88 - 0.78 (m, 2H).

### Step 2: synthesis of compound WX030-3

To a dry flask (40 mL) were added **WX030-2** (0.6 g, 2.73 mmol, 1 *eq.),* **WX016-3** (2.03 g, 10.90 mmol, 2.22 mL, *4 eq.), n*-butyllithium (2.5 M, 3.27 mL, 3 *eq.)* and tetrahydrofuran (6 mL). After a nitrogen purge, *N*,*N*,*N*',*N*'-tetramethylethylenediamine (411.85 mg, 3.54 mmol, 534.88 µL, 1.3 *eq.)* was slowly added at -78 °C, and the system was stirred at -78 °C for 1 h and at 25 °C for 1 h. After the reaction was completed, the reaction system was slowly added with methanol (6 mL) to quench the reaction, and the solvent was evaporated under reduced pressure to give crude **WX030-3,** which was used in the next step without purification.

### Step 3: synthesis of compound WX030-4

To a dry flask (8 mL), **WX030-3** (0.9 g, 2.70 mmol, 2 *eq.),* **WX005-2** (734.80 mg, 1.35 mmol, 1 *eq.),* potassium carbonate (558.73 mg, 4.04 mmol, 3 *eq.)* and solvents dioxane (1.5 mL) and water (0.15 mL) were added. After a nitrogen purge, [1,1'-bis(diphenylphosphino)ferrocene]dichloropalladium(II) (197.20 mg, 269.51 µmol, 0.2 *eq.)* was added, followed by another nitrogen purge. The reaction system was then stirred at 70 °C for 16 h. After the reaction was completed, the reaction system was cooled and the solvent was evaporated under reduced pressure to give a crude product. The crude product was purified by silica gel thin layer chromatography (dichloromethane:methanol = 1:0-100:1) to give a crude product (1 g). The crude product was separated by preparative high performance liquid chromatography (column: Xtimate C18 10 µm 250 mm × 80 mm; mobile phase: [water (10 mM NH₄HCO₃)-acetonitrile]; B%: 20%-55% (B was acetonitrile), 20 min) to give the target compound **WX030-4**.¹H NMR (400MHz, CDCl₃) δ 8.95 - 8.92 (m, 1H), 8.22 (s, 1H), 7.66 (s, 1H), 7.66 (d, J=11.3 Hz, 1H), 7.46 (s, 1H), 7.05 (d, J=7.3 Hz, 1H), 7.00 - 6.88 (m, 2H), 6.15 (q, J=7.0 Hz, 1H), 5.52 (s, 2H), 4.30 - 4.25 (m, 1H), 3.92 (s, 1H), 1.91 (d, J=7.1 Hz, 3H), 1.01 - 0.93 (m, 2H), 0.93 - 0.87 (m, 2H).

### Step 4: synthesis of compounds WX030 and WX031

**WX030-4** was separated by supercritical fluid chromatography (column: DAICEL CHIRALPAK AD-H (250 mm × 30 mm, 5 µm); mobile phase: [pure isopropanol]; B%: 50%-50% (B was pure isopropanol), 8 min) to give **WX030** (retention time: 2.09 min) and **WX031** (retention time: 2.39 **min).WX030:** ¹H NMR (400MHz, DMSO-*d*₆) δ =8.93 (dd, *J*=2.9, 4.6 Hz, 1H), 8.12 (ddd, *J*=2.8, 7.1, 9.9 Hz, 1H), 8.06 (s, 1H), 7.81 (dd, *J*=5.3, 9.8 Hz, 1H), 7.50 (s, 1H), 7.33 - 7.23 (m, 1H), 7.08 - 6.92 (m, 4H), 6.06 (q, *J*=6.8 Hz, 1H), 4.34 (tt, *J*=3.0, 5.9 Hz, 1H), 1.79 (d, *J*=6.8 Hz, 3H), 0.93 - 0.85 (m, 4H); **MS,** m/z= 559.0[M+1].**WX031:** 1H NMR (400MHz, DMSO-*d*₆) δ8.92 (dd, *J*=2.9, 4.6 Hz, 1H), 8.11 (ddd, *J*=2.8, 7.2, 9.8 Hz, 1H), 8.05 (s, 1H), 7.80 (dd, *J*=5.3, 9.7 Hz, 1H), 7.49 (s, 1H), 7.34 - 7.20 (m, 1H), 7.07 - 6.92 (m, 4H), 6.05 (q, *J*=6.9 Hz, 1H), 4.34 (tt, *J*=3.1, 5.9 Hz, 1H), 1.78 (d, *J*=7.0 Hz, 3H), 0.94 - 0.85 (m, 4H); **MS,** m/z= 559.0[M+1].

### Example 17: WX032 and WX033

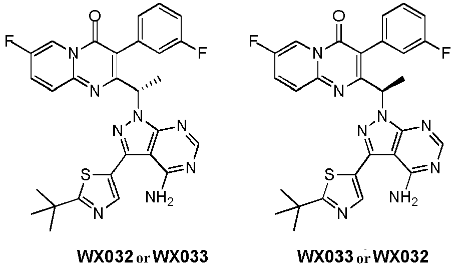

### Synthetic route:

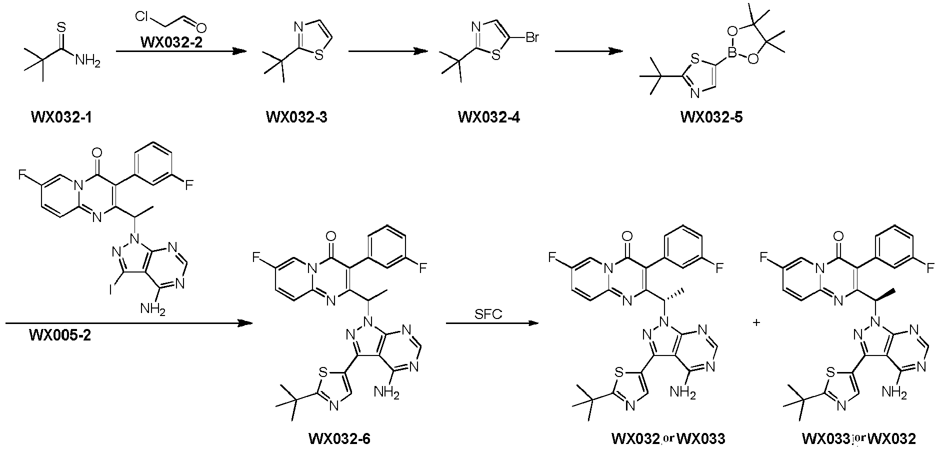

### Step 1: synthesis of compound WX032-3

**WX032-1** (2.5 g, 21.33 mmol, 1 *eq.),* **WX032-2** (6.70 g, 85.32 mmol, 5.49 mL, 4 *eq.)* and ethanol (40 mL) were added to a pre-dried flask, followed by acetic acid (2.56 g, 42.66 mmol, 2.44 mL). After three nitrogen purges, the reaction system was incubated at 50 °C for 1 h and then at 80 °C for 15 h. After the reaction was completed, the system was concentrated in vacuum, and the concentrated mixture was adjusted to a neutral pH with saturated sodium bicarbonate at 0 °C, and extracted with dichloromethane (20 mL × 3). The organic phase was collected, washed with saturated brine (30 mL × 1), dried over anhydrous sodium sulfate, and filtered, and the filtrate was concentrated under reduced pressure by rotary evaporation. The residue was purified by silica gel column chromatography (eluent: ethyl acetate/petroleum ether = 0-1%) to give the target compound **WX032-3.** ¹H NMR (400 MHz, DMSO-*d*₆) δ 1.37 (s, 9 H) 7.55 (d, J=3.31 Hz, 1 H) 7.68 (d, *J*=3.31 Hz, 1 H).

### Step 2: synthesis of compound WX032-4

**WX032-3** (1.2 g, 4.25 mmol, 1 *eq.)* and acetonitrile (25 mL) were added to a pre-dried flask, followed by bromosuccinimide (1.51 g, 8.50 mmol, 2 *eq.).* After three nitrogen purges, the reaction system was incubated at 25 °C for 16 h. After the reaction was completed, the system was concentrated in vacuum to give a crude product. The crude product was purified by silica gel column chromatography (eluent: ethyl acetate/petroleum ether = 0-1%) to give the target compound **WX032-4.** ¹H NMR (400 MHz, DMSO-*d*₆) δ 1.35 (s, 9 H) 7.73 (s, 1 H).

### Step 3: synthesis of compound WX032-5

**WX032-4** (900 mg, 4.09 mmol,1 *eq.)* and tetrahydrofuran (20 mL) were added to a pre-dried flask, followed by **WX016-3** (3.04 g, 16.35 mmol, 3.34 mL, 4 *eq.)* and *N*,*N*,*N*',*N*'-tetramethylethylenediamine (617.66 mg, 5.32 mmol, 802.16 µL, 1.3 *eq.).* After three nitrogen purges, *n*-Butyllithium (2.5 M, 4.91 mL) was slowly added dropwise at -78 °C, and the reaction system was incubated at -78 °C for 2 h and then stirred at 25 °C for 1 h. After the reaction was completed, the reaction system was added with methanol to quench the reaction, and concentrated by a vacuum pump to give **WX032-5.**

### Step 4: synthesis of compound WX032-6

**WX005-2** (100 mg, 183.39 µmol, 1 *eq.)* and **WX032-5** (196.00 mg, 366.78 µmol, 2 *eq.)* were added to a pre-dried flask, followed by dioxane (3 mL), water (1.5 mL) and potassium acetate (53.99 mg, 550.17 µmol, 3 *eq.).* After three nitrogen purges, [1,1'-bis(diphenylphosphino)ferrocene]palladium(II)dichloride (40.26 mg, 55.02 µmol, 0.3 *eq.)* was added, followed by another three nitrogen purges. The reaction system was incubated at 70 °C for 16 h. After the reaction was completed, the reaction system was cooled to room temperature, and concentrated by a vacuum pump. The residuewas dissolved with dichloromethane (10 mL), and added with water (10 mL). The aqueous phase was extracted with dichloromethane (20 mL × 2). The organic phases were collected and combined, washed with saturated brine (10 mL), dried over anhydrous sodium sulfate, filtered, and concentrated under reduced pressure to give a crude product. The crude product was separated by preparative high performance liquid chromatography (column: Xtimate C18 150 mm × 40 mm × 10 µm; mobile phase: [water (10 mM NH₄HCO₃)-acetonitrile]; B%: 50%-70% (B was acetonitrile), 7 min) to give **WX032-6**.¹H NMR (400 MHz, CDCl₃) δ 1.50 (s, 9 H) 1.93 (s, 2 H) 5.57 (s, 2 H) 6.18 (q, *J*=7.16 Hz, 1 H) 6.90 - 6.99 (m, 2 H) 7.07 (d, *J*=7.89 Hz, 1 H) 7.48 (dd, *J*=7.45, 3.07 Hz, 1 H) 7.66 - 7.69 (m, 2 H) 7.89 (s, 1 H) 8.23 (s, 1 H) 8.93 - 8.97 (m, 1 H).

### Step 5: synthesis of compounds WX032 and WX033

**WX032-6** (171 mg, 306.12 µmol, 1 *eq.)* was separated by supercritical fluid chromatography (column: DAICEL CHIRALPAK AS (250 mm × 30 mm, 10 µm); mobile phase: [0.1% ammonia in isopropanol]; B%: 48%-48% (B was 0.1% ammonia in isopropanol), 9 min) to give **WX032** (retention time: 2.98 min) and **WX033** (retention time: 3.26 min).**WX032:** ¹H NMR (400 MHz, CDCl₃) δ 1.50 (s, 9 H) 1.92 (d, *J*=6.97 Hz, 3 H) 5.66 (br s, 2 H) 6.18 (q, *J*=6.97 Hz, 1 H) 6.88 - 7.00 (m, 2 H) 7.07 (d, *J*=7.58 Hz, 1 H) 7.27 - 7.31 (m, 1 H) 7.61 - 7.74 (m, 2 H) 7.89 (s, 1 H) 8.22 (s, 1 H) 8.95 (dd, *J*=3.79, 2.69 Hz, 1 H); LCMS: m/z= 559.1 [M+1].**WX033:** ¹H NMR(400 MHz, CDCl₃) δ 1.50 (s, 9 H) 1.92 (d, *J*=6.97 Hz, 3 H) 5.66 (br s, 2 H) 6.18 (q, *J*=6.97 Hz, 1 H) 6.88 - 7.00 (m, 2 H) 7.07 (d, *J*=7.58 Hz, 1 H) 7.27 - 7.31 (m, 1 H) 7.61 - 7.74 (m, 2 H) 7.89 (s, 1 H) 8.22 (s, 1 H) 8.95 (dd, *J*=3.79, 2.69 Hz, 1 H); LCMS: m/z= 559.1 [M+1].

### Example 18: WX034 and WX035

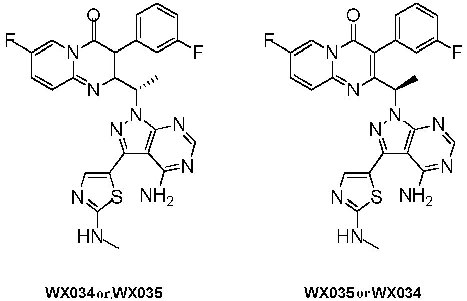

### Synthetic route:

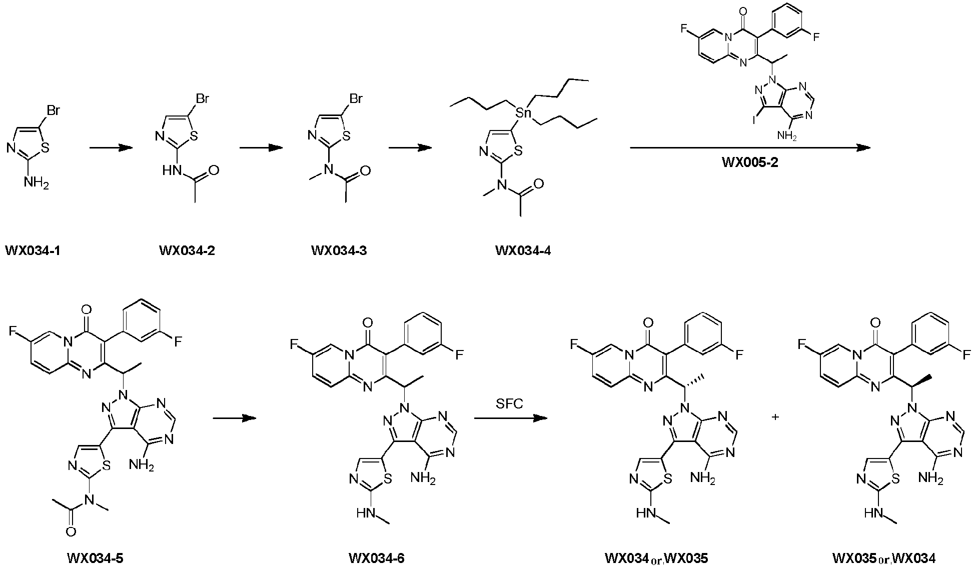

### Step 1: synthesis of compound WX034-2

**WX034-1** (3 g, 11.54 mmol, 1 *eq.)* and dichloromethane (30 mL) were added to a flask, followed by pyridine (5.48 g, 69.24 mmol, 5.59 mL, 6 *eq.).* Acetic anhydride (1.30 g, 12.69 mmol, 1.19 mL, 1.1 *eq.)* was added dropwise and the reaction system was incubated in a 30 °C oil bath for 1 h. After the reaction was completed, 1 N hydrochloric acid (30 mL) and dichloromethane (90 mL) were added. The organic phase was separated, dried overanhydrous sodium sulfate, filtered, and concentrated by rotary evaporation to give a crude **WX034-2** product.

### Step 2: synthesis of compound WX034-3

**WX034-2** (3.6 g, 16.28 mmol, 1 *eq.)* and *N*,*N-*dimethylformamide (30 mL) was added to a flask. Then potassium carbonate (5.63 g, 40.71 mmol, 2.5 *eq.)* was added and the reaction system was stirred for 0.5 h, followed by adding iodomethane (9.25 g, 65.14 mmol, 4.05 mL, 4 *eq.)* and another stirring in a 30 °C oil bath for 11.5 h. After the reaction was completed, the reaction system was concentrated by rotary evaporation, added with ethyl acetate (150 mL), stirred for 0.2 h, filtered, and concentrated to give a crude product. The crude product was purified by silica gel column chromatography (petroleum ether:ethyl acetate = 5:1-2:1) to give the target compound

**WX034-3.**

### Step 3: synthesis of compound WX034-4

**WX034-3** (0.8 g, 3.40 mmol, 1 *eq.),* dioxane (5 mL), tetrakis(triphenylphosphine)palladium(0) (589.82 mg, 510.42 µmol, 0.15 *eq.)* was added to a flask, and tri-*n*-butyltin hydride (4.95 g, 17.01 mmol, 4.50 mL, 5 *eq.)* was added dropwise. After three nitrogen purges, the reaction system was incubated in an 80 °C oil bath for 3 h. After the reaction was completed, the reaction system was concentrated by rotary evaporation to give a crude product, and the crude product was purified by silica gel thin layer chromatography (petroleum ether:ethyl acetate = 3:1) to give the target compound **WX034-4.**

### Step 4: synthesis of compound WX034-5

Toa 40-mL dry flask, compounds **WX005-2** (350.00 mg, 641.87 µmol, 1 *eq.)* and **WX034-4** (371.53 mg, 834.43 µmol, 1.3 *eq.)* and dioxane (21 mL) were added. After a nitrogen purge, tetrakis(triphenylphosphine)palladium(0) (74.17 mg, 64.19 µmol, 0.1 *eq.)* was added and the reaction system was stirred at 100 °C for 8 h. After the reaction was completed, the reaction system was cooled and the solvent was evaporated under reduced pressure to give a crude product. The crude product was purified by silica gel column chromatography (dichloromethane:methanol = 1:0-100:1) to give **WX034-5**.¹H NMR (400MHz, CD₃OD) δ 8.97 (br s, 1H), 8.05 (s, 1H), 8.02 - 7.94 (m, 1H), 7.91 - 7.84 (m, 1H), 7.73 (s, 1H), 7.20 (br s, 1H), 6.84 (br t, J=9.0 Hz, 2H), 6.20 (br d, J=7.0 Hz, 1H), 3.75 (s, 3H), 2.44 (s, 3H), 1.91 (d, J=7.0 Hz, 3H).

### Step 5: synthesis of compound WX034-6

To a pre-dried 40-mL flask were added **WX034-5** (0.4 g, 697.38 µmol, 1 *eq.)* and methanol (8 mL), followed by potassium carbonate (192.77 mg, 1.39 mmol, 2 *eq.).* The reaction system was stirred at 40 °C for 10 h. After the reaction was completed, the reaction system was cooled and the solvent was evaporated under reduced pressure to give a crude product. The crude product was separated by preparative high performance liquid chromatography (column: Xtimate C18 150 mm × 40 mm × 10 µm; mobile phase: [water (10 mM NH₄HCO₃)-acetonitrile]; B%: 40%-60% (B was acetonitrile), 7 min) to give the target compound **WX034-6.** ¹H NMR (400MHz, DMSO-*d*₆) δ 8.92 (dd, J=2.8, 4.6 Hz, 1H), 8.11 (ddd, J=2.9, 7.1, 9.8 Hz, 1H), 8.03 - 8.00 (m, 1H), 7.81 (dd, J=5.2, 9.8 Hz, 1H), 7.76 (q, J=4.6 Hz, 1H), 7.34 - 7.30 (m, 1H), 7.29 - 7.20 (m, 1H), 7.02 - 6.90 (m, 4H), 6.01 (q, J=7.0 Hz, 1H), 2.86 (d, J=4.8 Hz, 3H), 1.77 (d, J=7.0 Hz, 3H).

### Step 6: synthesis of compounds WX034 and WX035

Compound **WX034-6** was separated by supercritical fluid chromatography (column: DAICEL CHIRALPAK AD (250 mm × 50 mm, 10 µm); mobile phase: [isopropanol]; B%: 50%-50% (B was isopropanol), 4.5 min) to give **WX034** (retention time: 3.48 min) and **WX035** (retention time: 4.18 min).**WX034:** ¹H NMR (400MHz, DMSO-*d*₆) δ 8.92 (dd, J=2.8, 4.6 Hz, 1H), 8.11 (ddd, J=2.9, 7.1, 9.8 Hz, 1H), 8.03 - 8.00 (m, 1H), 7.81 (dd, J=5.2, 9.8 Hz, 1H), 7.76 (q, J=4.6 Hz, 1H), 7.34 - 7.30 (m, 1H), 7.29 - 7.20 (m, 1H), 7.02 - 6.90 (m, 4H), 6.01 (q, J=7.0 Hz, 1H), 2.86 (d, J=4.8 Hz, 3H), 1.77 (d, J=7.0 Hz, 3H); **MS:** m/z= 531.9[M+1].**WX035:** ¹H NMR (400MHz, DMSO-*d*₆) δ 8.92 (dd, J=2.8, 4.6 Hz, 1H), 8.11 (ddd, J=2.9, 7.1, 9.8 Hz, 1H), 8.03 - 8.00 (m, 1H), 7.81 (dd, J=5.2, 9.8 Hz, 1H), 7.76 (q, J=4.6 Hz, 1H), 7.34 - 7.30 (m, 1H), 7.29 - 7.20 (m, 1H), 7.02 - 6.90 (m, 4H), 6.01 (q, J=7.0 Hz, 1H), 2.86 (d, J=4.8 Hz, 3H), 1.77 (d, J=7.0 Hz, 3H); **MS:** m/z= 531.9[M+1].

### Example 19: WX036 and WX037

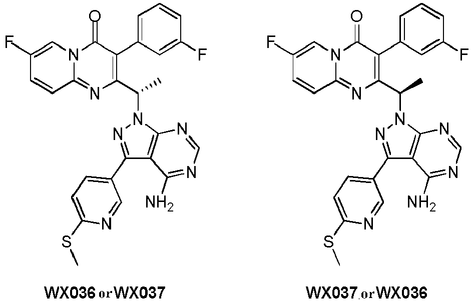

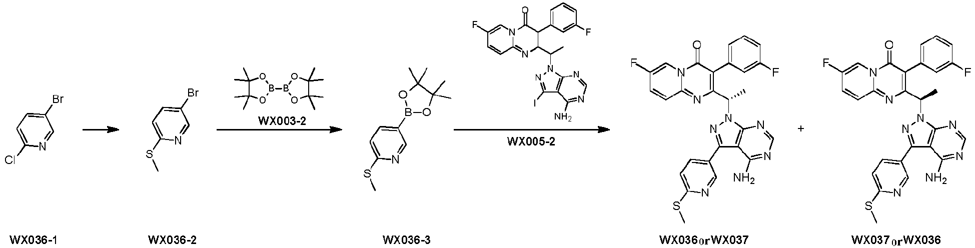

### Step 1: synthesis of compound WX036-2

**WX036-1** (4 g, 20.79 mmol, 1 *eq.)* was dissolved in a mixture of 20% (w/w) aqueous sodium thiomethoxide solution (44.00 g, 125.55 mmol, 40.00 mL, 6.04 *eq.)* and *N*,*N-*dimethylformamide (80 mL). The reaction system was stirred at 100 °C for 16 h. After the reaction was completed, the reaction system was concentrated, and the residue was resuspended in water (40 mL), and extracted with ethyl acetate (30 mL × 2). The organic phase was concentrated to give **WX036-2.**

### Step 2: synthesis of compound WX036-3

Under nitrogen atmosphere, **WX036-2** (2 g, 9.80 mmol, 1 *eq.),* **WX003-2** (3.00 g, 11.81 mmol, 1.21 *eq.),* [1,1'-bis(diphenylphosphino)ferrocene]dichloropalladium(II) (800.00 mg, 1.09 mmol, 1.12e-1 *eq.)* and potassium acetate (2.00 g, 20.38 mmol, 2.08 *eq.)* were added to dioxane (100 mL). The reaction system was stirred at 100 °C for 16 h. After the reaction was completed, the reaction system was concentrated, and the crude product was purified by silica gel column chromatography (petroleum ether:ethyl acetate = 1:0-20:1) to give **WX036-3.**

### Step 3: synthesis of compounds WX036 and WX037

Under nitrogen atmosphere, **WX005-2** (0.25 g, 458.48 µmol, 1 *eq.),* **WX036-3** (150.00 mg, 597.25 µmol, 1.30 *eq.),* [1,1'-bis(diphenylphosphino)ferrocene]dichloropalladium(II) (50.00 mg, 68.33 µmol, 1.49e-1 *eq.)* and potassium carbonate (150.00 mg, 1.09 mmol, 2.37 *eq.)* were added to a mixture of dioxane (20 mL) and water (5 mL). The reaction system was stirred at 100 °C for 16 h. After the reaction was completed, the reaction system was added with water, and extracted with ethyl acetate. The organic phase was concentrated, and the crude product was purified by silica gel column chromatography (ethyl acetate/petroleum ether = 1:1-1:0 to dichloromethane/methanol =20:1). The intermediate product was separated by supercritical fluid chromatography (column: DAICEL CHIRALPAK AS-H (250 mm × 30 mm, 5 µm); mobile phase: [0.1% ammonia in ethanol]; B%: 45%-45% (B was 0.1% ammonia in ethanol), min) to give **WX036** (retention time: 4.438 min) and **WX037** (retention time: 4.953 **min).WX036:** ¹H NMR (400 MHz, CDCl₃) δ 8.87 (dd, J=3.89, 2.64 Hz, 1 H) 8.65 (d, J=1.76 Hz, 1 H) 8.15 (s, 1 H) 7.72 (dd, J=8.28, 2.26 Hz, 1 H) 7.51 - 7.66 (m, 2 H) 7.23 - 7.27 (m, 1 H) 7.19 (s, 1 H) 6.98 (d, J=7.53 Hz, 1 H) 6.78 - 6.90 (m, 2 H) 6.13 (q, J=7.03 Hz, 1 H) 5.31 (br s, 2 H) 2.54 (s, 3 H) 1.87 (d, J=7.03 Hz, 3 H); LCMS: m/z= 543.1[M+1].**WX037**: ¹H NMR (400 MHz, CDCl₃) δ 8.96 (dd, J=3.76, 2.76 Hz, 1 H) 8.74 (d, J=1.51 Hz, 1 H) 8.24 (s, 1 H) 7.81 (dd, J=8.41, 2.38 Hz, 1 H) 7.57 - 7.76 (m, 2 H) 7.33 (d, J=8.28 Hz, 1 H) 7.25 - 7.28 (m, 1 H) 7.07 (d, J=7.78 Hz, 1 H) 6.84 - 6.98 (m, 2 H) 6.22 (q, J=6.94 Hz, 1 H) 5.37 (br s, 2 H) 2.64 (s, 3 H) 1.96 (d, J=7.03 Hz, 3 H).LCMS: m/z= 543.1[M+1].

### Example 20: WX038 and WX039

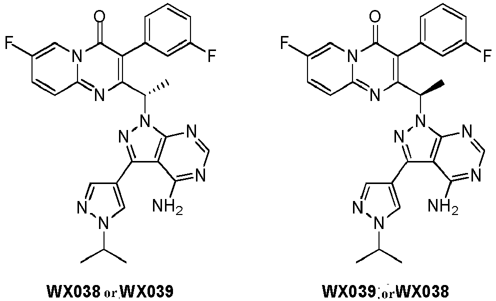

### Synthetic route:

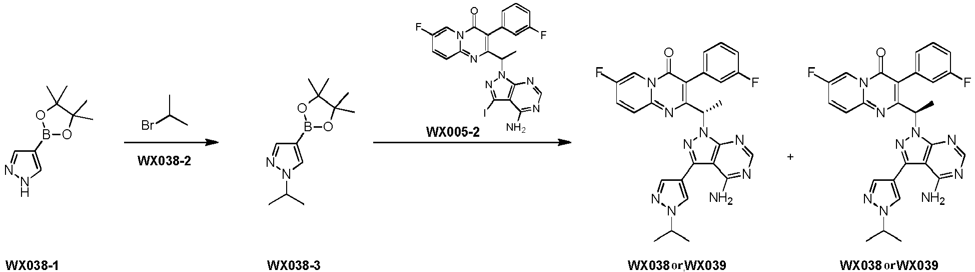

### Step 1: synthesis of compound WX038-3

Cesium carbonate (15.00 g, 46.04 mmol, 2.98 *eq.)* was added to a solution of **WX038-1** (3 g, 15.46 mmol) and **WX038-2** (3.60 g, 29.27 mmol, 2.75 mL) in acetonitrile (100 mL). The reaction system was stirred at 80 °C for 16 h. After the reaction was completed, ethyl acetate (100 mL) was added and the mixture was filtered. The filtrate was concentrated to give **WX038-3.**

### Step 2: synthesis of compounds WX038 and WX039

Under nitrogen atmosphere, **WX005-2** (0.3 g, 550.17 µmol), **WX038-3** (225.00 mg, 952.92 µmol), [1,1'-bis(diphenylphosphino)ferrocene]dichloropalladium(II) (75.00 mg, 102.50 µmol, 1.86e-1 *eq.),* and potassium carbonate (300.00 mg, 2.17 mmol, 3.95 *eq.)* were added to a flask, followed by water (10 mL) and dioxane (50 mL). The reaction system was stirred at 100 °C for 2 h. After the reaction was completed, the reaction system was concentrated to remove dioxane, and extracted with ethyl acetate (30 mL × 2). The organic phase was concentrated and the crude product was purified by silica gel column chromatography (ethyl acetate/petroleum ether = 1:1-1:0 to dichloromethane/methanol = 20:1) to give a crude product. The crude product was separated by supercritical fluid chromatography (column: Phenomenex-Amylose-1 (250 mm × 30 mm, 5 µm); mobile phase: [0.1% ammonia in ethanol]; B%: 35%-35% (B was 0.1% ammonia in ethanol), min) to give **WX038** (retention time: 4.723 min) and **WX039** (retention time: 5.470 min).**WX038**:¹HNMR (400 MHz, CDCl₃) δ 8.96 (dd, J=4.02, 2.76 Hz, 1 H) 8.20 (s, 1 H) 7.63 - 7.85 (m, 4 H) 7.40 - 7.58 (m, 1 H) 7.04 (br d, J=7.28 Hz, 1 H) 6.92 (br t, J=7.53 Hz, 2 H) 6.18 (q, J=7.03 Hz, 1 H) 5.49 (s, 2 H) 4.49 - 4.67 (m, 1 H) 1.94 (d, J=7.03 Hz, 3 H) 1.50 - 1.61 (m, 6 H); **MS:** m/z= 528.2[M+1].**WX039**:¹H NMR (400 MHz, CDCl₃) δ 8.87 (dd, J=4.02, 2.76 Hz, 1 H) 8.10 (s, 1 H) 7.71 (d, J=3.51 Hz, 2 H) 7.48 - 7.66 (m, 2 H) 7.17 (br s, 1 H) 6.95 (br d, J=7.53 Hz, 1 H) 6.82 (br t, J=7.78 Hz, 2 H) 6.09 (q, J=7.03 Hz, 1 H) 5.55 (br s, 2 H) 4.49 (spt, J=6.69 Hz, 1 H) 1.85 (d, J=7.03 Hz, 3 H) 1.50 (d, J=6.78 Hz, 6 H);**MS:** m/z= 528.2[M+1].

### Example 21: WX040 and WX041

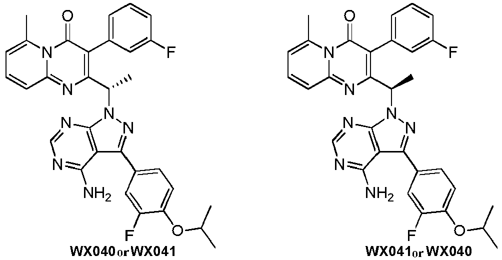

### Synthetic route:

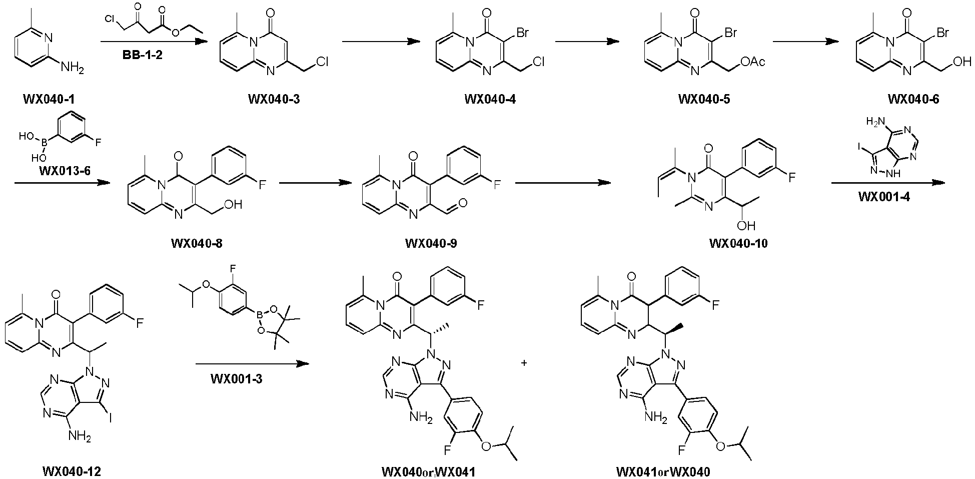

### Step 1: synthesis of compound WX040-3

Compounds WX040-1(3 g, 27.74 mmol, 1 *eq.)* and**BB-1-2** (7 g, 42.53 mmol, 1.53 *eq.)* were dissolved in polyphosphoric acid (40 mL) and the reaction system was incubated at 125 °C for 16 h. Water (200 mL) was added to the reaction system, and sodium hydroxide was added to adjust the pH to 8. A solid was precipitated. Dichloromethane (100 mL × 3) was added for extraction, and the organic phase was concentrated by rotary evaporation to give the target compound **WX040-3.**

### Step 2: synthesis of compound WX040-4

Compound **WX040-3** (3.2 g, 14.27 mmol, 1 *eq.;* 93.06% purify) and NBS (2.58 g, 14.52 mmol, 1.02 *eq.)* were dissolved in acetonitrile (60 mL), and the reaction system was stirred at 10 °C for 16 h. The reaction system was concentrated by rotary evaporation, added with water (100 mL), and extracted with dichloromethane (100 mL × 3). The organic phase was concentrated to give the target compound **WX040-4.**

### Step 3: synthesis of compound WX040-5

Compound **WX040-4** (3.5 g, 12.17 mmol, 1 *eq.;* crude) and potassium acetate (1.80 g, 18.34 mmol, 1.51 *eq.)* were dissolved in *N*,*N-*dimethylformamide (40 mL), and the reaction system was stirred at 40 °C for 3.5 h. *N*,*N-*dimethylformamide was removed by an oil pump, and the residue was added with water (100 mL), and extracted with dichloromethane (100 mL × 3). The organic phase was concentrated by rotary evaporation to give the target compound **WX040-5.**

### Step 4: synthesis of compound WX040-6

Compound **WX040-5** (3.5 g, 11.25 mmol, 1 *eq.*; crude) and hydrochloric acid (12 M, 3.50 mL, 3.73 *eq.)* were dissolved in 1,4-dioxane (60 mL), and the reaction system was incubated at 70 °C for 3 h. The solvent was removed by rotary evaporation and the residue was slurried with water. The mixture was filtered and the filtrate was extracted with dichloromethane (100 mL × 3). The organic phase was concentrated to give the target compound **WX040-6.**

### Step 5: synthesis of compound WX040-8

**WX040-6** (2.2 g, 8.18 mmol, 1 *eq.;* 87.24% purity), **WX013-6** (2.28 g, 16.28 mmol, 1.99 *eq.),* Pd(dppf)Cl₂ (0.3 g, 410.00 µmol, 5.01e-2 *eq.),* and potassium carbonate (4.33 g, 40.88 mmol, 5 *eq.)* were dissolved in acetonitrile (60 mL) and water (20 mL). After three nitrogen purges, the reaction system was incubated at 100 °C for 1 h. The reaction system was concentrated by rotary evaporation, diluted with water (200 mL), and extracted with dichloromethane (200 mL × 3). The organic phase was concentrated by rotary evaporation. The crude product was purified by silica gel column chromatography (methanol/ethyl acetate = 0-6%) to give the target compound **WX040-8.**

### Step 6: synthesis of compound WX040-9

DMSO (1.70 g, 21.76 mmol, 1.7 mL, 6.19 *eq.)* was dissolved in dichloromethane (10 mL), to which oxalyl chloride (1.45 g, 11.42 mmol, 1 mL, 3.25 *eq.)* was added. The mixture was stirred at -78 °C for 1 h before a solution of **WX040-8** (1 g, 3.52 mmol, 1 *eq.;* crude) in dichloromethane (20 mL) was added. The reaction system was stirred at -78 °C for 1 h before triethylamine (3.64 g, 35.92 mmol, 5 mL, 10.21 *eq.)* was added, and then the system was stirred at -78 °C for 1 h and at 15 °C for 1 h. The reaction system was diluted with water (40 mL), and extracted with dichloromethane (40 mL × 3). The organic phase was concentrated by rotary evaporation to give the target compound **WX040-9.**

### Step 7: synthesis of compound WX040-10

**WX040-9** (1.1 g, 3.90 mmol, 1 *eq.)* was dissolved in tetrahydrofuran (10 mL). At 0 °C, the reaction system was added with methylmagnesium bromide (3 M, 2.62 mL, 2.02 *eq.)* under nitrogen atmosphere and incubated at 10 °C for 5 h. Water was added to quench the reaction, and the reaction system was concentrated by rotary evaporation to give a crude product. The crude product was purified by silica gel column chromatography (methanol/dichloromethane = 0-6%) to give the target compound **WX040-10.**

### Step 8: synthesis of compound WX040-12

**WX040-10** (0.3 g, 1.01 mmol, 1 *eq.;* crude), **WX001-4** (240.00 mg, 919.46 µmol, 9.14e-1 *eq.)* and diisopropyl azodicarboxylate (312.00 mg, 1.54 mmol, 300.00 µL, 1.53 *eq.)* were dissolved in tetrahydrofuran (6 mL) and the reaction system was purged with nitrogen 3 times. Triphenylphosphine (420.00 mg, 1.60 mmol, 1.59 *eq.)* was added and the system was stirred at 45 °C for 3.5 h. The solvent was removed by rotary evaporation to give a crude product. The crude product was purified by silica gel column chromatography (methanol/dichloromethane = 0-6%) to give the target compound **WX040-12.**

### Step 9: synthesis of compounds WX040 and WX041

**WX040-12** (0.1 g, 184.73 µmol, 1 *eq.;* crude), **WX001-3** (99.88 mg, 356.53 µmol, 1.93 *eq.;* crude) and sodium carbonate (97.90 mg, 923.65 µmol, 5 *eq.)* were dissolved in *N*,*N-*dimethylformamide (4 mL), ethanol (2 mL) and water (2 mL). Pd(PPh₃)₄ (0.025 g, 21.63 µmol, 1.17e-1 *eq.)* was added under nitrogen atmosphere and the reaction system was stirred at 100 °C for 16 h. The reaction system was concentrated by rotary evaporation. The crude product was purified by silica gel column chromatography (methanol:dichloromethane = 0-10%), and was separated by conventional preparative high performance liquid chromatography (column: Boston Prime C18 150 mm × 30 mm, 5 µm; mobile phase: [water (0.05% ammonia v/v)-acetonitrile]; B%: 58%-88% (B was acetonitrile), 8 min) and supercritical fluid chromatography (column: DAICEL CHIRALPAK AD (250 mm × 30 mm,10 µm); mobile phase: 0.1% ammonia in ethanol; B%: 35%-35% (B was 0.1% ammonia in ethanol), min) to give **WX040** (retention time: 3.360) and **WX041** (retention time: 3.687).**WX040:** ¹H NMR (400 MHz, CDCl₃) δ 1.33 (d, J=6.02 Hz, 6 H) 1.84 (d, J=7.03 Hz, 3 H) 2.93 (s, 3 H) 3.65 (q, J=7.03 Hz, 1 H) 4.55 (spt, J=6.11 Hz, 1 H) 5.41 (br s, 1 H) 6.02 (q, J=6.94 Hz, 1 H) 6.58 - 6.65 (m, 1 H) 6.77 (br dd, J=8.41, 6.90 Hz, 2 H) 6.93 (br d, J=7.53 Hz, 1 H) 7.03 (t, J=8.41 Hz, 1 H) 7.11 - 7.17 (m, 1 H) 7.28 (br d, J=8.28 Hz, 1 H) 7.32 - 7.39 (m, 3 H) 8.12 (s, 1 H); **WX041:** ¹H NMR (400 MHz, CDCl₃) δ 1.33 (d, J=6.02 Hz, 6 H) 1.84 (d, *J*=7.03 Hz, 3 H) 2.93 (s, 3 H) 4.55 (spt, J=6.07 Hz, 1 H) 5.42 (br s, 2 H) 6.02 (q, J=7.03 Hz, 1 H) 6.55 - 6.63 (m, 1 H) 6.72 - 6.82 (m, 2 H) 6.93 (br d, J=7.53 Hz, 1 H) 7.03 (t, J=8.41 Hz, 1 H) 7.09 - 7.18 (m, 1 H) 7.28 (br d, J=8.28 Hz, 1 H) 7.32 - 7.41 (m, 3 H) 8.12 (s, 1 H).

### Example 22: WX042 and WX043

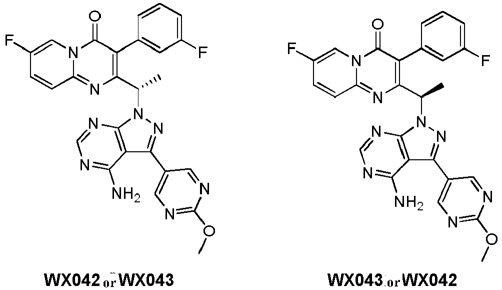

### Synthetic route:

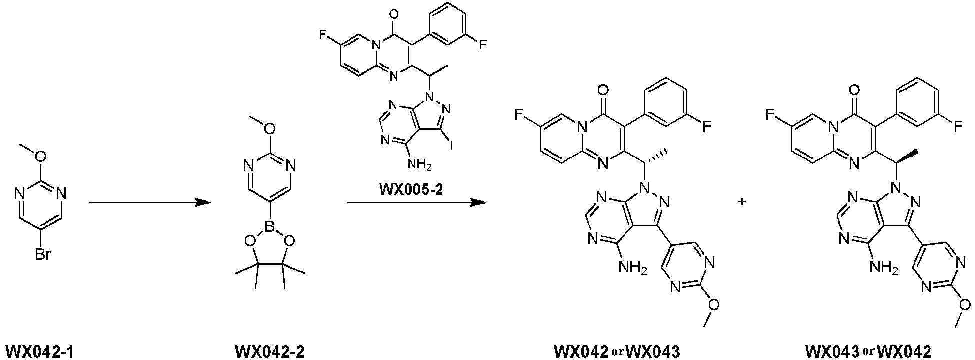

### Step 1: synthesis of target compound WX042-2

**WX042-1** (5 g, 26.45 mmol, 1 *eq.)* was dissolved in a mixed solution of ethylene glycol dimethyl ether (21 mL), ethanol (6 mL), water (2.1 mL) and toluene (12.6 mL), and bis(pinacolato)diboron (15.25 g, 60.05 mmol, 2.27 *eq.)* and potassium acetate (7.81 g, 79.61 mmol, 3.01 *eq.)* were added. Under nitrogen atmosphere, Pd(dppf)Cl₂.CH₂Cl₂ (4.32 g, 5.29 mmol, 0.2 *eq.)* was added, and the reaction system was incubated at 85 °C for 6 h. The organic solvent was removed by rotary evaporation, and the residue was added with water (about 20 mL), and extracted with dichloromethane (20 mL × 3). The organic phases were combined, dried over anhydrous sodium sulfate, filtered and concentrated. The crude product was separated by flash silica gel column chromatography (petroleum ether:ethyl acetate = 5:1) to give the target compound **WX042-2**.¹H NMR (400MHz, CD₃OD) δ 8.90 - 8.67 (s, 2H), 4.05 (s, 3H), 1.11 (s, 12H).

### Step 2: synthesis of compounds WX042 and WX043

**WX005-2** (1.5 g, 2.75 mmol, 1 **eq.)** and **WX042-2** (649.41 mg, 2.75 mmol, 1 *eq.)* was dissolved in a mixed solvent of *N*,*N-*dimethylformamide (100 mL), ethanol (50 mL) and water (50 mL) before potassium carbonate (1.14 g, 8.25 mmol, 3.0 *eq.)* was added. Under nitrogen atmosphere, Pd(PPh₃)₄ (635.76 mg, 550.17 µmol, 0.2 *eq.)* was added, and the reaction system was stirred at 100 °C for 2 h. The organic solvent was removed by rotary evaporation, and the residue was added with water (about 100 mL), and extracted with dichloromethane (100 mL × 3). The organic phases were combined, dried over anhydrous sodium sulfate, filtered and concentrated. The crude product was purified by preparative thin layer chromatography (dichloromethane:methanol = 15:1), slurried (petroleum ether/dichloromethane = 100 mL/10 mL) and separated by supercritical fluid chromatography (column: Xtimate C18 150 mm × 25 mm × 5 µm; mobile phase: [water (0.225% FA)-acetonitrile]; B%: 32%-52% (B was acetonitrile), 7 min) to give **WX042** (retention time: 2.078min) and **WX043** (retention time: 2.558min).**WX042:** ¹H NMR (400MHz, CDCl₃) δ 9.97 - 8.95 (m, 1H), 8.83 (s, 2H), 8.28 (s, 1H), 7.88 - 7.55 (m, 2H), 7.33-7.31 (m 1H), 7.11-7.09 (m, 1H), 6.98 - 6.94 (m, 2H), 6.25 - 6.19 (q, J=7.1 Hz, 1H), 5.32 - 5.28 (m, 2H), 4.18 (s, 3H), 1.96 (d, J=7.0 Hz, 3H). **WX043:** ¹H NMR (400MHz, CDCl₃) δ 8.97 (br d, J=3.5 Hz, 1H), 8.85 (s, 2H), 8.28 (s, 1H), 7.97 - 7.60 (m, 2H), 7.32 -7.31(m, 1H), 7.11 (d, J=7.5 Hz, 1H), 7.04 - 6.80 (m, 2H), 6.22 (q, J=7.1 Hz, 1H), 5.28 (m, 2H), 4.12 (s, 3H), 1.96 (d, J=7.0 Hz, 3H).

### Example 23: WX044 and WX045

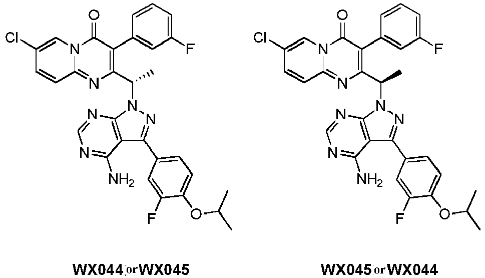

### Synthetic route:

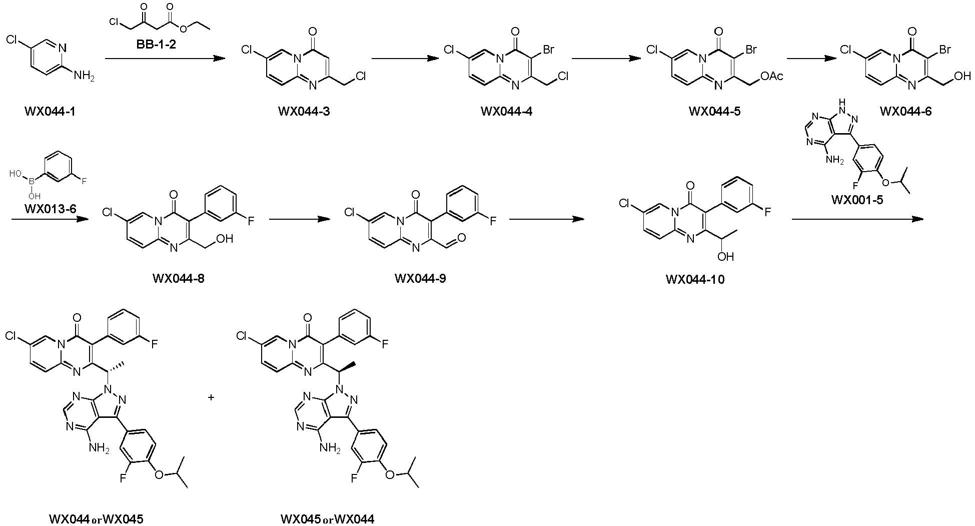

### Step 1: synthesis of target compound WX044-3

**WX044-1** (3 g, 23.34 mmol, 1 *eq.),* **BB-1-2** (4.99 g, 30.34 mmol, 1.3 *eq.)* and polyphosphoric acid (30 mL) were mixed and incubated at 125 °C for 6 h. Water (about 100 mL) was added to the flask to give a clear solution, and pH was adjusted to 8 with sodium hydroxide (about 20 g). Small solid particulates were precipitated. The mixture was filtered. The filter cake was mixed with toluene (about 20 mL) and the mixture was concentrated by rotary evaporation to give the target compound **WX044-3**.¹H NMR (400MHz, DMSO-*d*₆) δ 8.93 (d, *J*=2.0 Hz, 1H), 8.04 (dd, *J*=2.3, 9.3 Hz, 1H), 7.73 (d, *J*=9.5 Hz, 1H), 6.60 (s, 1H), 4.67 (s, 2H).

### Step 2: synthesis of target compound WX044-4

**WX044-3** (3.6 g, 15.72 mmol, 1 *eq.;* crude) was dissolved in acetic acid (90 mL) before NBS (3.08 g, 17.29 mmol, 1.1 *eq.)* was added. The reaction system was stirred at 20 °C for 5 h. Water (about 100 mL) was added to the flask and a solid was precipitated. The mixture was filtered. The filter cake was mixed with toluene (about 20 mL) and the mixture was concentrated by rotary evaporation to give the target compound **WX044-4,** which was a brownish yellow solid.

### Step 3: synthesis of target compound WX044-5

**WX044-4** (4 g, 12.99 mmol, 1 *eq.)* was dissolved in *N*,*N-*dimethylformamide (30 mL) before potassium acetate (1.91 g, 19.48 mmol, 1.5 *eq.)* was added. The reaction system was stirred at 40 °C for 3.5 h. Water (about 100 mL) was added to the flask. The mixture was filtered and the filter cake was mixed with toluene (about 20 mL). The mixture was concentrated by rotary evaporation to give the target compound **WX044-5.**

### Step 4: synthesis of target compound WX044-6

**WX044-5** (4 g, 12.06 mmol, 1 *eq.;* crude) was dissolved in 1,4-dioxane (30 mL) before concentrated hydrochloric acid (12 M, 4.00 mL, 3.98 *eq.)* was added. The reaction system was stirred at 70 °C for 3 h. Water (about 100 mL) was to the flask and a solid were precipitated. The mixture was filtered. The filter cake was dried by rotary evaporation to give the target compound **WX044-6.**¹H NMR (400MHz, DMSO-*d*₆) δ 8.94 (d, *J*=2.0 Hz, 1H), 8.07 (dd, *J*=2.1, 9.4 Hz, 1H), 7.77 (d, *J*=9.5 Hz, 1H), 4.57 (br s, 2H).

### Step 5: synthesis of target compound WX044-8

**WX044-6** (1.8 g, 6.22 mmol, 1 *eq.)* and **WX013-6** (869.93 mg, 6.22 mmol, 1.0 *eq.;* crude) were dissolved in a mixed solution of acetonitrile (50 mL) and water (15 mL), and sodium carbonate (2.64 g, 24.87 mmol, 4 *eq.)* was added. Under nitrogen atmosphere, Pd(dppf)Cl₂.CH₂Cl₂ (1.02 g, 1.24 mmol, 0.2 *eq.)* was added, and the reaction system was stirred at 70 °C for 1 h. The organic solvent was removed by rotary evaporation, and the residue was added with water (about 100 mL), and extracted with dichloromethane (100 mL × 3). The organic phases were combined, dried over anhydrous sodium sulfate, filtered and concentrated. The crude product was separated by flash silica gel column chromatography (dichloromethane:methanol = 20:1) to give the target compound **WX044-8.**

### Step 6: synthesis of target compound WX044-9

Under nitrogen atmosphere, Dess-Martin periodinane (1.39 g, 3.28 mmol, 1.02 mL, 2 *eq.)* was added to a solution of **WX044-8** (76.85% purity) in DMSO (20 mL). The system was stirred at room temperature (25 °C) for 1 h. The reaction system turned from a turbid suspension to a clear solution. The organic solvent was removed by rotary evaporation. The reaction system was added with water (about 10 mL) to quench the reaction, and extracted with dichloromethane (10 mL × 3). The organic phases were combined, dried over anhydrous sodium sulfate, filtered, and concentrated (no heating) to give a crude product. The crude product was washed with petroleum ether (about 20 mL) to give the target product **WX044-9**.¹H NMR (400MHz, DMSO-*d*₆) δ 9.82 (s, 1H), 9.02 (d, *J*=2.0 Hz, 1H), 8.12 (dd, *J*=2.3, 9.5 Hz, 1H), 7.93 (d, *J*=9.5 Hz, 1H), 7.53 - 7.47 (m, 2H), 7.34 - 7.23 (m, 4H).

### Step 7: synthesis of target compound WX044-10

**WX044-9** (0.4 g, 1.32 mmol, 1 *eq.)* was dissolved in tetrahydrofuran (10 mL). At 0 °C, methylmagnesium bromide (3 M, 2.20 mL, 5 *eq.)* was added dropwise and the reaction system was stirred for 1 h. The organic solvent was removed by rotary evaporation. The reaction system was added with water (about 100 mL) to quench the reaction, and extracted with dichloromethane (100 mL × 3). The organic phases were combined, dried over anhydrous sodium sulfate, filtered and concentrated. The crude product was purified by preparative thin layer chromatography (dichloromethane:methanol = 30:1) to give the target compound **WX044-10.**

### Step 8: synthesis of target compounds WX044 and WX045

**WX001-5** (90.14 mg, 313.75 µmol, 1 *eq.)* and **WX044-10** (0.1 g, 313.75 µmol, 1 *eq.)* were dissolved in tetrahydrofuran (3 mL) before triphenylphosphine (125.08 mg, 476.89 µmol, 1.52 eq.) was added. The mixture was stirred for 5 min at 50 °C. Under nitrogen atmosphere, diisopropyl azodicarboxylate (96.43 mg, 476.89 µmol, 92.72 µL, 1.52 eq.) was added, and the reaction system was stirred at 60 °C for 12 h. The organic solvent was removed by rotary evaporation. The reaction system was added with water (about 10 mL) to quench the reaction, and extracted with dichloromethane (10 mL × 3). The organic phases were combined, dried over anhydrous sodium sulfate, filtered and concentrated. The crude product was purified by preparative thin layer chromatography (dichloromethane:methanol = 15:1) and separated by preparative high performance liquid chromatography (column: Boston Prime C18 150 mm × 30 mm, 5 µm; mobile phase: [water (0.05% ammonia v/v)-acetonitrile]; B%: 50%-80% (B was acetonitrile), 9 min) to give **WX044** (retention time: 4.229min) and **WX045** (retention time: 9.398min).**WX044:** ¹H NMR (400MHz, CDCl₃) δ 9.04 (dd, *J*=0.9, 2.1 Hz, 1H), 8.19 (s, 1H), 7.75 - 7.60 (m, 2H), 7.40 (dd, *J*=2.3, 11.5 Hz, 1H), 7.34 (dd, *J*=1.1, 8.4 Hz, 1H), 7.10 (t, *J*=8.4 Hz, 1H), 7.06 - 6.97 (m, 1H), 6.90 (br dd, *J*=6.9, 8.2 Hz, 2H), 6.17(q, *J*=7.1 Hz, 1H),5.47 (br s, 2H), 4.72 - 4.51 (m, 1H), 1.92 (d, *J*=7.0 Hz, 3H), 1.40 (d, *J*=6.0 Hz, 6H); **WX045:** ¹H NMR (400MHz, CDCl₃) δ 9.04 (d, *J*=1.5 Hz, 1H), 8.19 (s, 1H), 7.81 - 7.57 (m, 2H), 7.40 (dd, *J*=2.0, 11.5 Hz, 1H), 7.34 (br d, *J*=8.5 Hz, 1H), 7.14 - 6.99 (m, 2H), 6.91 (br t, *J*=7.9 Hz, 2H), 6.16 (q, *J*=7.1 Hz, 1H), 5.50 (br s, 2H), 4.75 - 4.43 (m, 1H), 1.92 (d, *J*=7.0 Hz, 3H), 1.40 (d, *J*=6.0 Hz, 6H).

### Example 24: WX046 and WX047

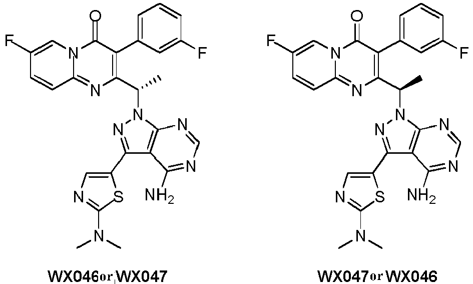

### Synthetic route:

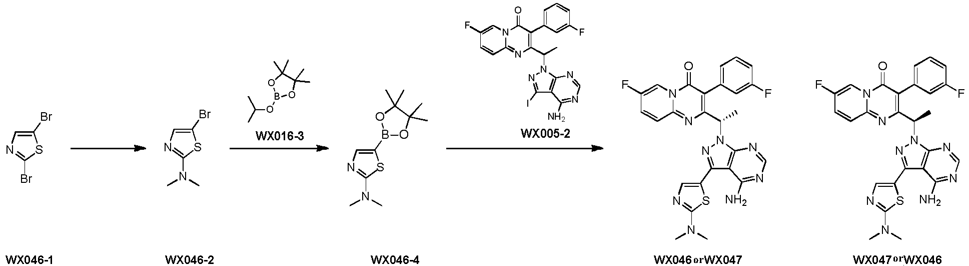

### Step 1: synthesis of target compound WX046-2

**WX046-1** (2 g, 8.23 mmol, 1 *eq.)* was added to a solution of dimethylamine (2 M, 40.00 mL, 9.72 *eq.)* in methanol. The mixture was stirred at 50 °C for 16 h. After the reaction was completed, the system was concentrated and the residue was resuspended in water (40 mL). Ethyl acetate (30 mL × 2) was added for extraction. The organic phase was concentrated to give the target compound **WX046-2.**

### Step 2: synthesis of target compound WX046-4

*n*-Butyllithium (2.5 M, 12.75 mL, 3 *eq.)* was added dropwise to a solution of **WX046-2** (2.2 g, 10.62 mmol, 1 *eq.)* and **WX016-3** (5.94 g, 31.93 mmol, 6.51 mL, 3.01 *eq.)* in tetrahydrofuran (60 mL) at -78 °C under nitrogen atmosphere. The reaction system was stirred at that temperature for 1 h, and then stirred at 30 °C for 1.5 h. The reaction was quenched with methanol (20 mL) to give the target compound **WX046-4.**

### Step 3: synthesis of target compounds WX046 and WX047

Pd(PPh₃)₄ (120.00 mg, 103.85 µmol, 1.42e-1 *eq.)* was added to a solution of **WX005-2** (0.4 g, 733.56 µmol, 1 *eq.),* sodium carbonate (320.00 mg, 3.86 mmol, 5.26 *eq.)* and **WX046-4** (560.00 mg, 2.20 mmol, 3 *eq.)* in ethanol (10 mL), water (10 mL) and *N*,*N-*dimethylformamide (20 mL) under nitrogen atmosphere. The reaction system was stirred at 100 °C for 2 h. After the reactionwas completed, the system was concentrated and purified by silica gel column chromatography (petroleum ether/ethyl acetate = 1:1-0:1 to ethyl acetate/methanol =9:1). The crude product was separated by preparative high performance liquid chromatography (column: Waters Xbridge 150 mm × 25 mm, 5 µm; mobile phase: [water (10 mM NH₄HCO₃)-acetonitrile]; B%: 27%-53.25% (B was acetonitrile), 7 min) and supercritical fluid chromatography (column: DAICEL CHIRALPAK AS-H (250 mm × 30 mm, 5 µm); mobile phase: [0.1% ammonia in isopropanol]; B%: 50%-50% (B was 0.1% ammonia in isopropanol), min) to give **WX046** (retention time: 1.661) and **WX047** (retention time: 2.976). **WX046**:¹H NMR (400 MHz, CDCl₃) δ 8.87 (br s, 1 H) 8.11 (s, 1 H) 7.50 - 7.70 (m, 2 H) 7.34 (s, 1 H) 7.12 - 7.18 (m, 1 H) 6.73 - 7.00 (m, 3 H) 6.07 (br d, J=7.00 Hz, 1 H) 5.56 (br s, 2 H) 3.09 (s, 6 H) 1.84 (br d, J=6.88 Hz, 3 H); **WX047:** ¹H NMR (400 MHz, CDCl₃) δ 8.77 - 9.04 (m, 1 H) 7.93 - 8.30 (m, 1 H) 7.46 - 7.71 (m, 2 H) 7.27 - 7.40 (m, 1 H) 7.27 - 7.40 (m, 1 H) 7.11 - 7.19 (m, 1 H) 6.95 (br d, J=7.50 Hz, 1 H) 6.75 - 6.90 (m, 1 H) 6.75 - 6.90 (m, 1 H) 6.07 (q, J=6.92 Hz, 1 H) 5.65 (br s, 2 H) 2.96 - 3.17 (m, 1 H) 2.96 - 3.17 (m, 5 H) 1.84 (d, J=7.00 Hz, 3 H).

### Example 25: WX048 and WX049

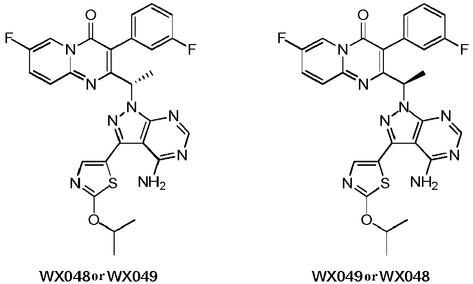

### Synthetic route:

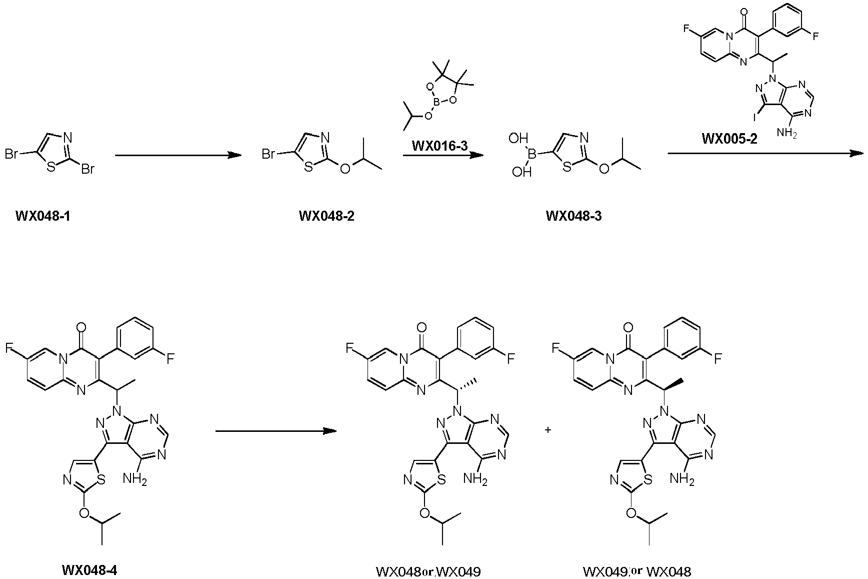

### Step 1: synthesis of compound WX048-2

**WX048-1** (3 g, 12.35 mmol, 1 *eq.)* was dissolved in a mixed solution of DMF (15 mL) and isopropanol (60 mL) in a pre-dried single-necked flask. At 0 °C, sodium hydrogen (1.98 g, 49.40 mmol, 60%, 4 *eq.)* was slowly added. The mixture was stirred for 0.5 h at 0 °C and for 4 h at 20 °C. The reaction system was added with water (100 mL) to quench the reaction, and extracted with ethyl acetate (50 mL × 3). The organic phases were combined, washed with saturated brine (50 mL × 5), dried over anhydrous sodium sulfate, filtered, and concentrated under reduced pressure. The crude product was purified by silica gel column chromatography (petroleum ether:ethyl acetate = 20:1-0:1) to give the target compound **WX048-2**.¹H NMR (400 MHz, DMSO-*d*₆) δ 1.31 - 1.37 (d, 6 H) 5.03 - 5.12 (m, 1 H) 7.27 (s, 1 H).

### Step 2: synthesis of compound WX048-3

**WX048-2** (0.3 g, 1.35 mmol,1 *eq.)* and tetrahydrofuran (6 mL) were added to a pre-dried flask, followed by **WX016-3** (1.01 g, 5.40 mmol, 1.10 mL, 4 *eq.)* and TMEDA (204.06 mg, 1.76 mmol, 265.01 µL, 1.3 *eq.).* After three nitrogen purges, *n*-Butyllithium (2.5 M, 1.62 mL, 3 *eq.)* was slowly added dropwise at -78°C. The reaction system was incubated at -78 °C for 2 h and stirred at 25 °C for 0.5 h. The reaction was quenched with methanol (10 mL). The reaction system was concentrated under reduced pressure to give the target compound **WX048-3.**

### Step 3: synthesis of compound WX048-4

**WX005-2** (282.59 mg, 518.24 µmol, 1 *eq.),* **WX048-3,** 1,4-dioxane (10 mL) and water (5 mL) were added to a pre-dried flask, followed by potassium acetate (152.58 mg, 1.55 mmol, 3 *eq.).* After three nitrogen purges, Pd(dppf)Cl₂.CH₂Cl₂ (84.64 mg, 103.65 µmol, 0.2 *eq.)* was added, followed by another three nitrogen purges. The reaction system was stirred at 70 °C for 16 h. The reaction was quenched with water (10 mL), and the aqueous phase was extracted with dichloromethane (10 mL × 3). The organic phases were combined, washed with saturated brine (10 mL × 2), dried over anhydrous sodium sulfate, filtered, and concentrated under reduced pressure to give a crude product. The crude product was purified by silica gel thin layer chromatography (petroleum ether:ethyl acetate = 0:1) to give **WX048-4.** LCMS: m/z =561.0 [M+1]⁺.

### Step 4: synthesis of compounds WX048 and WX049

**WX048-4** (150 mg, 267.58 µmol, 1 *eq.)* was purified by supercritical chromatography (column: DAICEL CHIRALPAK AY-H (250 mm × 30 mm, 5 µm); mobile phase: hexane-ethanol; B%, 40 min) (B was ethanol) without separation, and then separated by preparative high performance liquid chromatography (column: HUAPU C8 Extreme BDS 150 mm × 30 mm, 5 µm; mobile phase: water (10 mM NH₄HCO₃)-ACN; B%: 40%-60%, 10 min). The mixture was separated by supercritical chromatography (column: DAICEL CHIRALPAK AD-H (250 mm × 30 mm, 5 µm); mobile phase: 0.1% ammonia in isopropanol; B%: 45%-45% (B was isopropanol), 8 min) to give **WX048** (retention time: 5.81 min) and **WX049** (retention time: 8.07 min).**WX048:** ¹H NMR (400 MHz, CDCl₃) δ 1.45 (d, *J*=6.24 Hz, 6 H) 1.92 (d, *J*=6.97 Hz, 3 H) 5.24 (dt, *J*=12.38, 6.22 Hz, 1 H) 5.53 (s, 2 H) 6.15 (q, *J*=7.01 Hz, 1 H) 6.89 - 6.97 (m, 2 H) 7.05 (d, *J*=7.70 Hz, 1 H) 7.36 (s, 1 H) 7.61 - 7.72 (m, 2 H) 8.22 (s, 1 H) 8.94 (dd, *J*=3.73, 2.63 Hz, 1 H); LCMS: m/z = 561.0 [M+1]; **WX049:** ¹H NMR (400 MHz, CDCl₃) δ 1.45 (d, *J*=6.24 Hz, 6 H) 1.92 (d, *J*=6.97 Hz, 3 H) 5.19 - 5.29 (m, 1 H) 5.53 (s, 2 H) 6.15 (q, *J*=7.05 Hz, 1 H) 6.89 - 6.98 (m, 2 H) 7.05 (d, *J*=7.70 Hz, 1 H) 7.36 (s, 1 H) 7.62 - 7.71 (m, 2 H) 8.22 (s, 1 H) 8.94 (dd, *J*=3.73, 2.63 Hz, 1 H); LCMS: m/z = 561.0 [M+1]⁺.

### Example 26: WX050 and WX051

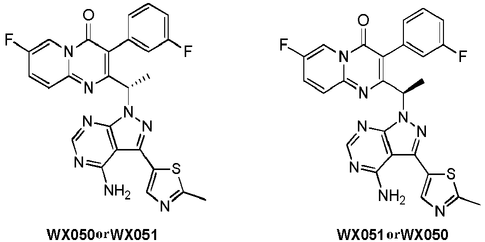

### Synthetic route:

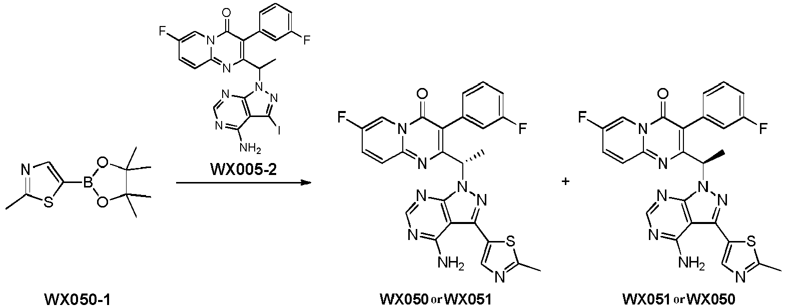

### Step 1: synthesis of target compounds WX050 and WX051

**WX050-1** (0.3 g, 550.17 µmol, 1 *eq.;* crude), **WX050-2** (124.96 mg, 550.17 µmol, 1 *eq.)* were dissolved in a mixed solvent of *N*,*N-*dimethylformamide (10 mL), ethanol (5 mL) and water (5 mL). Potassium carbonate (228.12 mg, 1.65 mmol, 3.0 *eq.)* was added. Under nitrogen atmosphere, Pd(PPh₃)₄ (127.15 mg, 110.03 µmol, 0.2 *eq.)* was added. The mixture was stirred for 2 h at 100 °C. The organic solvent was removed by rotary evaporation. The reaction system was added with water (about 10 mL) to quench the reaction, and extracted with dichloromethane (10 mL × 3). The organic phases were combined, dried over anhydrous sodium sulfate, filtered and concentrated. The crude product was separated by preparative high performance liquid chromatography (Waters Xbridge 150 mm × 25 mm, 5 µm; mobile phase [water (10 mM NH₄HCO₃)-acetonitrile]; B%: 27%-53.25% (B was acetonitrile), 7 min) to give a product. The product was separated by supercritical fluid chromatography (column: DAICEL CHIRALPAK AD (250 mm × 30 mm,10 µm); mobile phase: 0.1% ammonia in ethanol; B%: 455%-45% (B was ethanol)) to give **WX050** (retention time: 2.656 min) and **WX051** (retention time: 4.410 min).**WX050**:¹H NMR (400MHz, CDCl₃) δ 9.00 - 8.75 (m, 1H), 8.14 (s, 1H), 7.78(s, 1H), 7.59 - 7.57 (m, 2H), 7.25 - 7.19 (m, 1H), 6.98 (d, *J*=7.6 Hz, 1H), 6.94 - 6.75 (m, 2H), 6.09 (q, *J*=7.0 Hz, 1H), 5.51 (br s, 2H), 2.67 (s, 3H), 1.85 -1.79(d, *J*=7.0 Hz, 3H); **WX051:** ¹H NMR (400MHz, CDCl₃) δ 9.04 - 8.71 (m, 1H), 8.14 (s 1H), 7.78 (s, 1H), 7.69 - 7.53 (m, 2H), 7.23 - 7.20 (m, 1H), 6.98 (d, *J*=7.5 Hz, 1H), 6.93 - 6.68 (m, 2H), 6.09 (q, *J*=7.0 Hz, 1H), 5.52 (br s, 2H), 2.70 (s, 3H), 1.85 -1.79(d, *J*=7.0 Hz, 3H).

### Example 27: WX052 and WX053

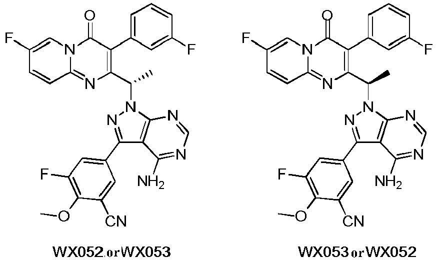

### Synthetic route:

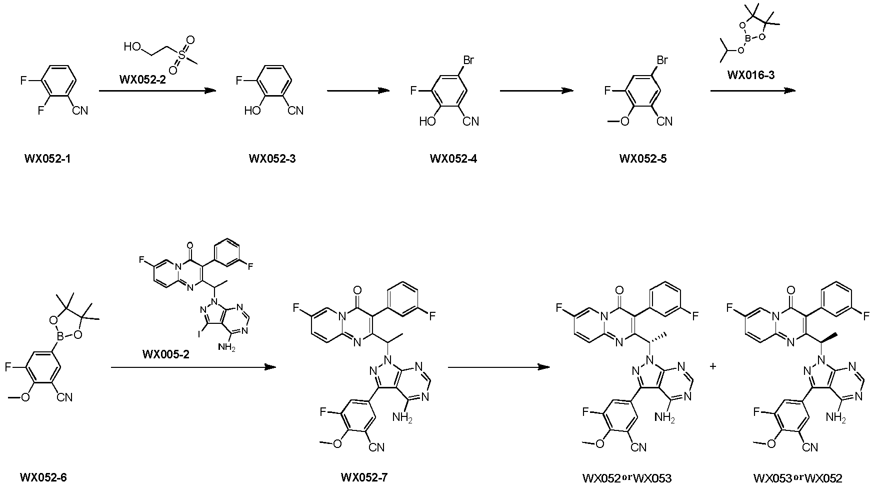

### Step 1: synthesis of compound WX052-3

**WX052-1** (10 g, 71.89 mmol, 8.00 mL, 1 *eq.),* **WX052-2** (8.93 g, 71.89 mmol, 1 *eq.)* and DMF (160 mL) were added to a pre-dried flask before sodium hydride (5.75 g, 143.78 mmol, 60%, 2 *eq.)* was added. The reaction system was stirred at 25 °C for 0.5 h. The reaction was quenched with water (50 mL). The aqueous phase was extracted with dichloromethane (100 mL × 3). The aqueous phase was collected and adjusted to about pH 2 with concentrated hydrochloric acid. A solid was precipitated and separated by filtration to give **WX052-3**.¹H NMR (400 MHz, DMSO-*d*₆) δ6.95 (td, *J*=8.04, 4.71 Hz, 1 H) 7.38 - 7.63 (m, 2 H).

### Step 2: synthesis of compound WX052-4

**WX052-3** (2.5 g, 18.23 mmol, 1 *eq.)* and acetonitrile (75 mL) were added to a pre-dried flask before NBS (3.25 g, 18.23 mmol, 1 *eq.)* was added. The mixture was stirred at 25 °C for 1 h. The reaction was quenched with saturated aqueous sodium carbonate (50 mL). The aqueous phase was extracted with dichloromethane (50 mL × 2). The aqueous phase was collected, adjusted to about pH 2 with concentrated hydrochloric, and extracted with dichloromethane (50 mL × 1). The organic phase was collected and concentrated under reduced pressure to give **WX052-4**.¹H NMR (400 MHz, DMSO-*d*₆) δ8.15 - 8.23 (m, 1 H) 8.29 (dd, *J*=10.58, 2.38 Hz, 1 H) 12.29 (br s, 1 H).

### Step 3: synthesis of compound WX052-5

**WX052-4** (3.8 g, 17.59 mmol, 1 *eq.)* and DMF (150 mL) were added to a pre-dried flask before sodium hydrogen (1.41 g, 35.18 mmol, 60%, 2 *eq.)* was added at 0 °C. The reaction system was stirred at 25 °C for 0.5 h. Then iodomethane (4.99 g, 35.18 mmol, 2.19 mL, 2 *eq.)* was added and the reaction system was further stirred at 25 °C for 15.5 h. The reaction was quenched with water (100 mL). A solid was precipitated and separated by filtration to give **WX052-5**.¹H NMR (400 MHz, DMSO-*d*₆) δ4.06 (d, *J*=2.81 Hz, 3 H) 7.93 - 7.97 (m, 1 H) 8.04 (dd, *J*=11.62, 2.32 Hz, 1 H).

### Step 4: synthesis of compound WX052-6

**WX052-5** (0.3 g, 1.30 mmol, 1 *eq.),* **WX016-3** (970.58 mg, 5.22 mmol, 1.06 mL, 4 *eq.)* and tetrahydrofuran (6 mL) were added to a pre-dried flask before TMEDA (197.01 mg, 1.70 mmol, 255.86 µL, 1.3 *eq.)* was added. After three nitrogen purges, *n*-butyllithium (2.5 M, 1.56 mL, 3 *eq.)* was slowly added dropwise at -78 °C, and the system was incubated at -78 °C for 2 h. The system was stirred for 0.5 h at 25 °C. The reaction was quenched with methanol (10 mL). The mixture was concentrated under reduced pressure to give **WX052-6.**

### Step 5: synthesis of compound WX052-7

**WX005-2** (217.97 mg, 399.75 µmol, 1 *eq.),* **WX052-6** (360 mg, 519.67 µmol, 1.3 *eq.),* 1,4-dioxane (10 mL) and water (1 mL) were added to a pre-dried flask, followed by potassium acetate (117.70 mg, 1.20 mmol, 3 *eq.).* After three nitrogen purges, Pd(dppf)Cl₂ (65.29 mg, 79.95 µmol, 0.2 *eq.)* was added, followed by another three nitrogen purges. The reaction system was stirred at 70 °C for 16 h. The reaction was quenched with water (10 mL), and the aqueous phase was extracted with dichloromethane (10 mL × 3). The organic phases were combined, washed with saturated brine (10 mL × 2), dried over anhydrous sodium sulfate, filtered, and concentrated under reduced pressure to give a crude product. The crude product was purified by silica gel thin layer chromatography (petroleum ether:ethyl acetate = 0:1) to give **WX052-7**.¹H NMR (400 MHz, DMSO-*d*₆) δ 1.80 (d, *J*=6.97 Hz, 3 H) 4.11 (d, *J*=2.69 Hz, 3 H) 6.10 (q, *J*=6.93 Hz, 1 H) 6.92 - 7.01 (m, 3 H) 7.25 (br d, *J*=6.36 Hz, 1 H) 7.53 - 7.64 (m, 1 H) 7.68 - 7.76 (m, 2 H) 7.80 - 7.90 (m, 2 H) 8.05 (s, 1 H) 8.11 (ddd, *J*=9.81, 7.00, 2.87 Hz, 1 H) 8.92 (dd, *J*=4.77, 2.81 Hz, 1 H); LCMS: m/z =569.0 [M+1]⁺.

### Step 6: synthesis of compounds WX052 and WX053

**WX052-7** (280 mg, 384.16 µmol, 1 *eq.)* was separated by supercritical chromatography (column: DAICEL CHIRALPAK AD-H (250 mm × 30 mm, 5 µm); mobile phase: [0.1% ammonia in isopropanol]; B%: 40%-40% (B was 0.1% ammonia in isopropanol), 8 min). The two separated fractions were dried by rotary evaporation and purified by preparative high performance liquid chromatography (column: Waters Xbridge 150 mm × 25 mm, 5 µm; mobile phase: [water (10 mM NH₄HCO₃)-acetonitrile]; B%: 35%-65%,10 min) to give **WX052** and **WX053.** The retention time of**WX052in** SFC was 6.2 min.The retention tine of**WX053** in SFC was 11.4 min.**WX052:** ¹H NMR (400 MHz, CDCl₃) δ 1.93 (d, *J*=6.97 Hz, 3 H) 4.22 (d, *J*=3.18 Hz, 3 H) 5.30 (s, 2 H) 6.20 (q, *J*=6.97 Hz, 1 H) 6.90 - 6.99 (m, 1 H) 6.90 - 6.99 (m, 1 H) 7.09 (d, *J*=7.58 Hz, 1 H) 7.29 - 7.36 (m, 1 H) 7.63 - 7.76 (m, 4 H) 8.25 (s, 1 H) 8.95 (ddd, *J*=4.49, 2.35, 1.10 Hz, 1 H),LCMS: m/z = 569.1 [M+1]⁺; **WX053:** ¹H NMR (400 MHz, CDCl₃) δ 1.93 (d, *J*=6.97 Hz, 3 H) 4.22 (d, *J*=3.18 Hz, 3 H) 5.35 (br s, 2 H) 6.20 (q, *J*=6.89 Hz, 1 H) 6.90 - 7.00 (m, 2 H) 7.09 (d, *J*=7.58 Hz, 1 H) 7.31 (br d, *J*=6.36 Hz, 1 H) 7.62 - 7.70 (m, 4 H) 8.25 (s, 1 H) 8.94 - 8.98 (m, 1 H),LCMS: m/z = 569.0 [M+1]⁺.

### Example 28: WX054 and WX055

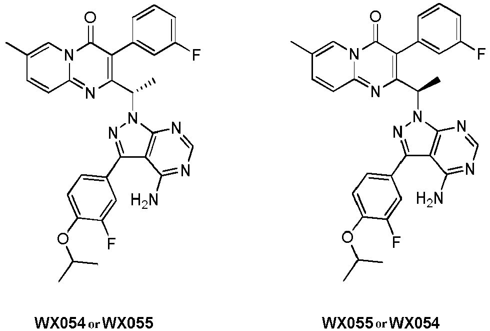

### Synthetic route:

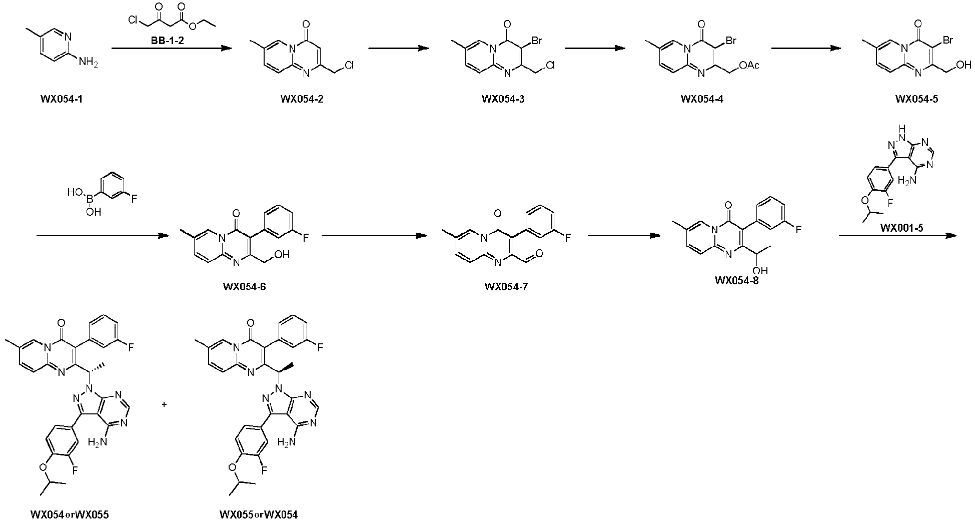

### Step 1: synthesis of target compound WX054-2

**WX054-1** (10 g, 92.47 mmol, 1 *eq.),* **BB-1-2** (22.83 g, 138.71 mmol, 1.5 *eq.)* was added to polyphosphoric acid (50 mL). The reaction system was incubated at 125 °C for 16 h. The system was diluted with water (1000 mL), adjusted to pH 10 with sodium hydroxide, and filtered. The filtrate was concentrated by rotary evaporation to give the target compound WX054-2.

### Step 2: synthesis of target compound WX054-3

**WX054-2** (20 g, 95.86 mmol, 1 *eq.;* crude) and NBS (18.77 g, 105.44 mmol, 1.1 *eq.)* were added to acetic acid (150 mL). The reaction system was incubated at 15 °C for 16 h. The system was concentrated by rotary evaporation, diluted with water (300 mL) and filtered. The filter cake was dried to give the target compound **WX054-3.**

### Step 3: synthesis of target compound WX054-4

**WX054-3** (27 g, 93.90 mmol, 1 *eq.;* crude) and potassium acetate (13.78 g, 140.43 mmol, 1.5 *eq.)* were dissolved in *N*,*N-*dimethylformamide (150 mL) and the reaction system was incubated at 40 °C for 3 h. The system was concentrated by rotary evaporation, diluted with water (500 mL) and filtered. The filter cake was dried to give the target compound **WX054-4.** ¹H NMR (400 MHz, DMSO-*d*₆) δ 8.82 (br d, J=0.8 Hz, 1 H), 7.89 - 8.03 (m, 1 H), 7.70 (d, J=9.0 Hz, 1 H), 5.19 (s, 2 H), 2.44 (s, 3 H), 2.16 (s, 3 H).

### Step 4: synthesis of target compound WX054-5

**WX054-4** (5 g, 16.07 mmol, 1 *eq.)* and concentrated hydrochloric acid (12 M, 4.59 mL, 3.43 *eq.)* were added to 1,4-dioxane (100 mL) and the reaction system was incubated at 70 °C for 2 h. The system was concentrated by rotary evaporation, diluted with water (100 mL), adjusted to pH 11 with sodium hydroxide, and filtered. The filter cake was dried to give the target compound **WX054-5**.¹H NMR (400 MHz, CDCl₃) δ 8.94 (s, 1 H), 7.66 - 7.78 (m, 2 H), 4.76 (br d, *J*=3.0 Hz, 2 H), 4.13 (br s, 1 H), 2.51 (s, 3 H).

### Step 5: synthesis of target compound WX054-6

**WX054-5** (3.4 g, 12.63 mmol, 1 *eq.),* **WX013-6** (3.54 g, 25.27 mmol, 2 *eq.),* Pd(dppf)Cl₂ (0.924 g, 1.26 mmol, 9.99e-2 *eq.),* and sodium carbonate (6.66 g, 62.87 mmol, 4.98 *eq.)* were added to acetonitrile (45 mL) and water (15 mL). The system was purged with nitrogen 3 times, and then incubated at 100 °C for 40 min. The reaction system was concentrated by rotary evaporation, diluted with water (50 mL), and extracted with dichloromethane (50 mL). The organic phase was concentrated by rotary evaporation. The crude product was purified by silica gel column chromatography (methanol/dichloromethane = 0-10%) to give the target compound **WX054-6.**

### Step 6: synthesis of target compound WX054-7

Oxalyl chloride (2.27 g, 17.87 mmol, 1.56 mL, 2.99 *eq.)* was dissolved in dichloromethane (17 mL), to which DMSO (2.80 g, 35.87 mmol, 2.80 mL, 6 *eq.)* was added. The mixture was stirred at -78 °C for 1 h before a solution of **WX054-6** (1.7 g, 5.98 mmol, 1 *eq.)* in dichloromethane (17 mL) was added to the mixture. The reaction system was stirred at -78 °C for 1 h before triethylamine (6.06 g, 59.85 mmol, 8.33 mL, 10.01 *eq.)* was added, and then the system was stirred at -78 °C for 1 h and at 15 °C for 1 h. The reaction system was diluted with water (100 mL), and extracted with dichloromethane (100 mL). The organic phase was concentrated by rotary evaporation at 20 °C to give the target compound **WX054-7.**

### Step 7: synthesis of target compound WX054-8

**WX054-7** (1.7 g, 6.02 mmol, 1 *eq.;* crude) was dissolved in tetrahydrofuran (20 mL). Under nitrogen atmosphere, methylmagnesium bromide (3 M, 4 mL, 1.99 *eq.)* was added at 0 °C. The system was incubated for 16 h at 15 °C. The reaction was quenched with water (10 mL). The mixture was concentrated by rotary evaporation, purified by column chromatography (methanol:dichloromethane = 0-10%), and separated by high performance liquid chromatography (column: Boston Prime C18 150 mm × 30 mm, 5 µm; mobile phase: [water (0.05% ammonia v/v)-acetonitrile]; B%: 35%-65% (B was acetonitrile), 8 min) to give the target compound **WX054-8**.¹H NMR (400 MHz, CDCl₃) δ 8.91 (s, 1 H), 7.62 - 7.73 (m, 2 H), 7.46 (td, *J*=7.8, 6.3 Hz, 1 H), 7.06 - 7.18 (m, 3 H), 4.77 - 4.90 (m, 1 H), 4.06 (d, *J*=8.5 Hz, 1 H), 2.49 (d, *J*=1.0 Hz, 3 H), 1.27 (d, *J*=6.5 Hz, 3 H)

### Step 8: synthesis of target compounds WX054 and WX055

**WX054-8** (0.35 g, 1.11 mmol, 1 *eq.,* 94.48%), **WX001-5** (0.35 g, 1.22 mmol, 1.1 *eq.),* and triphenylphosphine (0.436 g, 1.66 mmol, 1.5 *eq.)* were added to tetrahydrofuran (20 mL). Then diisopropyl azodicarboxylate (291.20 mg, 1.44 mmol, 280.00 µL, 1.3 *eq.)* was added to the system and the system was incubated at 40 °C for 3 h. The reaction system was concentrated by rotary evaporation, purified by column chromatography (EA:PE = 0-80%) and preparative high performance liquid chromatography (column: AgelaDuraShell 150 mm × 25 mm × 5 µm ; mobile phase: [water (0.04% ammonia + 10 mM NH₄HCO₃)-acetonitrile]; B%: 44%-74% (B was acetonitrile), 8.5 min), and separated by supercritical fluid chromatography (column: DAICEL CHIRALCEL OD (250 mm × 30 mm,10 µm); mobile phase: [0.1% ammonia in ethanol]; B%: 40%-40% (B was 0.1% ammonia in ethanol), min) to give **WX054** (retention time: 1.848 min) and **WX055** (retention time: 2.231 min).**WX054:** ¹H NMR (400 MHz, DMSO-*d*₆) δ 8.79 (s, 1 H), 8.03 (s, 1 H), 7.89 (dd, J=9.2, 1.9 Hz, 1 H), 7.68 (d, J=9.0 Hz, 1 H), 7.18 - 7.39 (m, 4 H), 6.83 - 7.01 (m, 3 H), 6.07 (q, J=7.0 Hz, 1 H), 4.70 (quin, J=6.0 Hz, 1 H), 2.42 (s, 3 H), 1.79 (d, J=6.8 Hz, 3 H), 1.33 (d, J=6.0 Hz, 6 H); **WX055:** ¹H NMR (400 MHz, DMSO-*d*₆) δ 8.79 (s, 1 H), 8.03 (s, 1 H), 7.89 (dd, J=9.0, 2.0 Hz, 1 H), 7.68 (d, J=9.0 Hz, 1 H), 7.18 - 7.39 (m, 4 H), 6.85 - 7.02 (m, 3 H), 6.07 (q, J=6.8 Hz, 1 H), 4.70 (quin, J=6.0 Hz, 1 H), 2.42 (s, 3 H), 1.79 (d, J=6.8 Hz, 3 H), 1.33 (d, J=6.0 Hz, 6 H).

### Example 29: WX056 and WX057

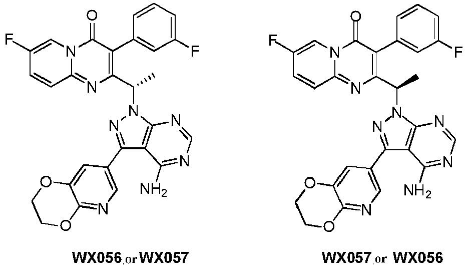

### Synthetic route:

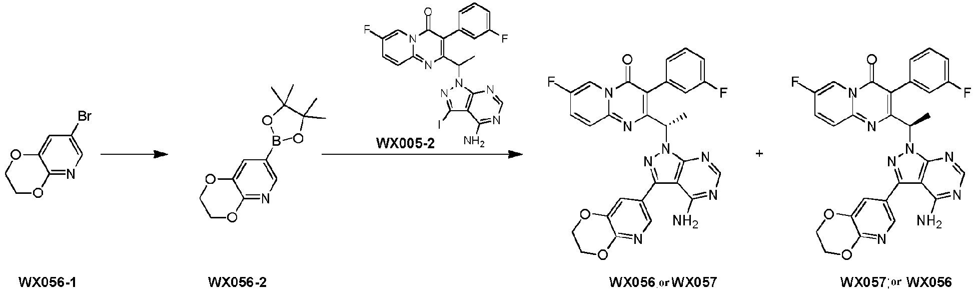

### Step 1: synthesis of target compounds WX056 and WX057

**WX056-1** (50 mg, 231.45 µmol, 1 *eq.),* bis(pinacolato)diboron (64.65 mg, 254.59 µmol, 1.1 eq.), Pd(dppf)Cl₂ (16.94 mg, 23.14 µmol, 0.1 eq.) and potassium acetate (45.43 mg, 462.89 µmol, 2 eq.) were added to the solvent tetrahydrofuran (2 mL). The reaction system was stirred at 110 °C for 1 h to give a solution of **WX056-2**. To the solution were added **WX005-2** (100.96 mg, 185.15 µmol, 0.8 eq.), potassium acetate (22.71 mg, 231.44 µmol, 1 eq.), and Pd(dppf)Cl₂ (16.93 mg, 23.14 µmol, 0.1 eq.), followed by tetrahydrofuran (2 mL) and water (0.5 mL). The reaction system was stirred at 90 °C for 3 h. The system was diluted with water (10 mL)/ethyl acetate (10 mL). The organic phase was collected after separation, and the aqueous phase was extracted with ethyl acetate (5 mL × 3). The organic phases were combined, washed with saturated brine (20 mL), dried over anhydrous sodium sulfate, and concentrated under reduced pressure to give a crude product. The crude product was purified by silica gel thin layer chromatography (dichloromethane:methanol = 20:1) and preparative high performance liquid chromatography (column: Welch Xtimate C18 150 mm × 30 mm, 5 µm; mobile phase: [water (10 mM NH₄HCO₃)-acetonitrile]; B%: 25%-55% (B was acetonitrile), 3 min), and separated by supercritical fluid chromatography (column: DAICEL CHIRALPAK AD (250 mm × 50 mm, 10 µm); mobile phase: 0.1% ammonia in ethanol; B%: 50%-50% (B was 0.1% ammonia in ethanol), min) to give **WX056** (retention time: 1.96 min) and **WX057** (retention time: 2.19 min).**WX056:** ¹H NMR (400 MHz, CDCl₃) δ 8.96 (dd, *J*=2.94, 4.06 Hz, 1H), 8.23 (s, 1H), 8.13 (d, *J*=2.00 Hz, 1H), 7.63-7.73 (m, 2H), 7.52 (d, *J*=2.00 Hz, 1H), 7.30 (br s, 1H), 7.07 (d, *J*=7.63 Hz, 1H), 6.89-6.99 (m, 2H), 6.21 (q, *J*=6.88 Hz, 1H), 5.39 (br s, 2H), 4.50-4.54 (m, 2H), 4.32-4.36 (m, 2H), 1.95 (d, *J*=7.00 Hz, 3H); LCMS: m/z = 555.3 [M+1]⁺.**WX057:** ¹H NMR (400 MHz, CDCl₃) δ 8.96 (dd, *J*=2.94, 4.06 Hz, 1H), 8.23 (s, 1H), 8.13 (d, *J*=2.00 Hz, 1H), 7.63-7.73 (m, 2H), 7.52 (d, *J*=2.00 Hz, 1H), 7.30 (br s, 1H), 7.07 (d, *J*=7.63 Hz, 1H), 6.89-6.99 (m, 2H), 6.21 (q, *J*=6.88 Hz, 1H), 5.39 (br s, 2H), 4.50-4.54 (m, 2H), 4.32-4.36 (m, 2H), 1.95 (d, *J*=7.00 Hz, 3H); LCMS: m/z = 555.3 [M+1]⁺.

### Experimental Example 1: In vitro Evaluation

### 1. In vitro enzyme activity assay

An appropriate substrate and ATP were incubated at a suitable condition for lipid kinase reaction, followed by a two-step detection of kinase activity using an ADP-Glo™ kit. Step I: The kinase reaction was terminated and residual ATP was completely removed, leaving ADP only; Step II: A kinase assay reagent was added to convert ADP to ATP by a luciferin/luciferase reaction. Finally, a fluorescence output value was detected and converted into kinase activity. The condition for PI3K enzymatic activity assayis shown in Table 1.

**Table 1. Condition for PI3K enzymatic activity detection**

| Subtype | Final concentration of enzyme | ATP (µM) | PIP2:3PS (µM) | Reaction time (min) |
|---|---|---|---|---|
| PI3K alpha | 0.2 nM | 40 | 50 | 120 |
| PI3K beta | 0.6 nM | 40 | 50 | 120 |
| PI3K delta | 0.25 nM | 40 | 50 | 120 |
| PI3K gamma | 0.4 nM | 25 | 50 | 120 |

### Materials and equipment:

1)

| | |
|---|---|
| Enzyme: PI3K alpha | Millipore #14-602-K |
| PI3K beta | Promega #V1751 |
| PI3K delta | Millipore #14-604-K |
| PI3K gamma | Millipore #14-558-K |

2) Kit: ADP-Glo™ lipid kinase and PIP2:3PS kit (Promega # V1792)
The kit comprises: 1 mM PIP2:3PS, 10× lipid dilution buffer, 1 M magnesium chloride, 10 mM ATP, 10 mM ADP, ADP-Glo reagent, detection buffer and substrate.
3) Plate: OptiPlate-384, white and transparent (PerkinElmer #6007299)

### Reagent preparation:

1) 10× reaction buffer: 500 mM HEPES (4-(2-hydroxyethyl)-1-piperazineethanesulfonic acid), pH 7.5, 500 mM NaCl, 9 mM MgCl₂; BSA: 10% stock solution, prepared in-house
2) Condition for final test system: 1× reaction system: 50 mM HEPES, 50 mM NaCl, 3 mM MgCl₂, 0.01% BSA (prepared on the day of experiment), 1% DMSO (v/v) +/- compound
3) Reaction system: 3 µL of enzyme and substrate mixture (1:1) + 2 µL of ATP/MgCl₂ mixture + 5 µL of ADP-Glo reagent + 10 µL detection reagent.

The specific experimental procedures are as follows:
1) Dilution: 50 nL of 100× compound/DMSO solution were transferred into a test plate using Echo
   - For PI3Kα, the compound was serially diluted by three fold from the highest concentration 0.111 mM to the 10th concentration.
   - For PI3Kβ/PI3Kδ/PI3Kγ, the compound was serially diluted by three fold from the highest concentration of 1.11 mM to the 10th concentration.
2) Kinase reaction:
   (1) The target compounds were prepared. 50 nL of 100× compound solution or DMSO was added to the corresponding plate
   (2) A 3.33× reaction buffer was prepared
   (3) A 3.33× PIP2:3PS solution were prepared; the PIP2:3PS solution should be thawed by vortex for at least 1 min before use
   (4) An ATP solution containing 5.25 mM MgCl₂ was prepared
   (5) 3.33× PI3Kα/PI3Kβ/PI3Kδ/PI3Kγ solutions were prepared
   (6) The lipid kinase solution and PIP2:3PS solution were mixed at a volume ratio of 1:1
   (7) The 3.33× lipid kinase buffer and PIP2:3PS solution were mixed at a volume ratio of 1:1
   (8) 3 µL of the mixed solution of reaction buffer and PIP2:3PS was added to Columns 1 and 2 of the plate
   (9) 3 µL of the mixed solution of the enzyme and PIP2:3PS was added to the wells of the plate other than Columns 1 and 2, and was centrifuged for 10 s (1000 rpm). The mixture was incubated at 23 °C for 20 min
   (10) 2 µL of ATP solution was added, and the mixture was centrifuged at 1000 rpm and was shaken to mix well
   (11) The plate was covered, shaken for about 30 s, and incubated for 2 h at 23 °C
   (12) 5 µL of ADP-Glo reagent containing 10 mM MgCl₂ was added
   (13) The mixture was centrifuged at 1000 rpm for 10 s, covered and shaken for about 30 s, and incubated at 23 °C for 60 min
   (14) 10 µL of kinase assay reagent was added
   (15) The mixture was centrifuged at 1000 rpm for 10 s, and incubated at 23 °C for 60 min
   (16) Fluorescence values were measured by an Envision system.

### 2. In vitro cell viability assay

### <1> Jeko-1 cell viability test

1) The compound powder was prepared into a 10 mM stock solution with DMSO and stored in a freezer at -20 °C.
2) The compound was diluted to 10 µM with DMEM medium (Invitrogen, Cat # 11965126) (1 mL of medium + 1 µL of 10 mM compound stock solution), and was serially diluted by 4 fold to the 8th gradient (40 µL of the solution +120 µL of diluent).
3) After 24 h of serum-free starvation, the serum-free medium was removed and the corresponding diluted compound was added at 100 µL/well. The cells were incubated for 2 h at 37 °C.
4) 6 µg/mL human recombinant IgM (Sigma Cat # 12386) was added at 5 µL/well. The mixture was incubated at 37 °C/CO₂ for 10 min.
5) The compound was removed by pipette. Lysis buffer was added at 50 µL/well. and the cells wereincubated on a shaker for 30 min.
6) After the cells were completely lysed, 16 µL of lysate was transferred to a 384-well plate and 4 µL of prepared AC + D2 in the kit was added. (AC: Eu3+-cryptate antibody binds to phosphorylated AKT, while D2 binds to non-phosphorylated AKT) The mixture was incubated at room temperature for 4 h.
7) After incubation, readings at 620 and 665 nm on EnVingen were recorded.

### <2> TMD-8 cell viability test

1) Cell culture
   The tumor cells wereincubated in an incubator at 37 °C and 5% CO₂ in the condition shown in Table 2. Cells were regularly passaged. Cells at logarithmic growth phase were transferred to the plate.
2) Plating
   (1). Cells were stained with trypan blue and viable cells were counted.
   (2). Cell concentration was adjusted to 7000 cells/well
   (3). 90 µL of cell suspension was added to each well of the plate and cell-free medium was added to blank control wells.
   (4). The plate was incubated overnight at 37 °C, 5% CO₂ and 100% relative humidity in an incubator.
3) Preparation of plate containing compound stock
   (1). Preparation of plate containing 400× compound stock: Compounds were diluted from the highest concentration gradient to lowest concentration with DMSO.
4) Preparation of 10× compound working solution and treatment
   (1). Preparation of 10× compound working solution: 78 µL of cell culture medium was added to a V-bottom 96-well plate, and 2 µL of compound stock solution was pipetted from the plate containing 400× compound stock into the cell cultures of 96-well plate. 2 µL of DMSO was added to the vehicle control wells and blank control wells. After compounds or DMSO was added, the mixture was well mixed by a pipette.
   (2). Adding compounds: 10 µL of 10× compound working solution was added to the cell culture plate as shown in Table 1. 10 µL of DMSO-cell culture medium mixture solution was added to the vehicle control wells and blank control wells. The final concentration of DMSO was 0.25%.
   (3). The 96-well cell plate was incubated in the incubator for another 72 h.
5) CellTiter-Glo luminescent cell viability assay

The following procedure was performed according to the instructions of Promega CellTiter-Glo luminescent cell viability assay kit (Promega-G7573).
(1). CellTiter-Glo buffer was thawed and let stand at room temperature.
(2). The CellTiter-Glo substrate was let stand at room temperature.
(3). The CellTiter-Glo buffer was added to a flask of CellTiter-Glo substrate to dissolve the substrate, thereby preparing a CellTiter-Glo working solution.
(4). The solution was vortexed to completely dissolve the substrate.
(5). The plate was incubated at room temperature for 30 min for equilibration.
(6). 50 µL (equal to half the volume of cell culture medium in each well) of CellTiter-Glo working solution was added to each well. The plate was covered by aluminum foil to avoid light.
(7). The plate was shaken on an orbital shaker for 2 min to induce cell lysis.
(8). The plate was let stand at room temperature for 10 min to stabilize the luminescence signals.
(9). The luminescence signals were detected on 2104 EnVision plate reader.

The results are shown in Table 2.

**Table 2: Results of in vitro screening for compounds disclosed herein**

| **Compound** | PI3Kα IC₅₀ (nM) | PI3Kβ IC₅₀ (nM) | PI3Kδ IC₅₀ (nM) | PI3Kγ IC₅₀ (nM) | Jeko-1 Cell IC₅₀ (nM) | TMD-8 Cell IC₅₀ (µM) |
|---|---|---|---|---|---|---|
| **TGR-1202** | >10000 | >10000 | 413 | 818 | 478 | 4.05 |
| **WX001** | >10000 | >10000 | 397 | 472 | 614 | 2.97 |
| **WX002** | >10000 | >10000 | >1000 | >10000 | - | - |
| **WX003** | - | - | >1000 | >10000 | - | - |
| **WX004** | >10000 | >10000 | 257 | 1221 | 1317 | - |
| **WX005** | >10000 | >10000 | 120 | 8064 | 828 | - |
| **WX006** | - | - | >1000 | >10000 | - | - |
| **WX007** | - | - | 1.88 | 188 | 125 | 0.589 |
| **WX008** | - | - | 783 | >10000 | - | - |
| **WX009** | - | - | >1000 | >10000 | - | - |
| **WX010** | >10000 | >10000 | 51.5 | 427 | 273 | - |
| **WX011** | - | - | 604 | >10000 | - | - |
| **WX012** | >10000 | >10000 | 54.3 | 2047 | 580 | - |
| **WX013** | - | - | 297 | 573 | - | - |
| **WX014** | - | - | 229 | 620 | - | - |
| **WX016** | - | - | 41.5 | 260 | - | 4.11 |
| **WX018** | - | - | 122 | 1549 | - | 3.42 |
| **WX020** | - | - | 491 | 275 | - | >50 |
| **WX022** | - | - | 601 | 9856 | - | - |
| **WX024** | - | - | 313 | 7168 | - | - |
| **WX025** | - | - | 461 | 1129 | - | - |
| **WX026** | - | - | 33.5 | 2221 | - | - |
| **WX028** | - | - | 330 | 2944 | - | - |
| **WX030** | - | - | 45.6 | 1190 | - | - |
| **WX032** | - | - | 16.3 | 1744 | - | - |
| **WX034** | - | - | 4 | 408 | - | - |
| **WX036** | - | - | 66.6 | 1212 | - | - |
| **WX038** | - | - | 39.9 | 2442 | - | 2.32 |
| **WX040** | - | - | 271 | 455 | - | - |
| **WX042** | - | - | 46.9 | 3593 | - | 2.79 |
| **WX046** | - | - | 3.48 | 494 | - | - |
| **WX048** | - | - | 72.5 | 2030 | - | - |
| **WX050** | - | - | 13.9 | 582 | - | - |
| **WX056** | >10000 | >10000 | 188 | 735 | - | - |

| | | | | | | |
|---|---|---|---|---|---|---|
| "-" indicates that no test was performed | | | | | | |

### Conclusion: The compounds disclosed herein can well inhibit the activity of PI3K kinase, and has higher subtype selectivity for PI3Kα/β/γ. In addition, the phosphorylation level of Akt downstream of PI3K in cells can be well inhibited.

### Experimental Example 2: In Vivo Study

### 1. In vivo DMPK study

Objective: To evaluate the plasma concentration of the compounds and pharmacokinetics after a single dose in female Balb/c mice.

Procedures: 8 healthy adult female Balb/c mice were randomized, 4 for intravenous administration and 4 for oral administration. The compounds were mixed with a proper amount of vehicle for intravenous injection (DMSO/PEG200/water (5:45:50 v/v/v)). The mixture was vortexed and treated with ultrasound to give a 1.0 mg/mL clear solution. The clear solution was filtered through a microporous membrane filter for later use. For the oral group, the vehicle was 0.5% MC/0.2% Tween 80. The compound was mixed with the vehicle, and the mixture was vortexed and ultrasonically treated to give 1.0 mg/mL uniform suspension for later use. The mice were administered intravenously at 1 mg/kg or orally at 3 mg/kg. Whole blood was collected at certain time points, and plasma was separated. The drug concentration was measured by LC-MS/MS, and pharmacokinetic parameters were calculated using Phoenix WinNonlin software (Pharsight, USA). The results are shown in Table 3.

**Table 3. Pharmacokinetic results of the compounds in mice**

| **Compound** | **PPB% (human, mouse)** | **Cₘₐₓ (nM)** | **F%** | **Oral DNAUC (nM.h/mpk)** | **V_{d} (L/kg)** | **Cl (mL/min/kg)** | **T_{1/2} (h)** |
|---|---|---|---|---|---|---|---|
| **TGR-1202** | 99.7%, 99.7% | 651 | 91.7% | 3043 | 4.20 | 8.54 | 5.66 |
| **WX001** | 96.6%, 97.7% | 1610 | 101% | 7179 | 2.02 | 4.44 | 4.80 |
| **WX007** | 78.3%, 91.4% | 1591 | 84.7% | 674.0 | 1.75 | 36.7 | 0.644 |
| **WX010** | 86.9%, 92.3% | 2860 | - | 4968 | - | - | - |
| **WX012** | 74.4%, 80.8% | 3175 | - | 2820 | - | - | - |
| **WX016** | - | 2180 | - | 5599 | - | - | 3.85 |
| **WX018** | - | 2245 | - | 1244 | - | - | 1.4 |
| **WX026** | 73.7%, 85.4% | 5255 | - | 2406 | - | - | 1.99 |
| **WX032** | - | 1495 | - | 2662 | - | - | 18.4 |
| **WX038** | - | 1400(1 mpk) | 83.8% | 1825 | 1.28 | 14.2 | 1.76 |
| **WX042** | - | 2620 | 49.3% | 2583 | 1.43 | 5.54 | 3.15 |
| **WX046** | - | 1560 | - | 1648 | - | - | 1.73 |
| **WX050** | - | 1265 | - | 1513 | - | - | 2.38 |
| **WX056** | - | 4950 | - | 2037 | - | - | - |

| | | | | | | | |
|---|---|---|---|---|---|---|---|
| "-" indicates that no test was performed Note: PPB%: plasma protein binding rate; pharmacokinetic parameters for oral administration (Cmax: highest concentration of drug in plasma; F%: oral bioavailability, Oral DNAUC: dose-normalized area under curve); pharmacokinetic parameters for intravenous administration (Vd: apparent volume of distribution; Cl: clearance; T_{1/2}: half-life). | | | | | | | |

### Conclusion: Compared with TGR-1202, the compounds disclosed herein have lower plasma protein binding rate, i.e., more free molecules in the body; the compounds exhibit high exposure, low clearance and good oral bioavailability in mice.

### 2. In vivo efficacy study

Objectives: To evaluate the efficacy of the compounds on human lymphoma TMD-8 subcutaneous xenograft tumors in a CB-17 SCID mouse model.

### Procedures:

### (1) Cell culture

Human lymphoma TMD-8 cells (Shanghai Junrui-UFBN1682) were cultivated in an RPMI 1640 medium containing 10% fetal bovine serum, 100 U/mL penicillin and 100 µg/mL streptomycin through monolayer culture *in vitro* in an incubator at 37 °C/5% CO2. The cells were digested with trypsin-EDTA twice a week for passaging as per conventional practice. When the saturation reached 80%-90%, the cells were collected, counted and grafted

### (2) Tumor cell grafting

0.2 mL (1 × 10⁷ cells) of TMD-8 cells (along with matrigel in a volume ratio of 1:1) was subcutaneously grafted on the right back of each mouse, and the mice were randomized when the mean tumor volume was 99 mm³.

### (3) Preparation of test compound

Vehicle group: 0.5% MC/0.2% Tween 80/99.3% water.

Test compound group: A certain amount of the compound was dissolved in a corresponding volume of vehicle in a brown flask. The mixture was vortexed to give a uniform suspension or clear solution.

The experimental indices were to investigate whether tumor growth was inhibited or delayed or the tumor was cured. Tumor diameters were measured twice weekly using a vernier caliper. The tumor volume was calculated according to the following formula: V = 0.5*a* × *b²,* where *a* and *b* represent the long diameter and short diameter of the tumor, respectively.

The efficacy of compounds against tumor was evaluated by TGI (%). TGI (%) refers to the rate of tumor growth inhibition. Calculation of TGI (%): TGI (%) = [1 - (average tumor volume at the end of administration in a treatment group - average tumor volume at the start of administration of the treatment group) / (average tumor volume at the end of treatment of the solvent control group - average tumor volume at the start of treatment of the solvent control group)] × 100%.

**Table 4. Antitumor efficacy of the compounds in TMD-8 mouse xenograft tumor model**

| **Group** | | **TGI^{C} (%)** |
|---|---|---|
| Vehicle group | | -- |
| ACP-196 75 mg/kg(DO-D21) | | 93.74 |
| ACP-196 50 mg/kg(D0-D17)+25 mg/kg(D18-D20) | | 105.84 |
| TGR-1202 100 mg/kg(D0-D21) | ACP-196 75 mg/kg(D0-D21) | 103.25 |
| WX001 100 mg/kg(D0-D21) | ACP-196 75 mg/kg(DO-D21) | 103.95 |
| WX001 75 mg/kg(D0-D20) | ACP-196 50 mg/kg(D0-D17)+25 mg/kg(D18-D20) | 108.10 |
| WX001 50 mg/kg(D0-D20) | ACP-196 50 mg/kg(D0-D17)+25 mg/kg(D18-D20) | 108.14 |
| WX001 25 mg/kg(D0-D20) | ACP-196 50 mg/kg(D0-D17)+25 mg/kg(D18-D20) | 108.10 |
| WX007 100 mg/kg(DO-D21) | ACP-196 75 mg/kg(DO-D21) | 103.16 |
| WX042 50 mg/kg(D0-D20) | ACP-196 50 mg/kg(D0-D17)+25 mg/kg(D18-D20) | 108.01 |

**Conclusion: The combination of the target compounds with a second generation BTK inhibitor ACP-196 exhibits a remarkable tumor regression effect in a TMD-8 mouse subcutaneous xenograft tumor model.**

## Claims

1. A compound of formula (I), an isomer thereof or a pharmaceutically acceptable salt thereof, 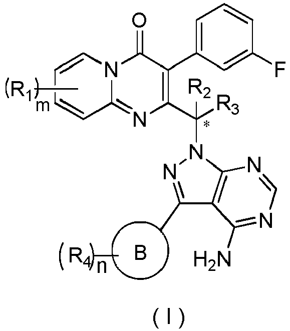 wherein,
R₁ iseach independently selected from H, F, Cl, Br, I, OH, NH₂, CN, and C₁₋₃ alkyl optionally substituted with 1, 2 or 3 R_{c};
R₂ and R₃ are each independently selected from H and C₁₋₃ alkyl optionally substituted with 1, 2 or 3 Rₐ;
R₄ iseach independently selected from H, F, Cl, Br, I, OH, NH₂, CN, COOH, C₁₋₆ alkyl, C₁₋₆ alkoxy, C₁₋₆ alkylthio, C₁₋₆ alkylamino, C₃-₆ cycloalkyl and C₃-₆ cycloalkyl-O-, wherein the C₁₋₆ alkyl, C₁₋₆ alkoxy, C₁₋₆ alkylthio, C₁₋₆ alkylamino, C₃-₆ cycloalkyl and C₃-₆ cycloalkyl-O- are optionally substituted with 1, 2 or 3 R_{b};
or, two R₄ and atoms connected thereto together form a 5-8 membered heterocycloalkenyl;
ring B is selected from phenyl and 5-6 membered heteroaryl;
m is selected from 1, 2 and 3;
n is selected from 1, 2 and 3;
Rₐ, R_{b} and Rₑ are each independently selected from F, Cl, Br, I, OH, NH₂, CN, COOH, CH₃, CH₂CH₃ and OCH₃; the carbon atom with "*" is a chiral carbon atom present in a form of a single (R)- or (S)- enantiomer or in a form enriched with one enantiomer; and
the 5-6 membered heteroaryl contains 1, 2, 3 or 4 heteroatoms or heteroatom groups independently selected from -NH-, -O-, -S- and N.

2. The compound, the isomer thereof or the pharmaceutically acceptable salt thereof according to claim 1, wherein R₁ is selected from H, F, Cl, Br, I, OH, NH₂, CN, and CH₃ optionally substituted with 1, 2 or 3 R_{c},.

3. The compound, the isomer thereof or the pharmaceutically acceptable salt thereof according to claim 2, wherein R₁ is selected from H, F, Cl, Br, I, OH, NH₂, CN and CH₃.

4. The compound, the isomer thereof or the pharmaceutically acceptable salt thereof according to claim 1, wherein R₂ and R₃ are each independently selected from H and CH₃ optionally substituted with 1, 2 or 3 Rₐ.

5. The compound, the isomer thereof or the pharmaceutically acceptable salt thereof according to claim 4, wherein R₂ and R₃ are each independently selected from H and CH₃.

6. The compound, the isomer thereof or the pharmaceutically acceptable salt thereof according to claim 1, wherein R₄ is selected from H, F, Cl, Br, I, OH, NH₂, CN, COOH, C₁₋₃ alkyl, C₁₋₃ alkylamino, C₁₋₃ alkylthio, C₃₋₄ cycloalkyl-O- and C₁₋₃ alkoxy, wherein the C₁₋₃ alkyl, C₁₋₃ alkylamino, C₁₋₃ alkylthio, C₃₋₄ cycloalkyl-O- and C₁₋₃ alkoxy are optionally substituted with 1, 2 or 3 R_{b}.

7. The compound, the isomer thereof or the pharmaceutically acceptable salt thereof according to claim 6, wherein R₄ is selected from H, F, Cl, Br, I, OH, NH₂, CN, COOH, CH₃, CH(CH₃)₂, CH₂CH₃, NHCH₃, SCH₃, OCH₃, OCH₂CH₃, 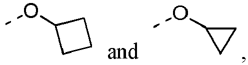 wherein the CH₃, CH(CH₃)₂, CH₂CH₃, NHCH₃, SCH₃, OCH₃, OCH₂CH₃, 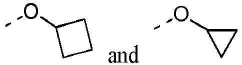 are optionally substituted with 1, 2 or 3 R_{b}.

8. The compound, the isomer thereof or the pharmaceutically acceptable salt thereof according to claim 7, wherein R₄ is selected from H, F, Cl, Br, I, OH, NH₂, CN, COOH, CH₃, CH₂F, CHF₂, CF₃, CH₂CH₃, CH(CH₃)₂, C(CH₃)₃, CH₂CF₃, NHCH₃, N(CH₃)₂, SCH₃, CH₂OCH₃, OCH₃, OCH(CH₃)₂, OCH₂CHF₂, OCH₂CF₃, OCH₂CH₂OCH₃,

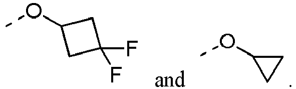

9. The compound, the isomer thereof or the pharmaceutically acceptable salt thereof according to claim 1, wherein two R₄ and atoms connected thereto together form

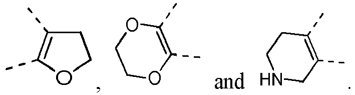

10. The compound, the isomer thereof or the pharmaceutically acceptable salt thereof according to claim 1, wherein ring B is selected from phenyl, thiazolyl, pyrazinyl, pyridazinyl, imidazolyl, pyrimidinyl, pyrazolyl and pyridinyl.

11. The compound, the isomer thereof or the pharmaceutically acceptable salt thereof according to claim 10, wherein the structural unit 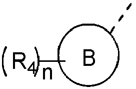 is selected from

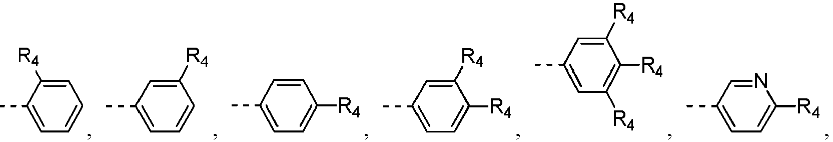

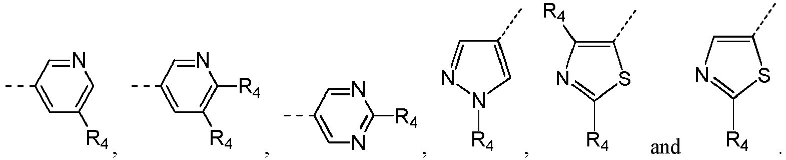

12. The compound, the isomer thereof or the pharmaceutically acceptable salt thereof according to claim 8, 9 or 11, wherein the structural unit 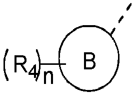 is selected from

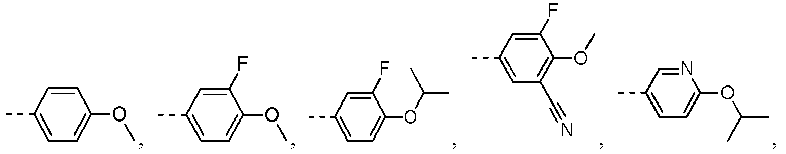

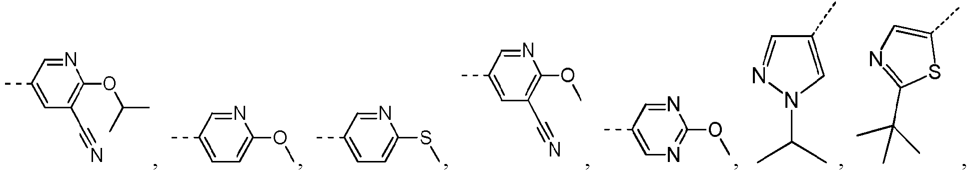

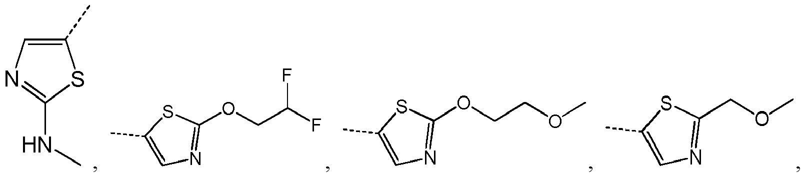

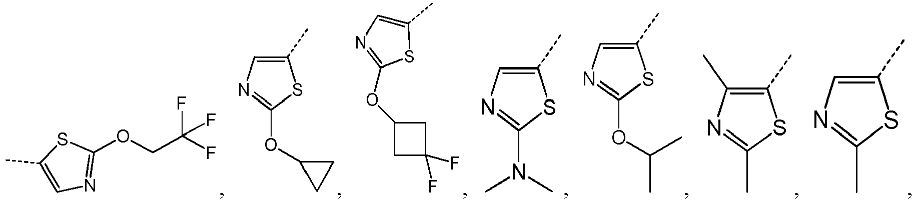

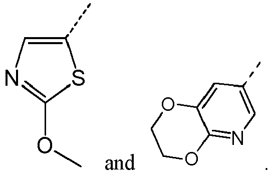

13. The compound, the isomer thereof or the pharmaceutically acceptable salt thereof according to any of claims 1 to 9, selected from: 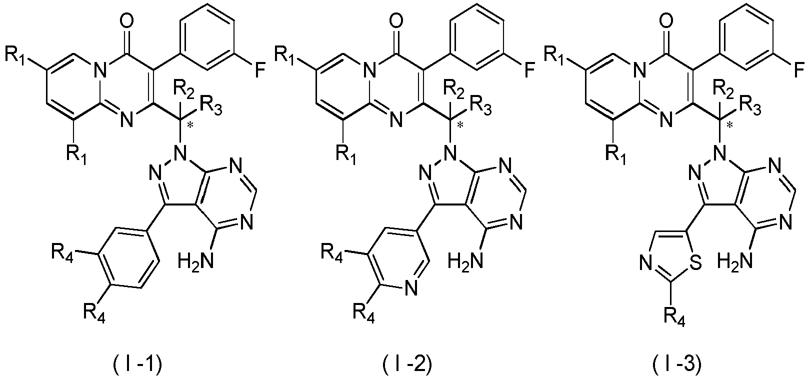 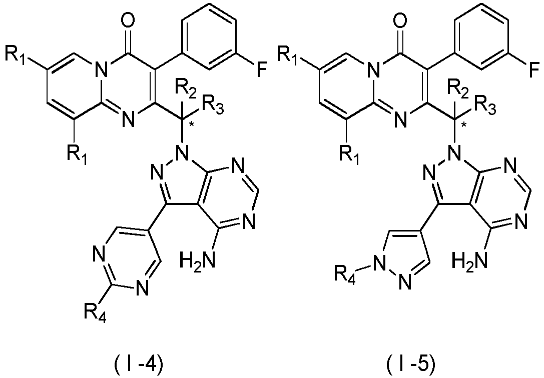 wherein,
R₁ is defined as in any of claims 1 to 3;
R₂ and R₃ are defined as in claim 1, 4 or 5;
the carbon atom with "*" is a chiral carbon atom present in a form of a single (R)- or (S)- enantiomer or in a form enriched with one enantiomer; and
R₄ is defined as in any of claims 1 and 6 to 9.

14. The compound, the isomer thereof or the pharmaceutically acceptable salt thereof according to claim 13, selected from: 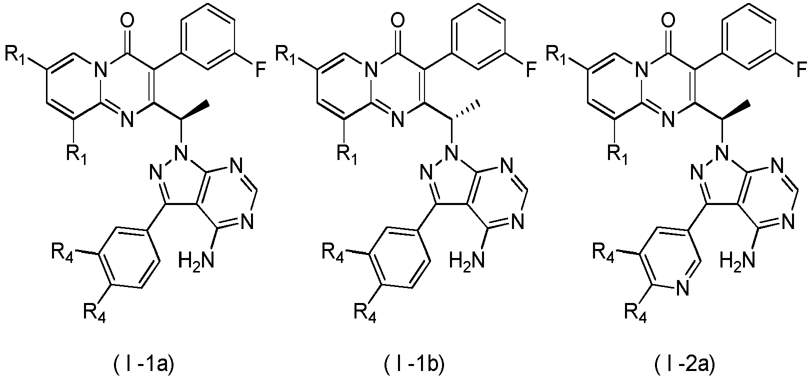 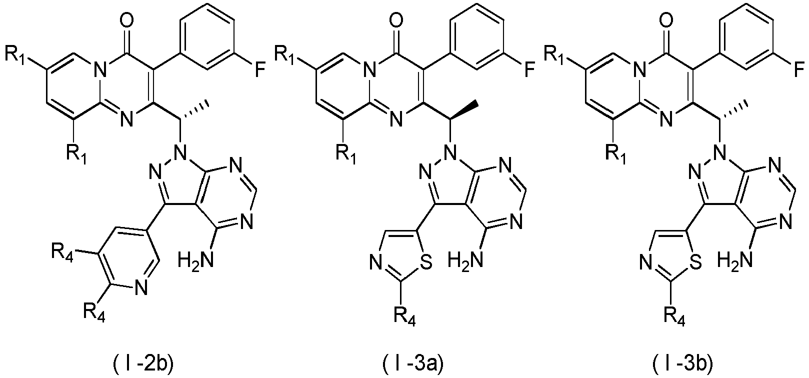 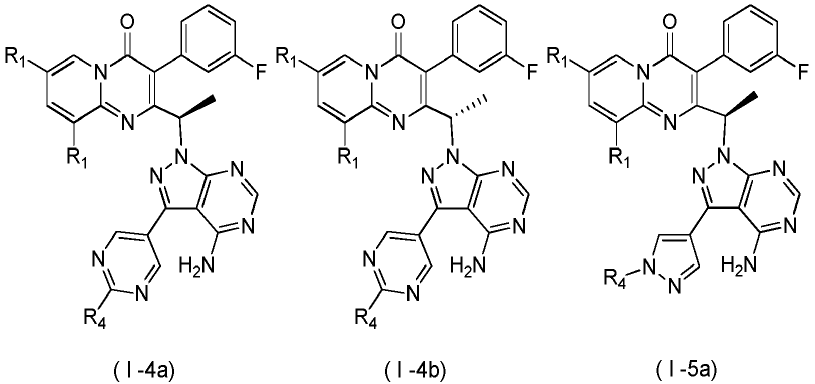 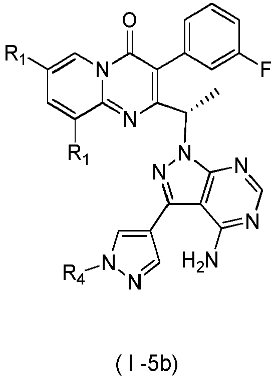 wherein,
R₁ and R₄ are defined as in claim 13.

15. A compound of the following formula, an isomer thereof or a pharmaceutically acceptable salt thereof, wherein the compound is selected from

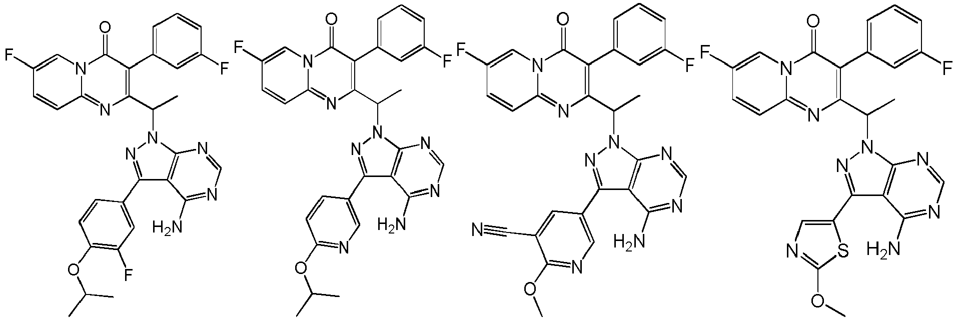

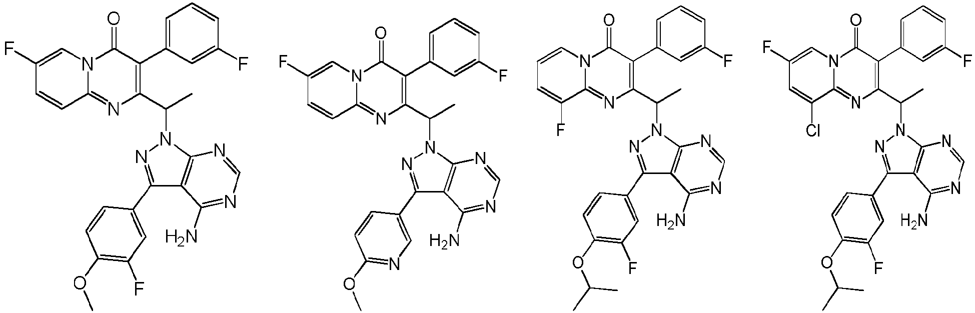

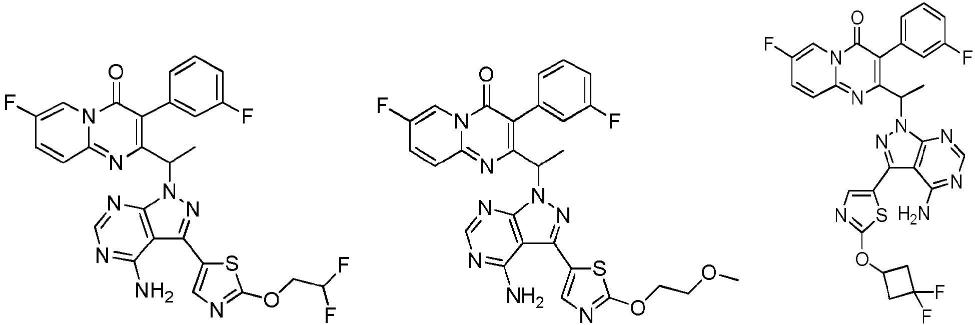

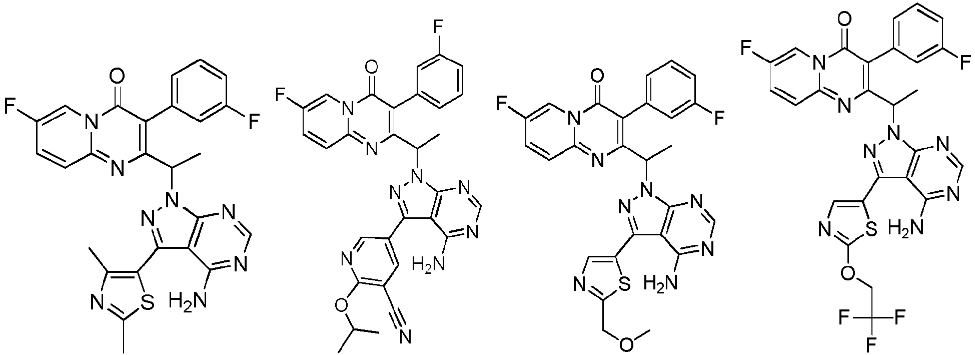

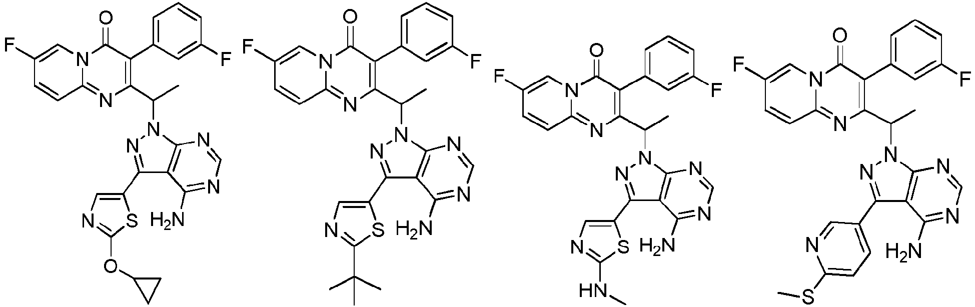

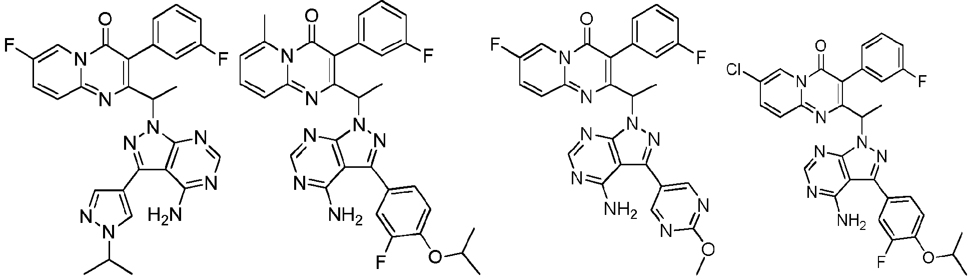

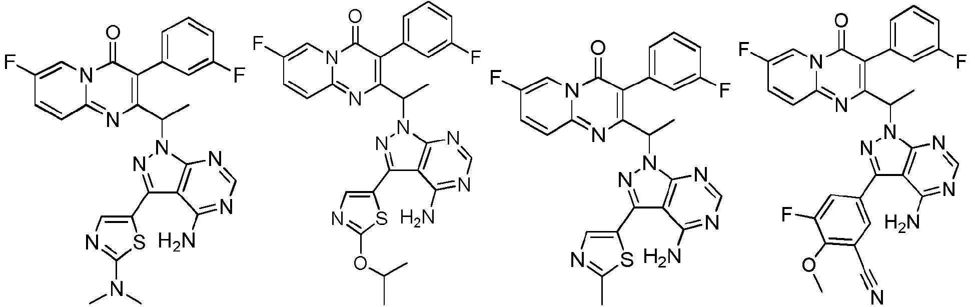

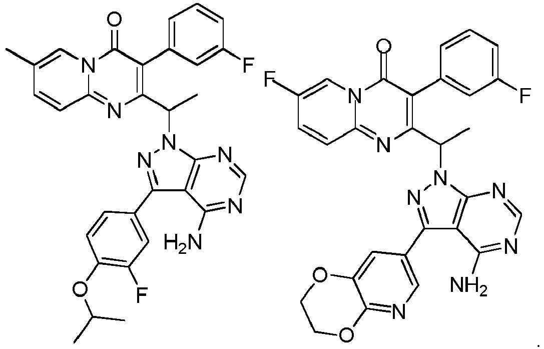

16. The compound according to claim 15, selected from:

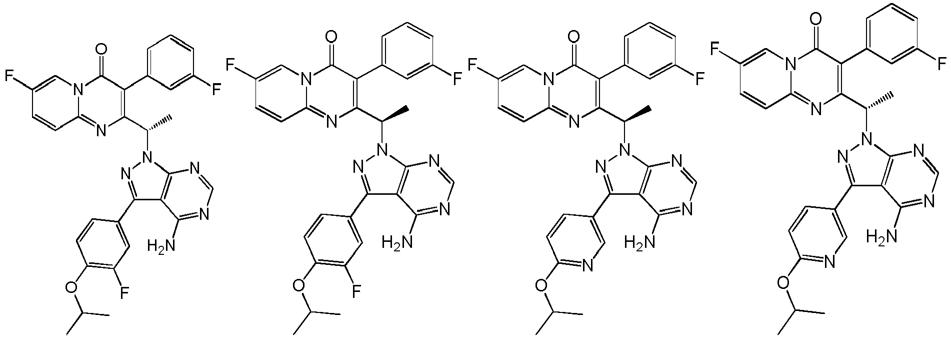

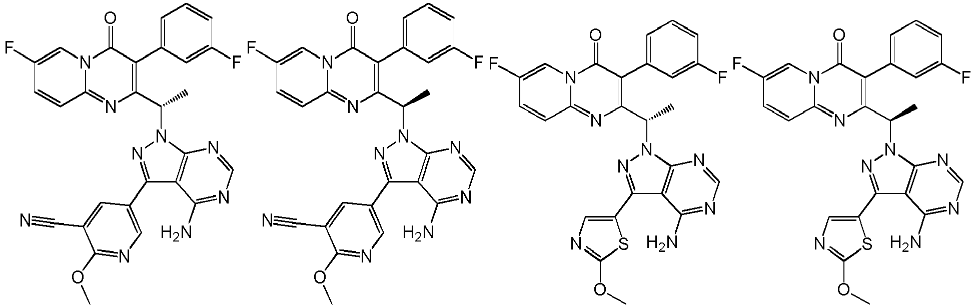

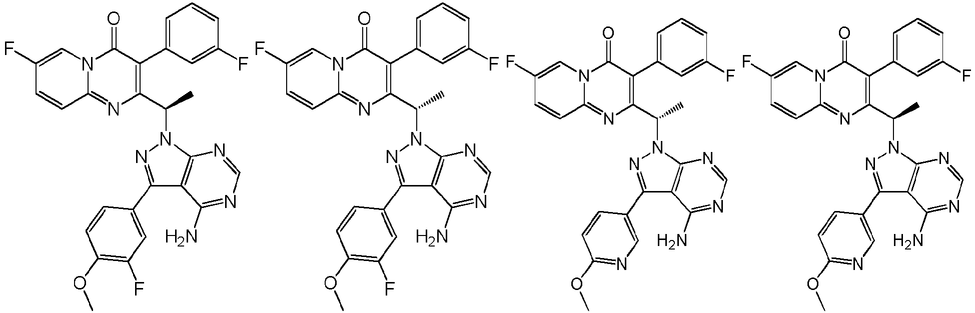

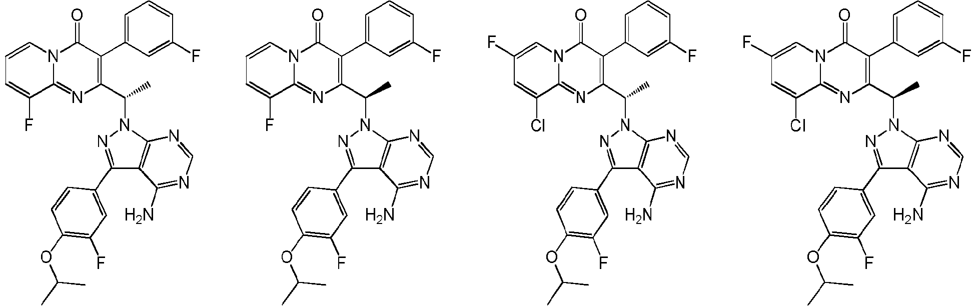

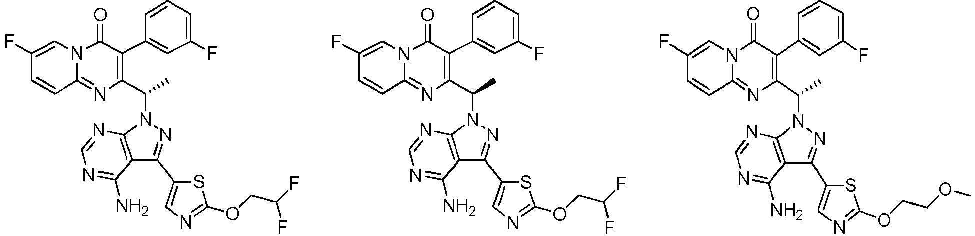

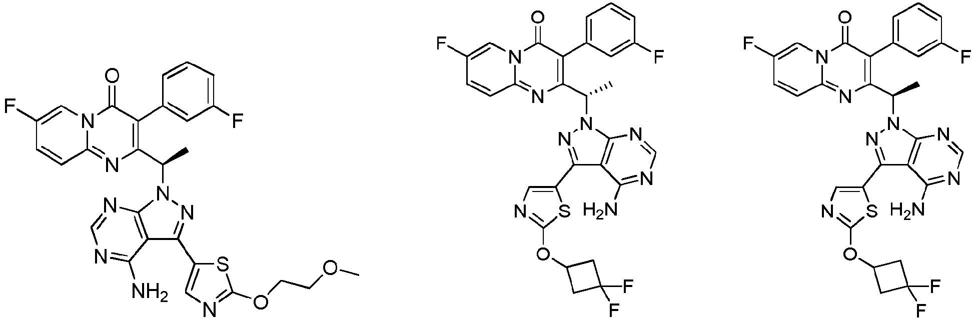

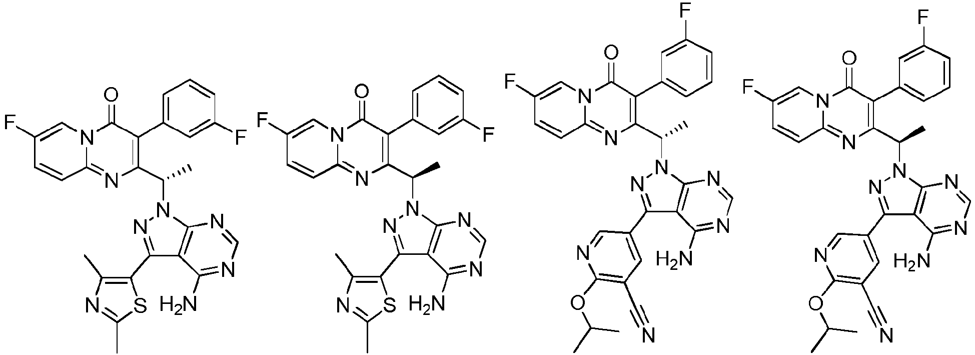

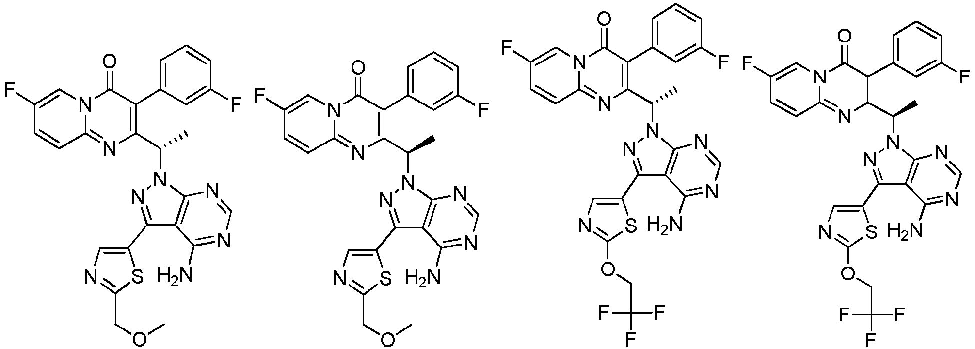

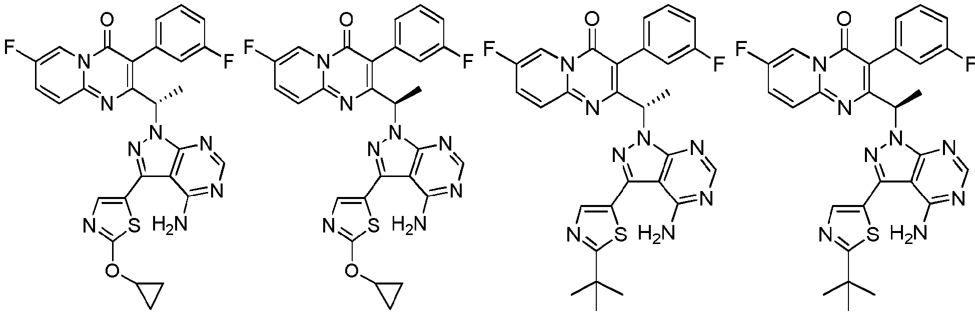

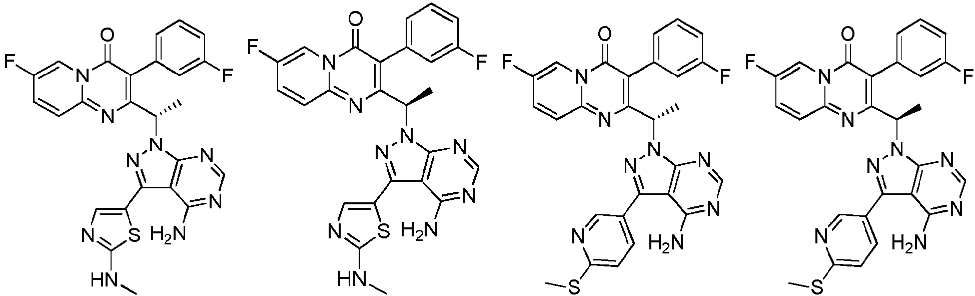

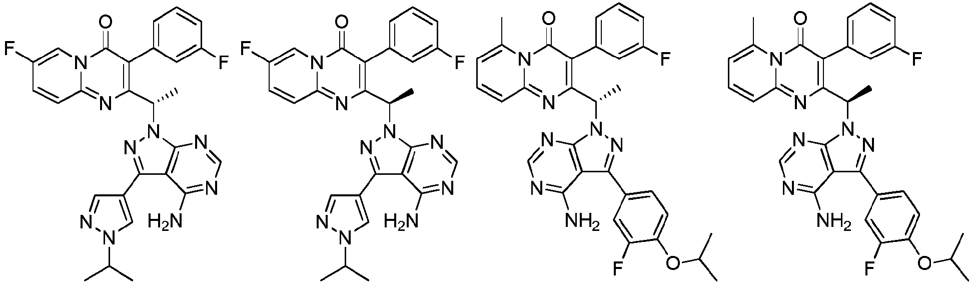

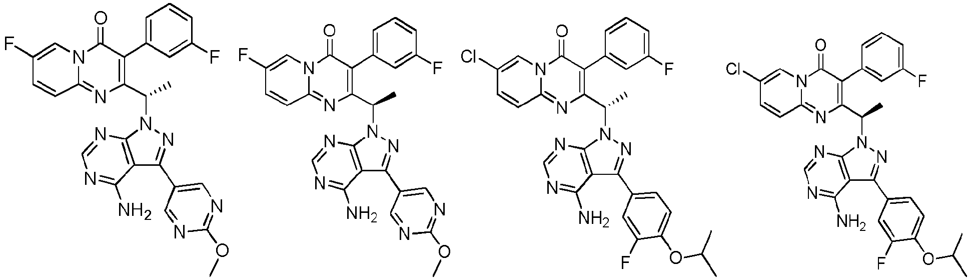

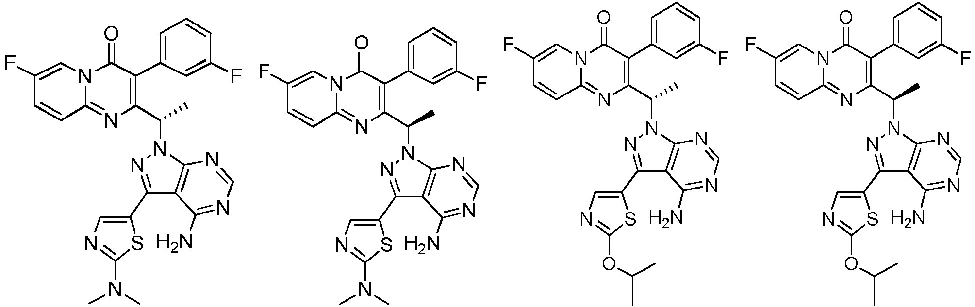

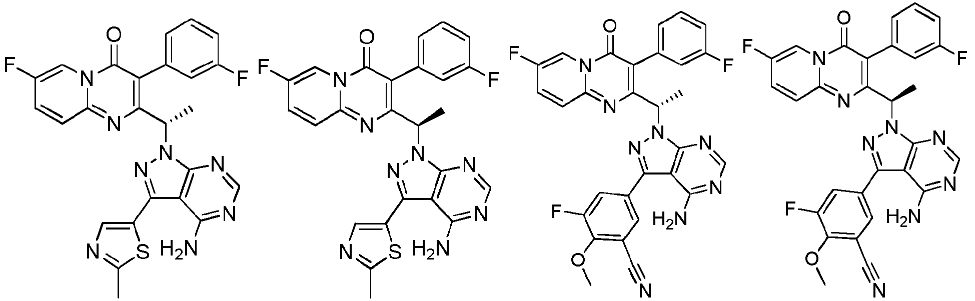

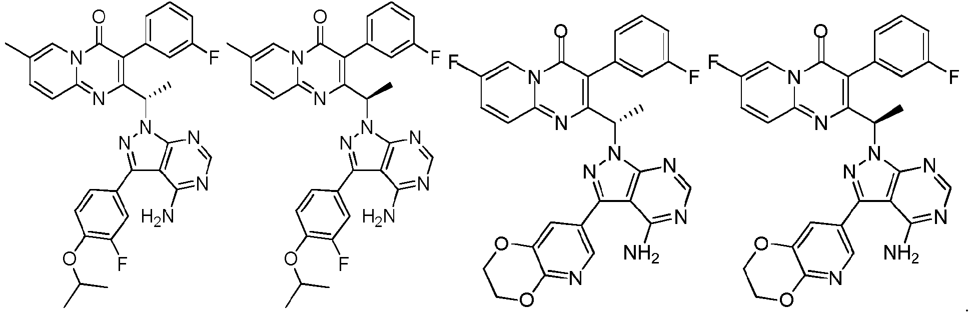

17. Use of the compound, the isomer thereof or the pharmaceutically acceptable salt thereof according to any of claims 1 to 16 in preparing a medicament for the treatment ofa disease related to PI3K.

18. The use according to claim 17, wherein the medicament is for use in the treatment ofchronic lymphocytic leukemia, small lymphocytic lymphoma, marginal zone lymphoma, follicular lymphoma, mantle cell lymphoma, and diffuse large B-cell lymphoma.
